(19) Europäisches Patentamt / European Patent Office / Office européen des brevets

(11) **EP 3 418 378 B1**

(12) **EUROPEAN PATENT SPECIFICATION**

(45) Date of publication and mention
of the grant of the patent:
**25.12.2024 Bulletin 2024/52**

(21) Application number: **17177514.1**

(22) Date of filing: **22.06.2017**

(51) International Patent Classification (IPC):
***C12N 5/0797*** (2010.01)  ***C12N 5/079*** (2010.01)
***C12N 5/0793*** (2010.01)

(52) Cooperative Patent Classification (CPC):
**C12N 5/0618; C12N 5/0619; C12N 5/0622;**
**C12N 5/0623;** C12N 2501/105; C12N 2501/11;
C12N 2501/115; C12N 2501/155; C12N 2501/235;
C12N 2501/41; C12N 2501/415; C12N 2501/60;
C12N 2501/602; C12N 2501/604; C12N 2501/727;
(Cont.)

(54) **NOVEL METHODS FOR THE GENERATION AND USE OF HUMAN INDUCED NEURAL BORDER STEM CELLS**

NEUARTIGE VERFAHREN ZUR ERZEUGUNG UND VERWENDUNG VON MENSCHLICH INDUZIERTEN NEURONALEN GRENZSTAMMZELLEN

NOUVEAUX PROCÉDÉS DE PRODUCTION ET UTILISATION DE CELLULES SOUCHES DE BORDURE NEURONALE INDUITES CHEZ L'HOMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.12.2018 Bulletin 2018/52**

(73) Proprietor: **HI-STEM gGmbH**
**Im Deutschen Krebsforschungszentrum DKFZ**
**69120 Heidelberg (DE)**

(72) Inventors:
• **Trumpp, Dr. Andreas**
**69115 Heidelberg (DE)**
• **Frank, Edenhofer**
**50374 Erftstadt (DE)**
• **Thier, Marc**
**69126 Heidelberg (DE)**

(74) Representative: **Wallinger Ricker Schlotter Tostmann**
**Patent- und Rechtsanwälte mbB**
**Zweibrückenstraße 5-7**
**80331 München (DE)**

(56) References cited:
• **VIVEK K. BAJPAI ET AL: "Reprogramming Postnatal Human Epidermal Keratinocytes Toward Functional Neural Crest Fates : Human Keratinocyte-Derived Neural Crest Cells", STEM CELLS., vol. 35, no. 5, 5 March 2017 (2017-03-05), US, pages 1402 - 1415, XP055442008, ISSN: 1066-5099, DOI: 10.1002/stem.2583**
• **YONG JUN KIM ET AL: "Generation of Multipotent Induced Neural Crest by Direct Reprogramming of Human Postnatal Fibroblasts with a Single Transcription Factor", CELL STEM CELL, vol. 15, no. 4, 1 October 2014 (2014-10-01), AMSTERDAM, NL, pages 497 - 506, XP055417102, ISSN: 1934-5909, DOI: 10.1016/ j.stem.2014.07.013**
• **BUNG RAFFAELA ET AL: "Partial Dedifferentiation of Murine Radial Glia-Type Neural Stem Cells by Brn2 and c-Myc Yields Early Neuroepithelial Progenitors", JOURNAL OF MOLECULAR BIOLOGY, ACADEMIC PRESS, UNITED KINGDOM, vol. 428, no. 7, 10 November 2015 (2015-11-10), pages 1476 - 1483, XP029495648, ISSN: 0022-2836, DOI: 10.1016/ J.JMB.2015.10.028**

- MAMORU ISHII ET AL: "A Stable Cranial Neural Crest Cell Line from Mouse", STEM CELLS AND DEVELOPMENT, vol. 21, no. 17, 20 November 2012 (2012-11-20), NL, pages 3069 - 3080, XP055442225, ISSN: 1547-3287, DOI: 10.1089/scd.2012.0155
- REUVEN EDRI ET AL: "Analysing human neural stem cell ontogeny by consecutive isolation of Notch active neural progenitors", NATURE COMMUNICATIONS, vol. 6, 23 March 2015 (2015-03-23), pages 6500, XP055275483, DOI: 10.1038/ncomms7500
- MARCO MAGISTRI ET AL: "A comparative transcriptomic analysis of astrocytes differentiation from human neural progenitor cells", EUROPEAN JOURNAL OF NEUROSCIENCE., vol. 44, no. 10, 25 September 2016 (2016-09-25), GB, pages 2858 - 2870, XP055442270, ISSN: 0953-816X, DOI: 10.1111/ejn.13382
- BLACK JOSHUA B ET AL: "Targeted Epigenetic Remodeling of Endogenous Loci by CRISPR/Cas9-Based Transcriptional Activators Directly Converts Fibroblasts to Neuronal Cells", CELL STEM CELL, ELSEVIER, CELL PRESS, AMSTERDAM, NL, vol. 19, no. 3, 11 August 2016 (2016-08-11), pages 406 - 414, XP029711990, ISSN: 1934-5909, DOI: 10.1016/J.STEM.2016.07.001
- PIETRO GIUSEPPE MAZZARA ET AL: "Two factor-based reprogramming of rodent and human fibroblasts into Schwann cells", NATURE COMMUNICATIONS, vol. 8, 7 February 2017 (2017-02-07), pages 14088, XP055442373, DOI: 10.1038/ncomms14088
- HENGZHU ZHANG ET AL: "Reprogramming A375 cells to induced-resembled neuronal cells by structured overexpression of specific transcription genes", MOLECULAR MEDICINE REPORTS, vol. 14, no. 4, 8 August 2016 (2016-08-08), GR, pages 3134 - 3144, XP055442381, ISSN: 1791-2997, DOI: 10.3892/mmr.2016.5598
- GASCÓN SERGIO ET AL: "Identification and Successful Negotiation of a Metabolic Checkpoint in Direct Neuronal Reprogramming", CELL STEM CELL, vol. 18, no. 3, 3 March 2016 (2016-03-03), pages 396 - 409, XP029451694, ISSN: 1934-5909, DOI: 10.1016/J.STEM.2015.12.003
- DANIELA ROELLIG: "Dynamic transcriptional signature and cell fate analysis reveals plasticity of individual neural plate border cells", 29 March 2017 (2017-03-29), XP055442277, Retrieved from the Internet <URL:https://www.ncbi.nlm.nih.gov/pmc/articles/PMC5371430/pdf/elife-21620.pdf> [retrieved on 20180118]

(52) Cooperative Patent Classification (CPC): (Cont.)
C12N 2501/999; C12N 2506/11; C12N 2506/115; C12N 2506/1307; C12N 2510/00

**Description**

**FIELD OF THE INVENTION**

This invention relates to a novel approach for the generation of human induced neural border stem cells (iNBSCs) by the direct reprogramming of somatic cells (peripheral blood, skin biopsies), including the differentiation of these stem cells into cell types of the CNS and the neural crest lineages.

**BACKGROUND OF THE INVENTION**

Various types of neural stem and progenitor cells (NSPCs) can be derived by directed differentiation from pluripotent stem cells (PSCs) as well as fetal and adult brain tissue ([1], [2], [3], [4], [5], [6], [7]). However, NSPCs exhibit large variability with respect to self-renewal and differentiation capacity, which depends on (a) their cellular origin (i.e. embryonic stem cells (ESCs), induced pluripotent stem cells (iPSCs), primary tissues), (b) species (mouse, human) and (c) culture condition. Furthermore, the generation of ESC- and iPSC-derived NSPCs suffers from technical hurdles such as lengthy and inefficient differentiation protocols leading to populations comprised of heterogeneous cell types and the risk of co-maintaining tumour-prone pluripotent remnants. The, variability of NSPC generated by directed differentiation and cell types derived therof is particularly problematic when future clinical applications are envisaged.

Similar drawbacks must be considered in the direct conversion towards neural fates especially when taking recourse to protocols, which make use of the Yamanaka-factors (OCT4, SOX2, KLF4, MYC). These are well known to give rise to more than one cell type during reprogramming ([8], [9], [10]), thereby resulting in significant heterogeneity.-Recently, we demonstrated that overexpression of Sox2, Klf4 and c-Myc together with curtailed activity of Oct4 induces a radial glia-type stem cell population from mouse fibroblasts ([11]). Using a similar approach, we subsequently showed that it is possible to unlock even earlier developmental stages ([12]). Nevertheless, this combination of factors failed to convert human cells (M. Thier, unpublished observations) and required neural cells as starting material.

To date, a serious drawback of many direct reprogramming approaches is the generation of cells with limited self-renewal capacity and differentiation potential and the derivation of mixed progenitor cultures without defined physiological counterparts. These limitations hamper the use of directly reprogrammed cells for research requiring patient-derived defined, expandable neural progenitor cell lines that are a prerequisite for many clinical applications.

Thus, despite certain progress that has been made in the generation of neural stem and progenitor cells, there are still many limitations and there is still no robust and safe way for generating such cells.

The solution to this problem, i.e. the reprogramming of human adult somatic cells into early, defined, expandable and self-renewing neural progenitors with broad but definable differentiation potential, is neither provided nor suggested by the prior art.

**OBJECTS OF THE INVENTION**

It was thus an object of the invention to provide a novel method for the generation of defined and self-renewing neural progenitors with broad but definable differentiation potential that can be used to further characterize such cells, their generation and differentiation, the development of disease models for studying neurological disorders and diseases and for developing novel treatments based on such models and/or such cells differentiated *in vitro*.

**SUMMARY OF THE INVENTION**

Surprisingly it was found that by overexpressing stage-specific transcription factors, in combination with providing adequate signalling cues by the growth medium, the direct reprogramming of adult somatic cells to early embryonic neural progenitors with stem cell features could be achieved.

Thus, in a first aspect, the present invention relates to an *in vitro* method for generating induced neural border stem cells by the direct reprogramming of somatic human cells, comprising the step of culturing said somatic human cells in the presence of a mixture of transcription factors, wherein said mixture comprises the factors BRN2, SOX2, KLF4 and ZIC3, and wherein said culturing is performed in the presence of GSK-3 inhibitor Chir99021; Alk5 inhibitor II; and hedgehog/smoothened agonist Purmorphamine. The present application further describes an *in vitro* method for the direct differentiation of pluripotent human stem cells, particularly embryonic stem cells (ESCs) or induced pluripotent stem cells (iPSCs), comprising the step of culturing said pluripotent human stem cells in the presence of a GSK-3 inhibitor, particularly Chir99021; an Alk5 inhibitor, particularly Alk5 inhibitor II; and a hedgehog/smoothened agonist, particularly Purmorphamine.

The present application further describes an *in vitro* method for generation of induced neural border stem cells, comprising the step of culturing mature human cells in the presence of a mixture of transcription factors, wherein said mixture comprises the factors BRN2, SOX2, KLF4 and ZIC3, and wherein said culturing is performed in the presence of a GSK-3 inhibitor, particularly Chir99021; an Alk5 inhibitor, particularly Alk5 inhibitor II; and a hedgehog/smoothened agonist, particularly Purmorphamine. The present application further describes an *in vitro* method for the generation of neural border stem cells, comprising the step of culturing pluripotent human stem cells, particularly embryonic stem cells (ESCs) or induced pluripotent stem cells (iPSCs), in the presence of a GSK-3 inhibitor, particularly Chir99021; an Alk5 inhibitor, particularly Alk5 inhibitor II; and a hedgehog/smoothened agonist, particularly Purmorphamine.

The present application further describes a nucleic acid sequence encoding BRN2, SOX2, KLF4 and ZIC3.

The present application further describes a polycistronic vector encoding BRN2, SOX2, KLF4 and ZIC3.

I The present application further describes a kit comprising at least two, more particularly all three components selected from: a GSK-3 inhibitor, particularly Chir99021; an Alk5 inhibitor, particularly Alk5 inhibitor II; and a hedgehog/smoothened agonist, particularly Purmorphamine.

The present application further describes an isolated (induced) neural border stem cell line.

The present application further describes an *in vitro* method of expanding the isolated (induced) neural border stem cell line as described in the present application, comprising the step of culturing cells from said isolated (induced) neural border stem cell line, particularly wherein said culturing is performed in the presence of proliferation-supporting cytokines, particularly Notch-signaling activating substances, particularly a substance selected from DLL1, DLL3 and DLL4, Jagged-1, and Jagged-2, more particularly from DLL4 and JAGGED-1.

In a next aspect, the present invention relates to an *in vitro* method for generating differentiated induced neural border stem cells, comprising the steps of (i) performing the *in vitro* method of the present invention for generating induced neural border stem cells, and (ii) culturing said induced neural border stem cells or cells from said isolated induced neural border stem cell line in the presence of differentiation factors.

The present application further describes isolated central nervous system primed neural progenitor cell line of the central nervous system lineage, particularly (i) wherein said cell line is of the same development status as primary neural progenitor cells obtainable from embryos of gestation week 8 to 12, and/or (ii) wherein said cell line is characterized by progenitor markers LONRF2, ZNF217, NESTIN, SOX1 and SOX2, particularly LONRF2 and ZNF217, and by being negative for MSX1, PAX3 and TFAP2, and/or (iii) wherein said cell line is characterized by epigenetically corresponding to mature human cells, particularly wherein said cell line has been obtained from said mature human cells in a direct reprogramming method according to the present invention.

In a next aspect, the present invention relates to an *in vitro* method for generating CNS progenitor cells, comprising the steps of (i) performing the *in vitro* method of the present invention for generating induced neural border stem cells, and (ii) culturing induced neural border stem cells or cells from said isolated induced neural border stem cell line in a medium comprising GSK-3 inhibitor Chir99021; aALK inhibitor SB431542; hedgehog/smoothened agonist Purmorphamine; bFGF; and LIF.

The present application further describes an isolated central nervous system progenitor cell line, particularly wherein said cell line is characterized by epigenetically corresponding to mature human cells, particularly wherein said cell line has been obtained from said mature human cells in a direct reprogramming method according to the present invention.

In a next aspect, the present invention relates to an *in vitro* method of generating differentiated central nervous system progenitor cells as described in the present application, comprising the steps of (i) performing the *in vitro* method of the present invention for generating CNS progenitor cells, and (ii) culturing said central nervous system progenitor cells in the presence of differentiation factors.

The present application further describes an isolated cell population having a radial glia type stem cell phenotype, particularly wherein said cell population is characterized by epigenetically corresponding to mature human cells, particularly wherein said cell population has been obtained from said mature human cells in a direct reprogramming method according to the present invention.

The present application further describes an isolated differentiated (induced) neural border stem cell line of the neural crest lineage, particularly wherein said cell line is characterized by epigenetically corresponding to mature human cells, particularly wherein said cell line has been obtained from said mature human cells in a direct reprogramming method according to the present invention.

In a next aspect, the present invention relates to an *in vitro* method for generating neural crest progenitor cells, comprising the steps of (i) performing the *in vitro* method of the present invention for generating induced neural border stem cells, and (ii) culturing induced neural border stem cells or cells from said isolated induced neural border stem cell line for three days in the presence of GSK-3 inhibitor Chir99021; Alk5 inhibitor II; and BMP4; followed by culturing in the presence of GSK-3 inhibitor Chir99021, FGF8, IGF1 and DAPT.

The present application further describes an isolated neural crest progenitor cell line, particularly wherein said cell line is characterized by epigenetically corresponding to mature human cells, particularly wherein said cell line has been obtained from said mature human cells in a direct reprogramming method according to the present invention.

In a next aspect, the present invention relates to an *in vitro* method of generating a differentiated neural crest progenitor cell line, comprising the step of (i) performing the *in vitro* method of the present invention for generating said neural crest progenitor cell line, and (ii) culturing cells from said neural crest progenitor cell line in the presence of differentiation factors.

The present application further describes an isolated cell population having a neural border stem cell phenotype, particularly wherein said cell line is characterized by epigenetically corresponding to mature human cells, particularly wherein said cell population has been obtained from said mature human cells in a direct reprogramming method according to the present invention.

The present application further describes an *in vitro* method for the generation of dopaminergic neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, (ii) changing to a medium that is supplemented with FGF8 and a hedgehog/smoothened agonist, particularly Purmorphamine, on murine fibroblasts; (iii) culturing the cells in the medium according to (ii) for 7 days on a gelatinous protein mixture secreted by Engelbreth-Holm-Swarm mouse sarcoma cells (Matrigel), (iv) changing to a medium that is supplemented with a hedgehog/smoothened agonist, particularly Purmorphamine; (v) culturing the cells in the medium according to (iv) for 2 days, and (vi) changing the medium to maturation medium; and (vii) culturing the cells for 5 weeks in said maturation medium.

The present application further describes an *in vitro* method for the generation of motor neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 2 days on a gelatinous protein mixture secreted by Engelbreth-Holm-Swarm mouse sarcoma cells (Matrigel), (iv) changing to a medium that is supplemented with a hedgehog/smoothened agonist, particularly Purmorphamine, and all-trans retinoic acid; (v) culturing the cells in the medium according to (iv) for 7 days, and (vi) changing the medium to maturation medium; and (vii) culturing the cells for 5 weeks in said maturation medium.

The present application further describes an *in vitro* method for the generation of glutamatergic and gabaergic neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to maturation medium comprising BDNF and GDNF; and (v) culturing the cells for 5 weeks in said maturation medium.

The present application further describes an *in vitro* method for the generation of astrocytes, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to a maturation medium comprising BDNF, GDNF and 1% FCS, and (v) culturing the cells for 5 weeks in said maturation medium.

The present application further describes an *in vitro* method for the generation of oligodendrocytes, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to a medium comprising T3, IGF, Forskolin, PDGF, and EGF, (v) culturing the cells for 2 weeks in the medium according to (iv), (vi) changing the medium to a medium comprising T3, IGF, Forskolin, PDGF, Dorsomorphin, (vii) culturing the cells for 1 week in the medium according to (vi), (viii) changing the medium to a medium comprising T3, IGF, and Forskolin, and (ix) culturing the cells for 3 weeks in the medium according to (viii).

The present application further describes an *in vitro* method for the generation of neural crest-derived neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a GSK-3 inhibitor, particularly Chir99021; an Alk5 inhibitor, particularly Alk5 inhibitor II, and BMP4; (iii) culturing the cells in the medium according to (ii) for 3 days, (iv) changing the medium to a medium comprising a GSK-3 inhibitor, particularly Chir99021; an FGF inhibitor, particularly SU5402, a Notch inhibitor, particularly DAPT, and NGF, (v) culturing the cells for 10 days in the medium according to (iv), (vi) changing the medium to a maturation medium comprising BDNF, GDNF and NGF, and (vii) culturing the cells for 3 weeks in the maturation medium according to (vi).

The present application further describes an *in vitro* method for the generation of cells having a mesenchymal stem cell phenotype, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a GSK-3 inhibitor, particularly Chir99021; an Alk5 inhibitor, particularly Alk5 inhibitor II, and BMP4; (iii) culturing the cells in the medium according to (ii) for 3 days, (iv) changing the medium to a medium comprising a GSK-3 inhibitor, particularly Chir99021; FGF8, IGF, and a Notch inhibitor, particularly DAPT, (v) culturing the cells for 7 days in the medium according to (iv), (vi) changing the medium to a maturation medium comprising bFGF and IGF, (vii) culturing the cells for 2 weeks in the maturation medium according to (vi), (viii) changing the medium to a mesenchymal stem cell medium, and (ix) culturing in said mesenchymal stem cell medium.

The present application further describes an *in vitro* method for the generation of cells having a mesenchymal stem cell phenotype, comprising the steps of (i) seeding iNBSCs on plates coated with a gelatinous protein mixture secreted by Engelbreth-Holm-Swarm mouse sarcoma cells (Matrigel),(ii) culturing the cells in 4 $\mu$M Chir99028, 10 ng/ml BMP4 and 10 $\mu$M DAPT for 7 days; (iii) culturing the cells in basal medium containing 10 ng/ml bFGF and 10 ng/ml IGF-1 for at least 5 passages; (iv) stabilizing the cells by switching the cultures to mesenchymal stem cell medium and culturing for at least 2 passages.

The present application further describes an *in vitro* method for the differentiation of cells having a mesenchymal stem cell phenotype into adipocytes, comprising the steps of (i) generating said cells having a mesenchymal stem cell phenotype by the *in vitro* method as described in the present application, (ii) changing the medium to a mesenchymal induction medium comprising 10% FCS; (iii) culturing the cells in the medium according to (ii) for 5 days, (iv) changing the medium to a adipogenesis differentiation medium, and (v) culturing the cells in the medium according to (iv).

The present application further describes an *in vitro* method for the differentiation of cells having a mesenchymal stem cell phenotype into chondrocytes, comprising the steps of (i) generating said cells having a mesenchymal stem cell phenotype by the *in* vitro method as described in the present application, (ii) changing the medium to a mesenchymal induction medium comprising10% FCS; (iii) culturing the cells in the medium according to (ii) for 5 days, (iv) changing the medium to a chondrocyte differentiation medium, and (v) culturing the cells in the medium according to (iv).

The present application further describes an *in vitro* method for the differentiation of cells having a mesenchymal stem cell phenotype into smooth muscle cells, comprising the steps of (i) generating said cells having a mesenchymal stem cell phenotype by the *in vitro* method as described in the present application, (ii) changing the medium to a mesenchymal induction medium comprising10% FCS; and (iii) culturing the cells in the medium according to (ii) for 3 to 5 weeks.

The present application further describes an *in vitro* method for the generation of a neural tube-like 3D culture, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) embedding of a single cell suspension of the cells cultured according to step (i) in a gelatinous protein mixture secreted by Engelbreth-Holm-Swarm mouse sarcoma cells (Matrigel) and adding a medium comprising SB and a hedgehog/smoothened agonist, particularly Purmorphamine; (iii) culturing said single cell suspension according to (ii) for 9 days; (iv) changing the medium to a medium comprising a GSK-3 inhibitor, particularly Chir99021, SB, a hedgehog/smoothened agonist, particularly Purmorphamine, and bFGF and (v) culturing for 4 days.

The present application further describes an *in vitro* method for the generation of a neural crest-like 3D culture, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) embedding of a single cell suspension of the cells cultured according to step (i) in a gelatinous protein mixture secreted by Engelbreth-Holm-Swarm mouse sarcoma cells (Matrigel) and adding a medium comprising a medium comprising a GSK-3 inhibitor, particularly Chir99021, an Alk5 inhibitor, particularly Alk5 inhibitor II, BMP4, and FGF2, and (iii) culturing for 12 days.

The present application further describes an *in vitro* method for the generation of cells representing a mutant phenotype, comprising the steps of (i) causing or allowing the modification of a gene sequence, the transcription or translation of a gene sequence, and/or of a protein encoded by a gene sequence of cells from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, or cells generated by the method as described in the present application.

The present application further describes an *in vitro* method for drug screening, comprising the step of exposing cells from an isolated (induced) neural border stem cell line as described in the present application, an isolated differentiated (induced) neural border stem cell line of the central nervous system lineage as described in the present application, an isolated central nervous system progenitor cell as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application to a drug substance.

The present application further describes a pharmaceutical composition comprising cells from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application.

The present application further describes a cell from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application for use in the treatment of a patient suffering from a neural disorder.

## FIGURES

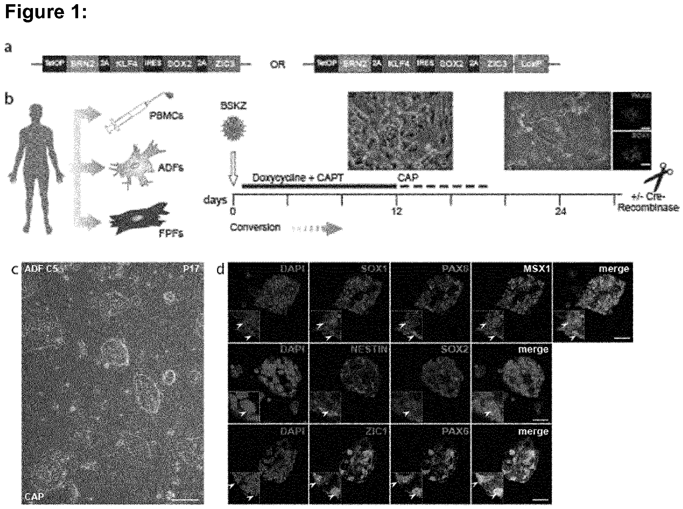

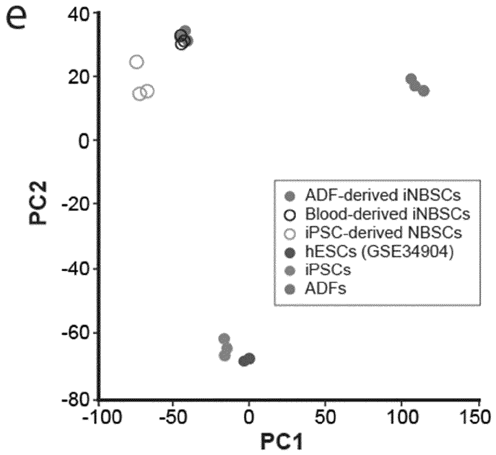

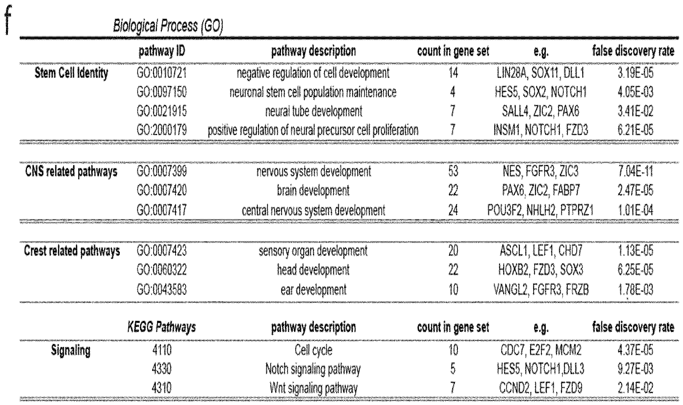

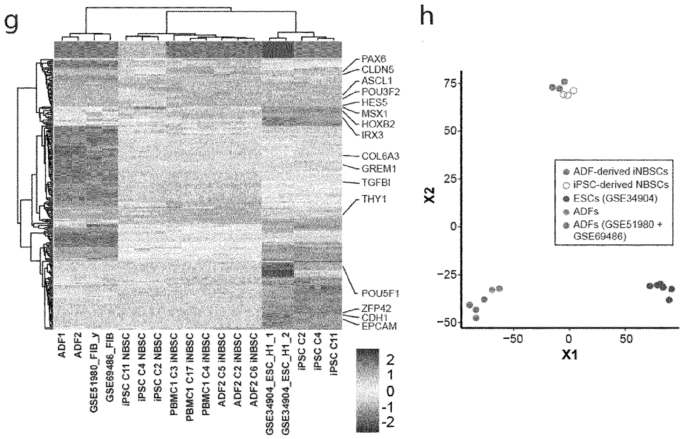

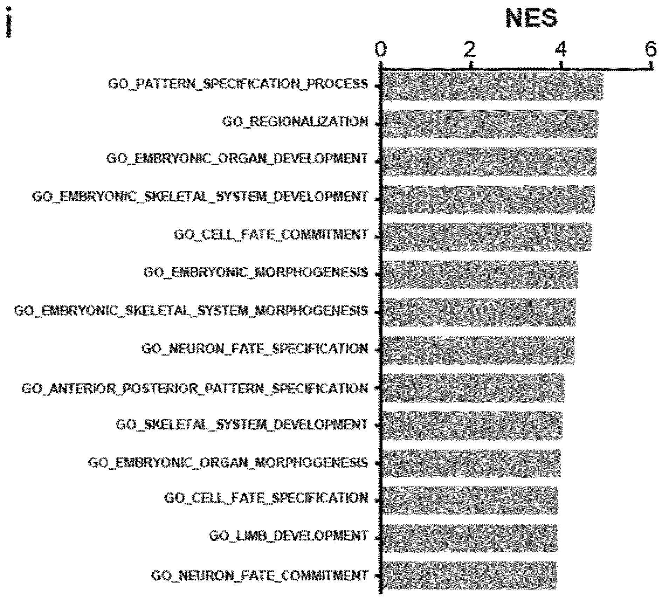

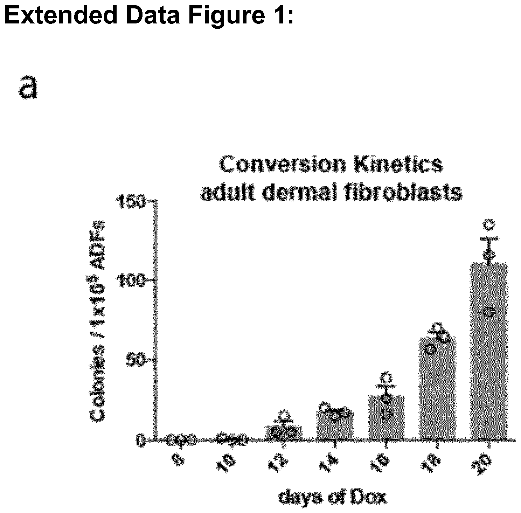

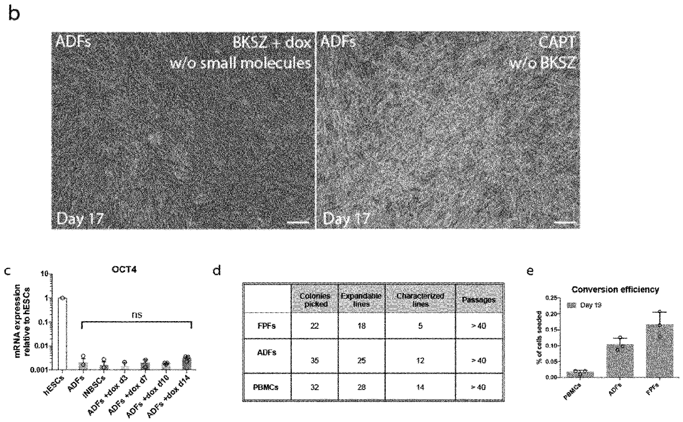

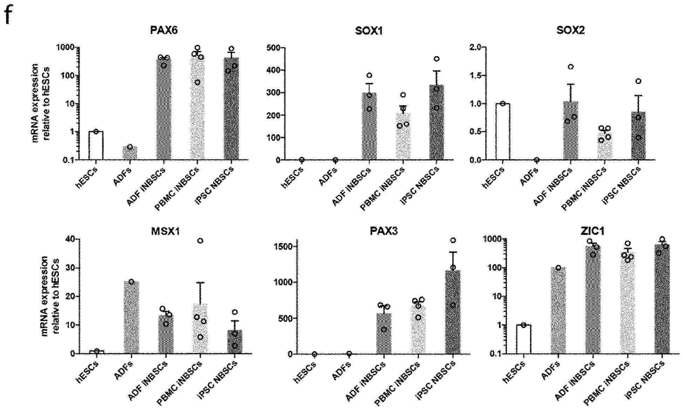

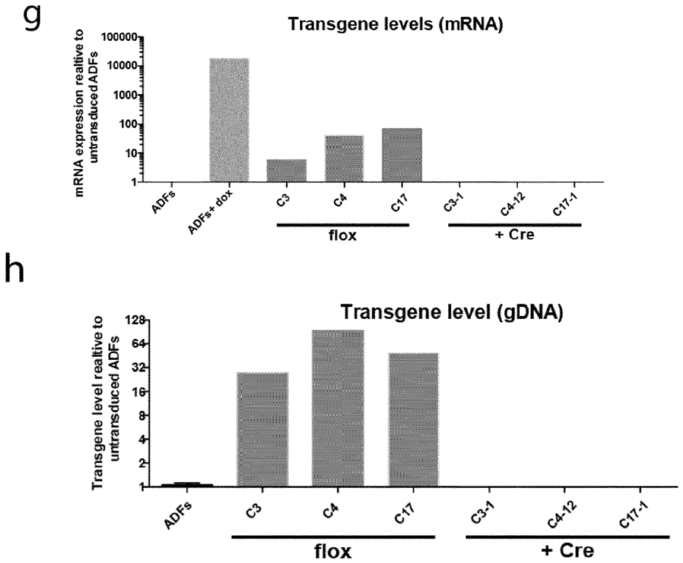

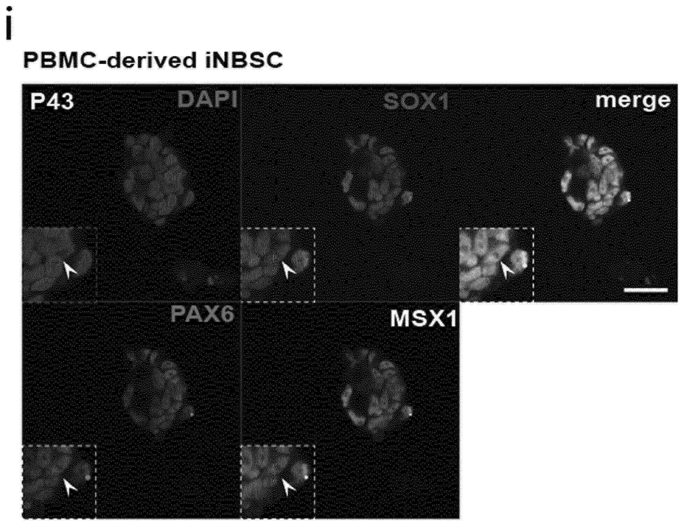

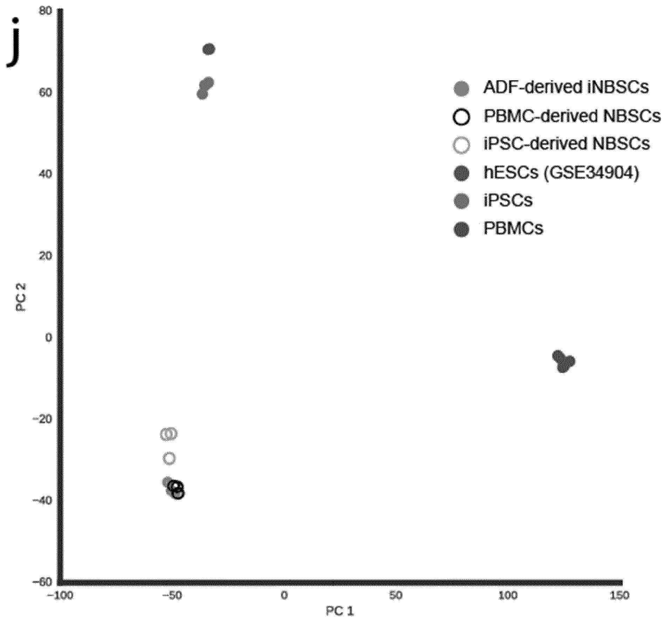

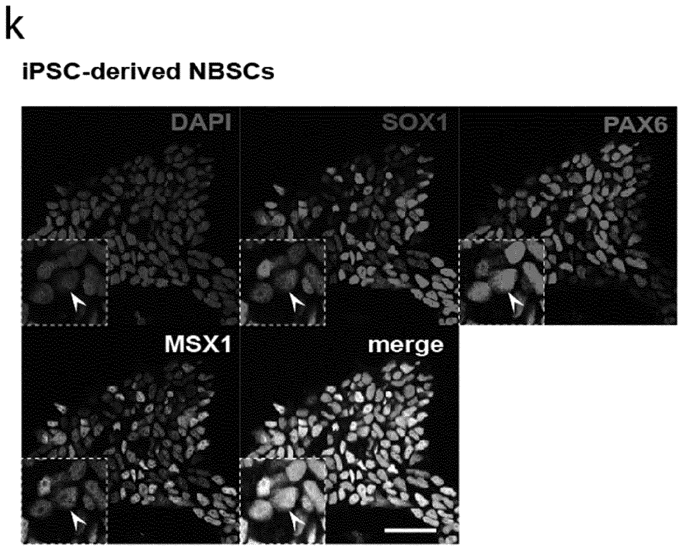

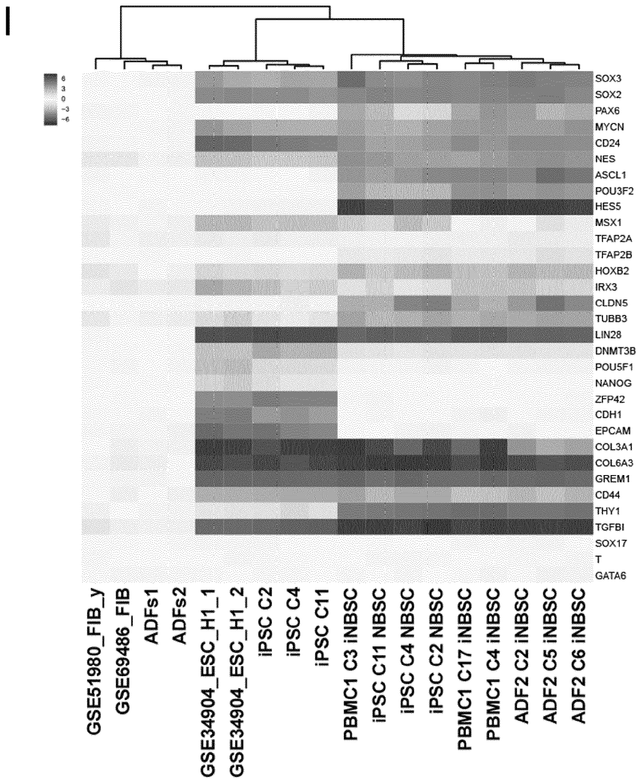

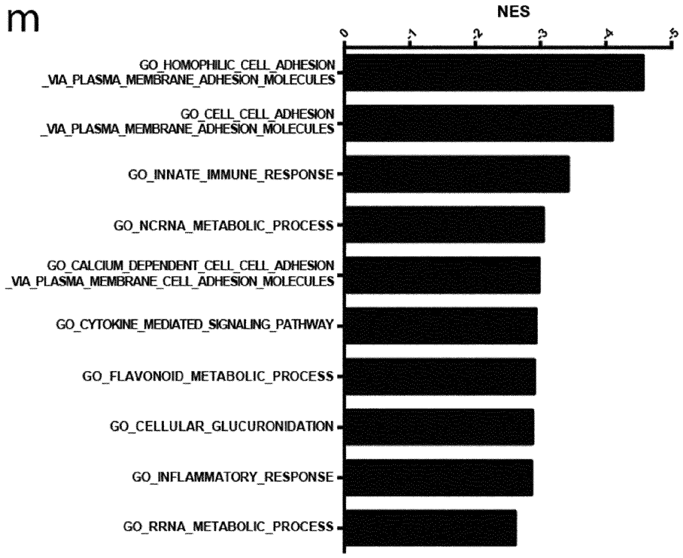

**Figure 1** shows the direct conversion of somatic cells into neural border stem cells. **a,** Reprogramming vectors used for neural conversion of somatic cells. **b,** A schematic overview of the conversion of somatic cells into neural progenitors. FPFs, ADFs and PBMCs were transduced with BKSZ transgenes and reprogramming was initiated one day after by addition of doxycycline (dox) and CAPT. From day 12 onwards dox was removed and conversions were switched to CAP supplemented medium. Picking of colonies was performed from day 21 onwards. Immunofluorescence pictures show staining for PAX6 and SOX1 of a representative colony before picking; scale bar 50 $\mu$m. **c,** BF image of a representative neural progenitor line derived from ADFs. **d,** Representative confocal images of an ADF-derived neural progenitor line staining positive for neural border and neural stem cell markers; arrow heads mark double- and triple-positive cells, respectively; scale bar 50 $\mu$m. **e,** Microarray-based transcriptional profiling of ADF-, Blood- and iPSC-derived (i)NBSCs, PSCs and ADFs. Principal component analysis shows three clearly distinct clusters, representing (i)NBSCs (ADF-, Blood-, iPSC-derived) and its somatic cell (ADF) and PSC (hESCs/iPSCs) ancestors, respectively. **f,** Gene Ontology (GO) processes based on 200 top upregulated genes in iNBSC lines compared to ADFs. **g,** Expression heatmap of iNBSCs, ADFs and PSCs based on the 200 probes showing highest contribution for the segregation of samples in the principal component analysis. Specific markers for iNBSCs, ADFs and PSCs are highlighted. **h,** Array-based methylation profiling of ADF-derived iNBSCs, PSCs and ADFs. Multidimensional scaling plot reveals distinct clusters of each population. i, Gene set enrichment analysis of hypomethylated promoter sites of iNBSCs compared to ADFs. Terms are ranked by normalized enrichment score (NES).

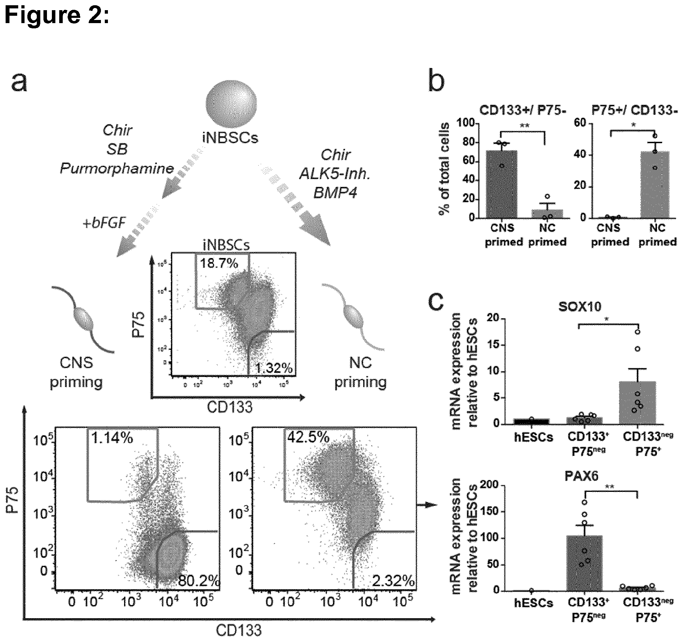

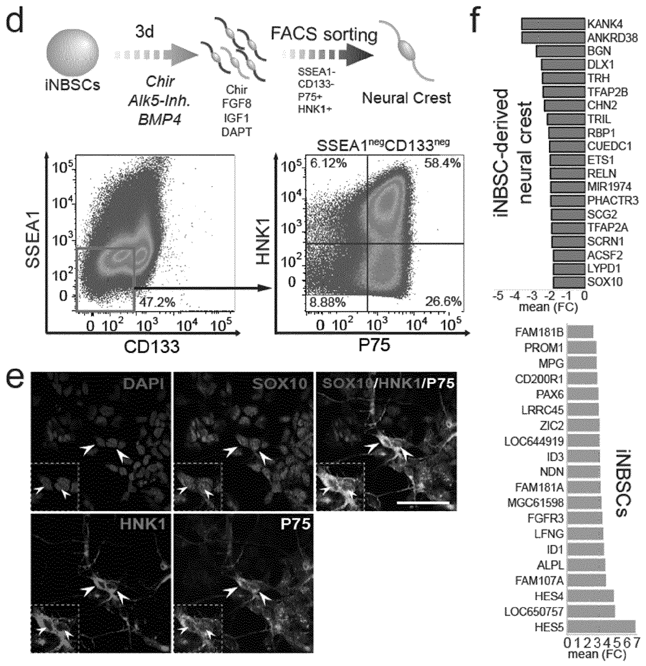

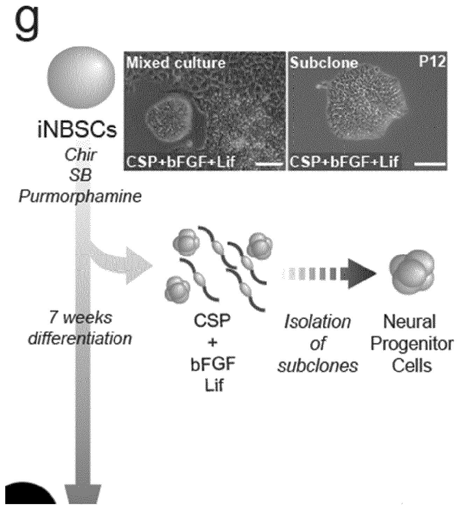

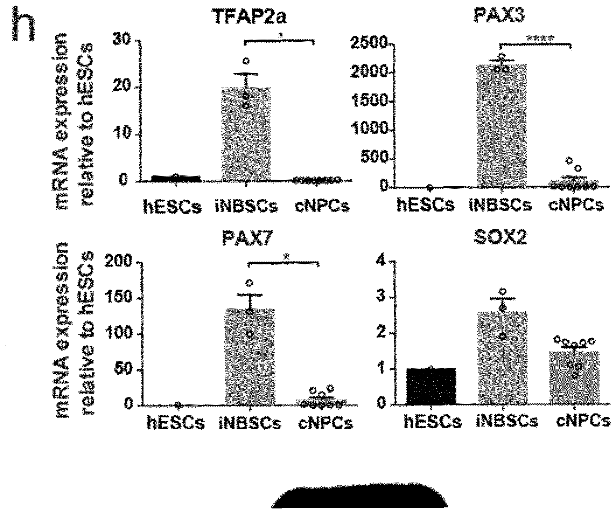

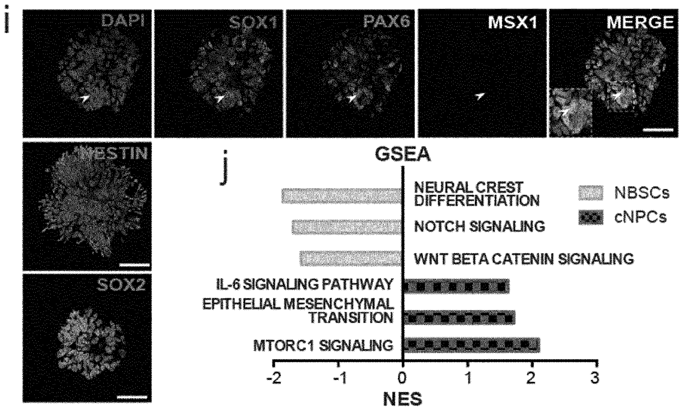

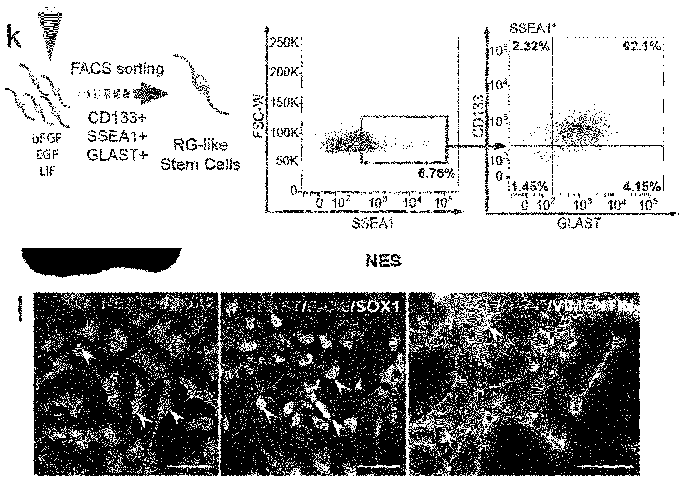

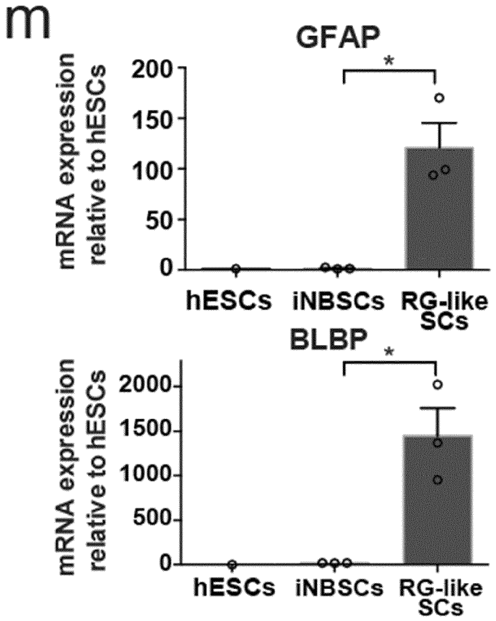

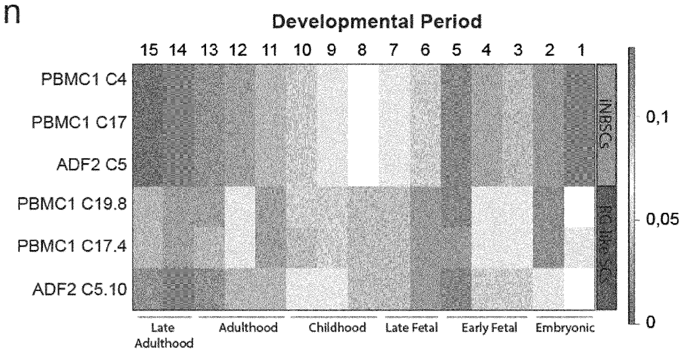

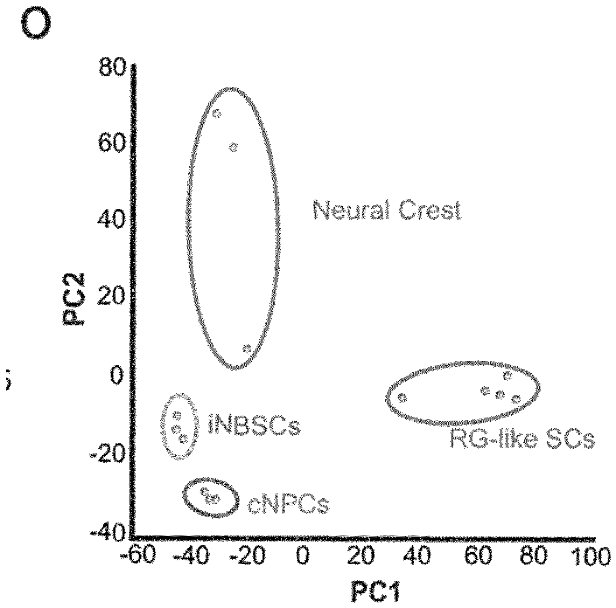

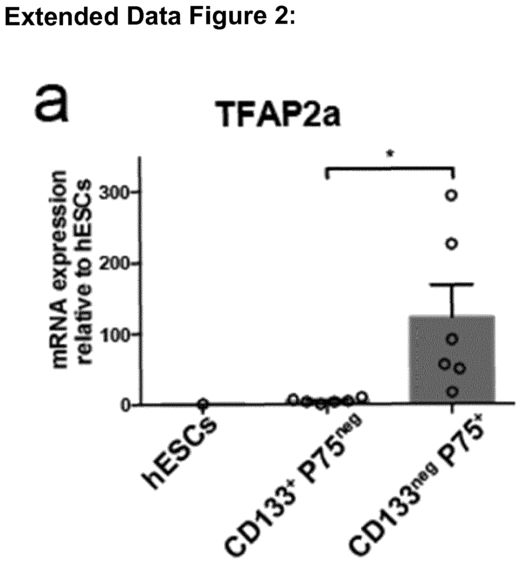

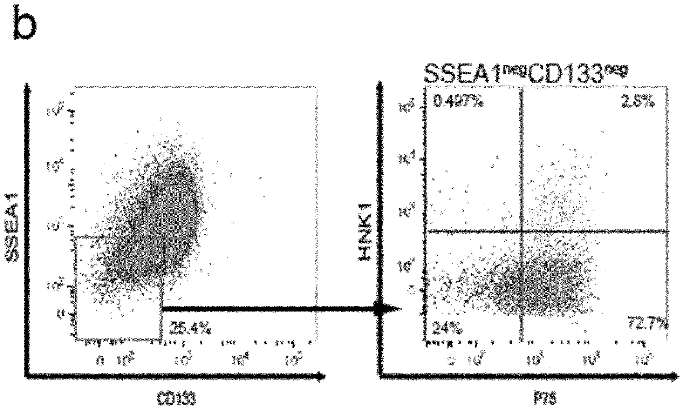

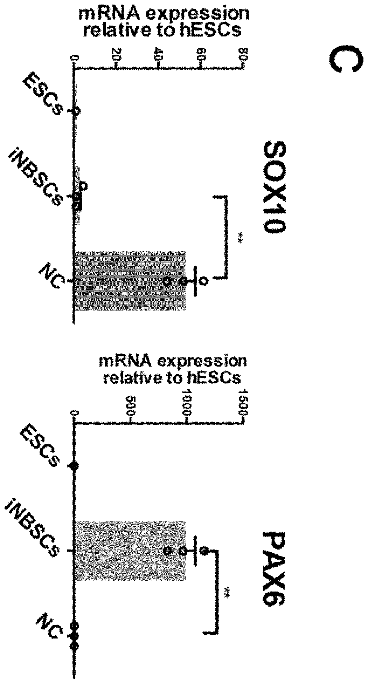

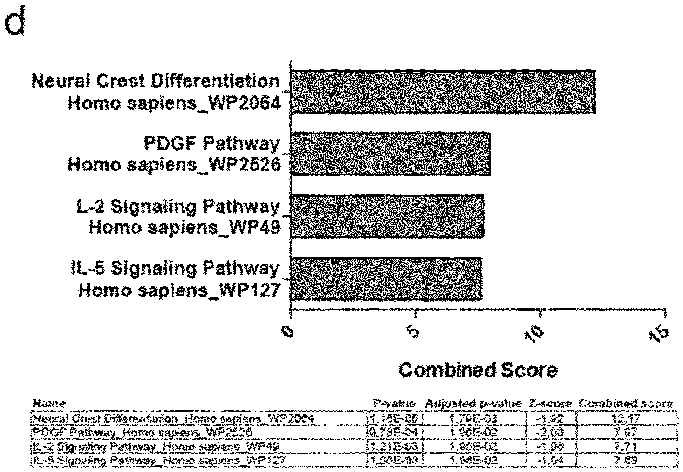

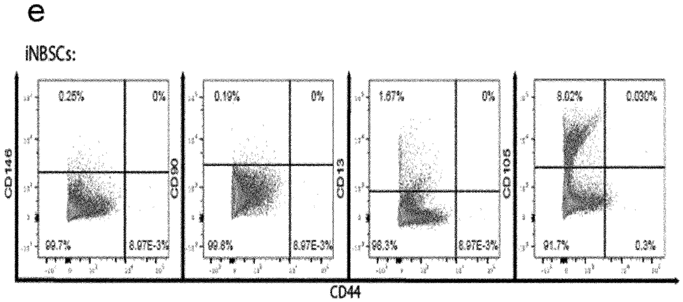

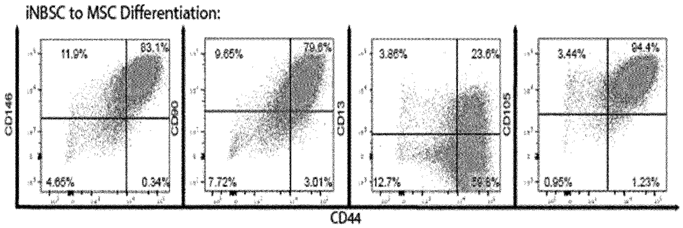

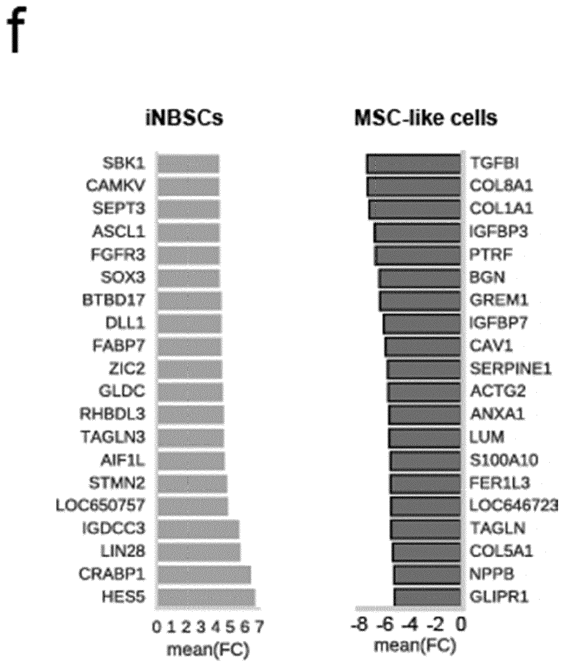

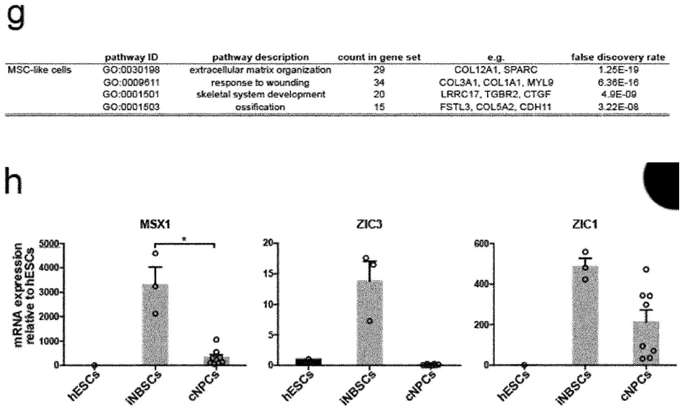

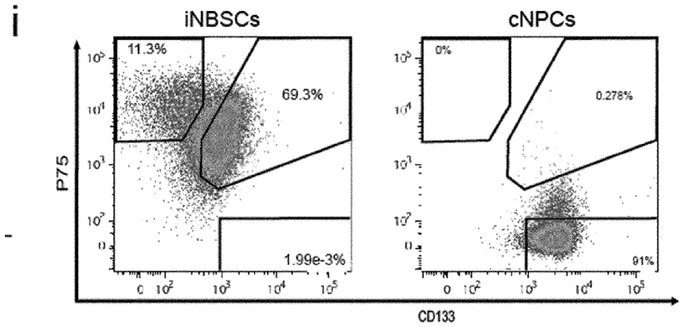

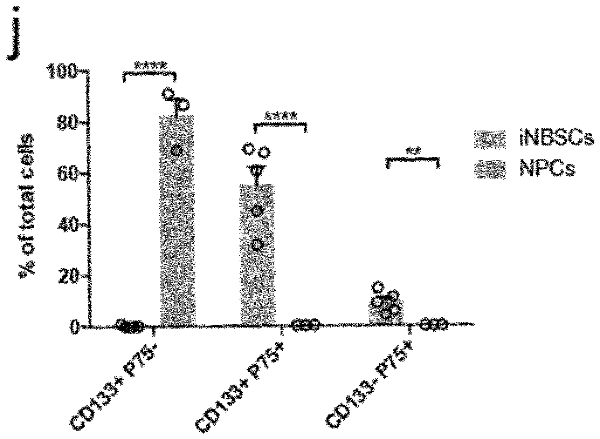

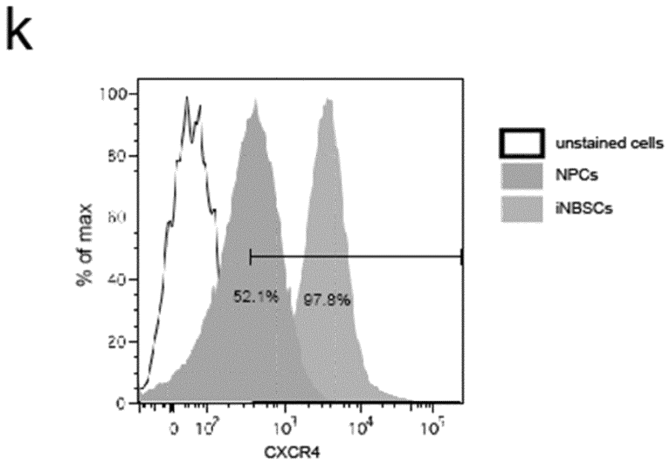

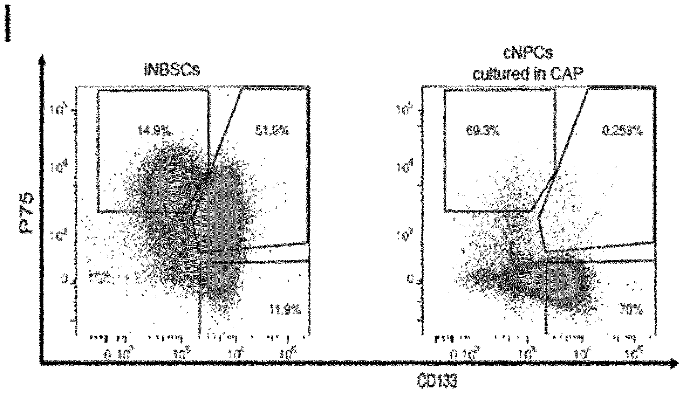

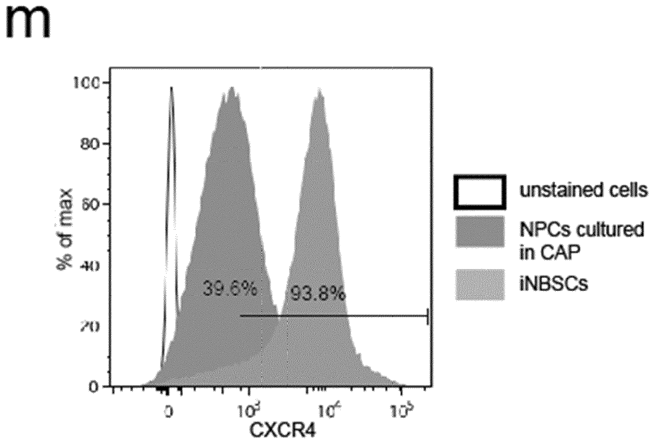

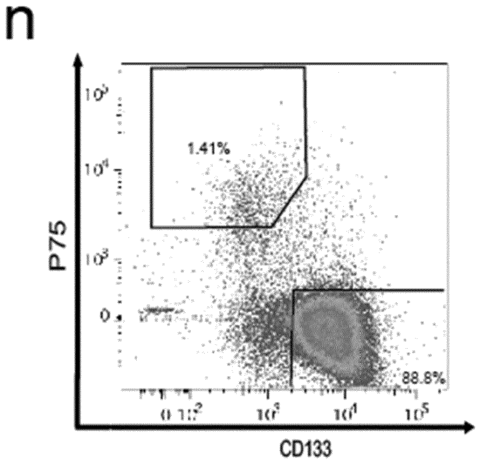

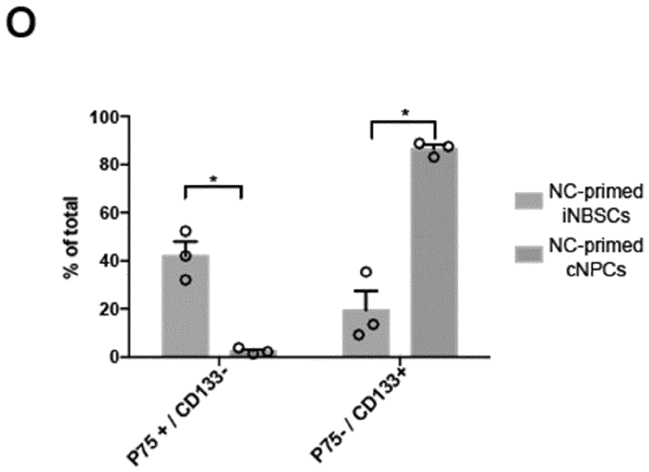

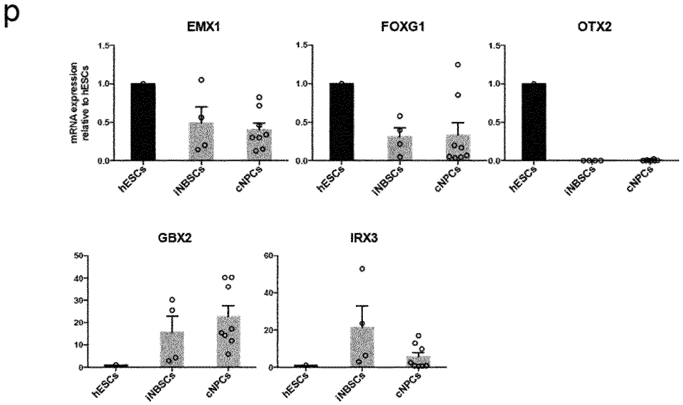

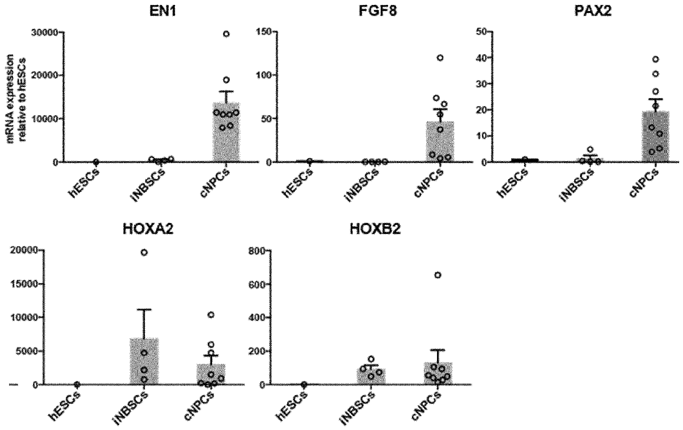

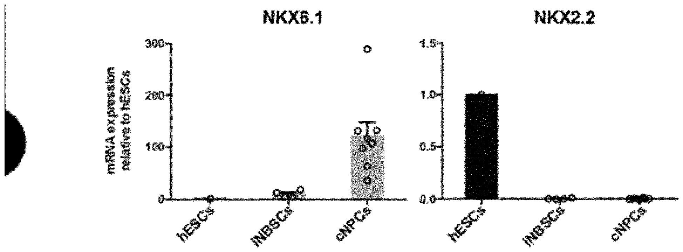

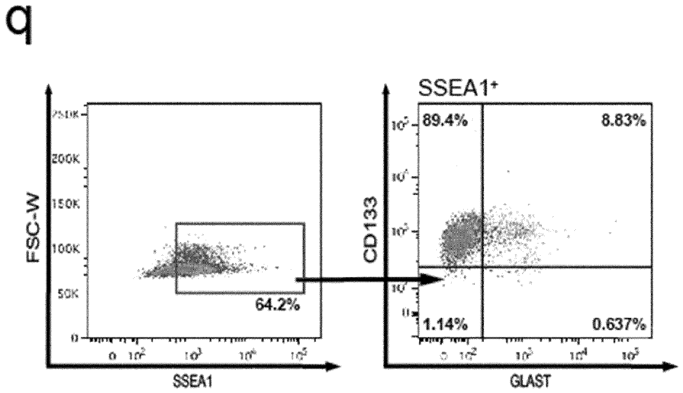

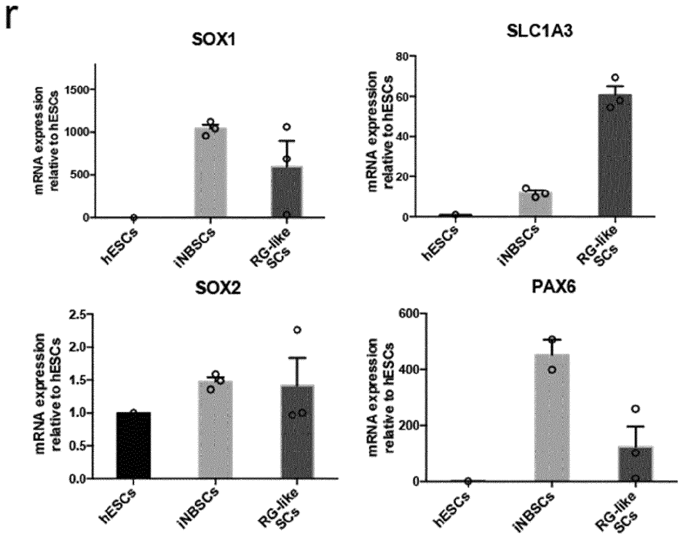

**Figure 2** shows that iNBSCs recapitulate early neural development and give rise to CNS and neural crest progenitors. **a,** Directed differentiation of iNBSCs towards CNS and NC fates. iNBSCs were cultured in medium supplemented with CSP and bFGF or CA and BMP4 for 5 days to direct differentiation towards CNS and NC lineages, respectively. Subsequently, cultures were analyzed by flow cytometry for expression of CD133 and P75. Gating scheme and representative results are shown for iNBSCs, CNS- and NC-primed cultures. **b,** Quantification of CD133$^+$/P75$^{neg}$ and P75$^+$/CD133$^{neg}$ population after CNS- and NC-priming of three independent iNBSC clones (t-test, *P<0.05, **P<0.01, Mean with SEM). c, mRNA expression of CNS and NC markers after CNS- or NC-priming of iNBSCs. CNS- and NC-primed cultures were FACS-sorted for CD133$^+$/P75$^{neg}$ or P75$^+$/CD133$^{neg}$ respectively and analyzed by qRT-PCR (n=6, t-test, *P<0.05, **P<0.01, Mean with SEM). **d,** Representative cytometry data after differentiation of iNBSCs towards NCSC-like cells. Cells were cultured in CAB for 3 days before switching to culture medium supplemented with Chir99028, FGF8, IGF and DAPT for another 7 days. Right panel shows SSEA-1$^{neg}$/CD133$^{neg}$ cell population. Cell sorting was performed on SSEA- $1^{neg}$/CD133$^{neg}$/P75$^+$/HNK1$^+$. **e,** Confocal images of stainings for neural crest markers on SSEA-1$^{neg}$/CD133$^{neg}$/P75$^+$/HNK1$^+$ sorted cells. **f,** Top DEGs (log2 FC>2) between SSEA-1$^{neg}$/CD133$^{neg}$/P75$^+$/HNK1$^+$ sorted neural crest (red) and iNBSCs (grey). **g,** Derivation of cNPCs from iNBSCs. Scheme and representative BF pictures of iNBSCs cultured in CSP with bFGF and LIF before (left) and after (right) derivation of stable cNPC subclones; scale bar 250 $\mu$m. **h,** Expression of neural border markers in independent iNBSC (n=3) and cNPC (n=8) lines. mRNA expression was analyzed by qRT-PCR and normalized to hESCs (t-test, *P<0.05, ****P<0.0001, Mean with SEM). **i,** Confocal images of neural progenitor markers in cNPCs. Arrowheads (upper panel) indicate SOX1/ PAX6 double-positive cells; note absence of MSX1; scale bar 50 $\mu$m. **j,** Selection of processes generated by gene set enrichment analysis (GSEA) of cNPCs (blue) and iNBSCs (grey). **k,** Derivation of RG-like stem cells from iNBSCs. iNBSCs were cultured for 7 weeks in neuronal differentiation medium before addition of bFGF, EGF and LIF to the medium. Subsequently cultures were FACS sorted for CD133$^+$/SSEA1$^+$/GLAST$^+$ triple positive cells to enrich for RG-like SCs. FACS plot shows a representative differentiation prior to FACS sorting. **l,** Immunofluoresence stainings for radial glia markers on iNBSC-derived RG-like SCs; scale bar 50 $\mu$m. **m,** mRNA expression of radial glia-associated markers in independent RG-like SCs (n=3) and iNBSCs (n=3); (t-test, *P<0.05, Mean with SEM). **n,** Temporal identity of RG-like SCs and iNBSCs. Expression data of iNBSCs and RG-like stem cells (CD133$^+$/SSEA1$^+$/GLAST$^+$) were analyzed by machine learning framework (CoNTExT) to match data with transcriptome atlas of the developing human brain. **o,** Transcriptional landscape of iNBSCs and their progeny. PCA of iNBSCs, cNPCs, neural crest (SSEA-1$^{neg}$/CD133$^{neg}$/P75$^+$/HNK1$^+$) and RG-like SCs (CD133$^+$/SSEA1$^+$/GLAST$^+$) reveals four distinct clusters with increasing distance in the process of development.

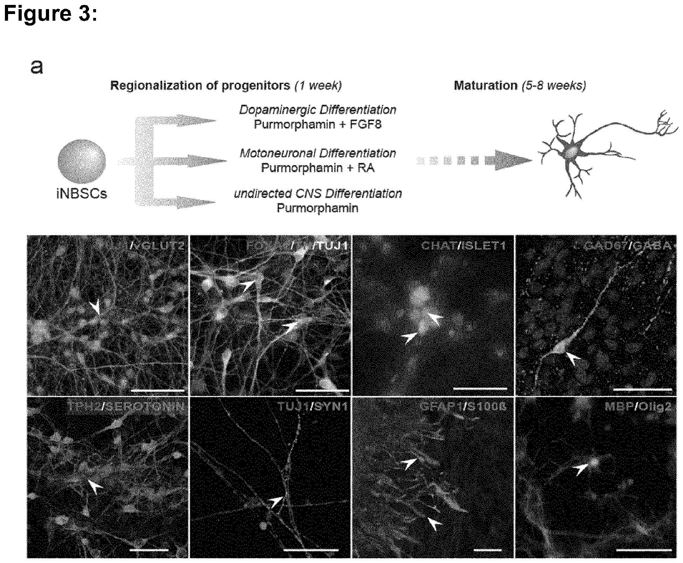

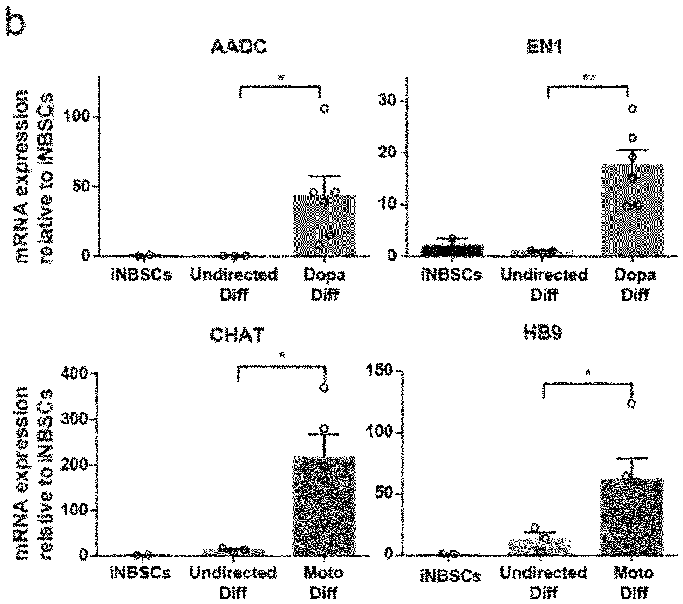

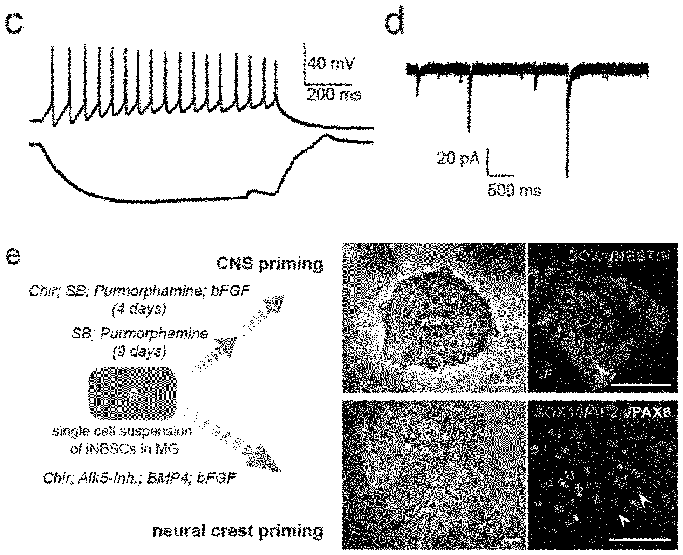

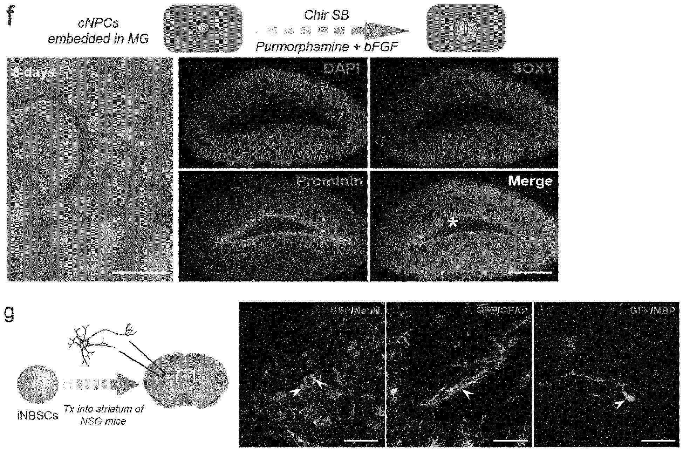

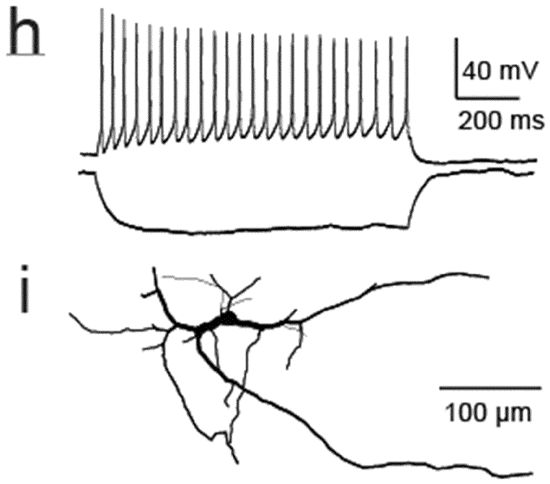

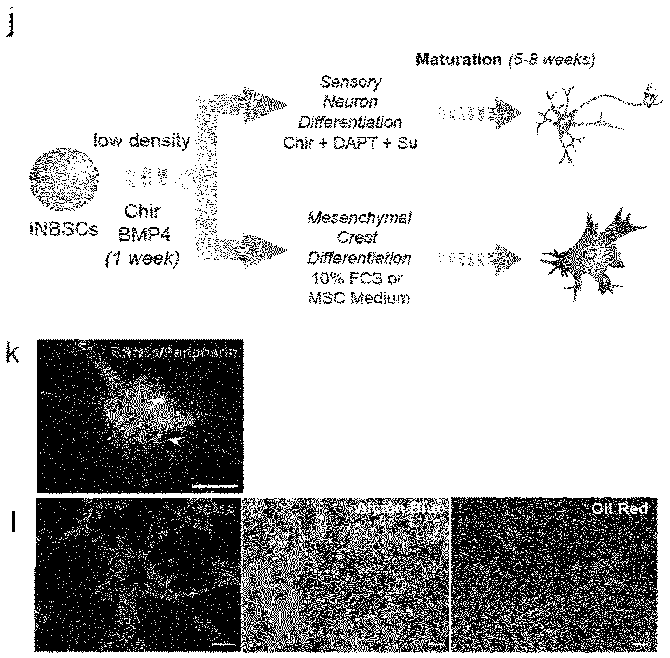

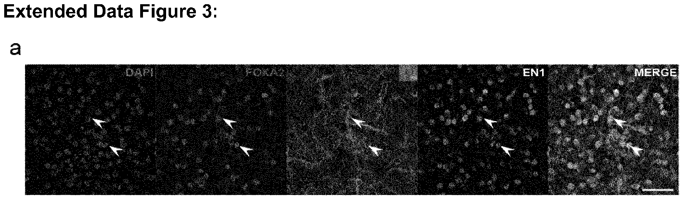

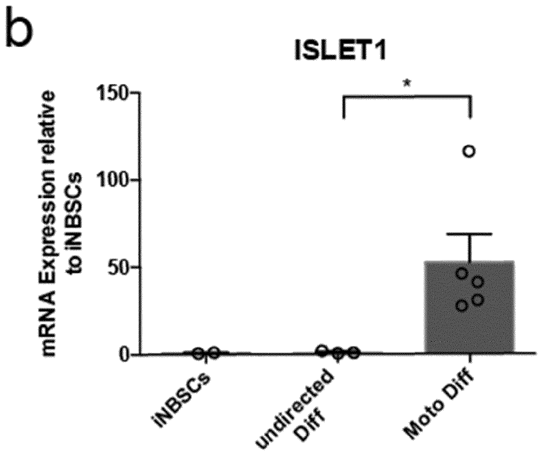

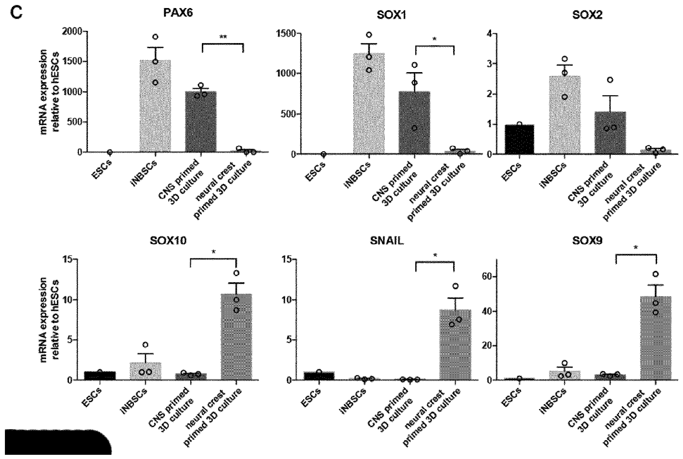

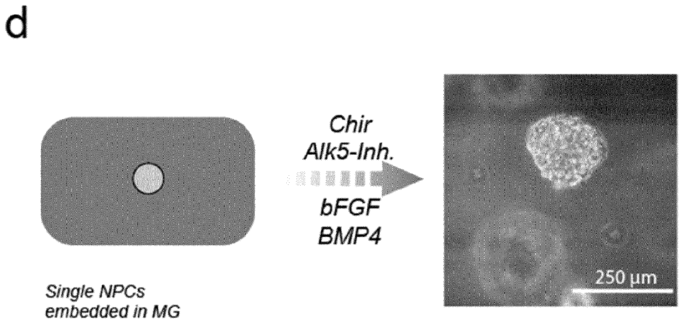

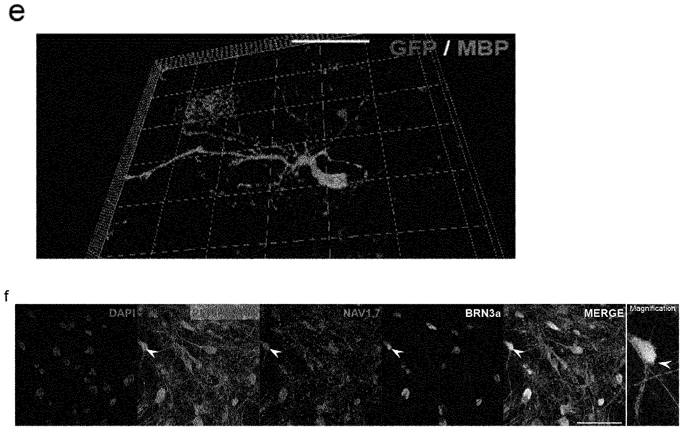

**Figure 3** shows the differentiation of iNBSCs into mature CNS and neural crest progeny. Immunofluorescence pictures of iNBSCs differentiated into various neuronal and glial subtypes. Stainings were performed after >6 weeks (neuronal stainings) or >10 (glia stainings) weeks of differentiation. Arrowheads indicate glutamatergic neurons, dopaminergic neurons, motoneurons and gabaergic neurons (left to right, upper panel), as well as serotonin neurons, synapse formation, astrocytes and oligodendrocytes (left to right, lower panel), respectively. Scale bar 50 $\mu$m. **b,** mRNA expression of subtype specific neuronal markers. iNBSCs were directed towards dopaminergic (green, Dopa Diff) or motoneural fate (blue, Moto Diff) and matured for >7 weeks before qRT-PCRs were performed. Marker expression is presented for an undirected and a directed (Dopa/ Moto) differentiation protocol (t-test, *P<0.05, **P<0.01, Mean with SEM). **c,** Electrophysiological properties of a >6 weeks *in vitro* differentiated neuron. Repetitive trains of action potentials in response to depolarizing voltage steps, **d,** Spontaneous post-synaptic currents of a >6 weeks *in vitro* differentiated neuron. e, iNBSCs form neural tube-like structures and migratory crest-like cells upon 3D culture. iNBSCs were embedded as single cell suspension in MG and directed towards CNS or neural crest fate. Upon CNS priming neural tube-like structures could be observed (upper BF image, scale bar 100 $\mu$m); neural crest priming resulted in cells migrating throughout the matrix (lower BF image, scale bar 100 $\mu$m). Arrowheads indicate representative SOX1/NESTIN double positive neuroepithelial structures and SOX10$^+$/AP2a$^+$/PAX6$^{neg}$ cells after CNS- and NC-priming, respectively; scale bar 50 $\mu$m. **f,** cNPCs form neural tube-like structures upon differentiation in 3D. cNPCs were embedded as single cell suspension in MG and differentiated under CNS priming conditions. BF image shows a representative section of a 3D culture after 8 days of differentiation; scale bar 250 $\mu$m. Representative neural tube-like structure stained positive for neuroepithelial markers SOX1 and CD133; asterisk marks luminal distribution of CD133. Scale bar 50 $\mu$m. **g,** iNBSCs give rise to all three major neural lineages *in vivo.* GFP transduced iNBSCs were differentiated for 8 days, followed by transplantation into the striatum of 8 weeks old NOD.*Prkdc$^{scid}$.Il2rg$^{null}$* (NSG) mice. Mice were analyzed 7-8 weeks post transplantation. Arrowheads in confocal images mark co-staining of iNBSC progeny (GFP) and markers for neurons (NeuN), astrocytes (GFAP) and oligodendrocytes (MBP). **h,** Electrophysiological properties of >6 weeks *in vivo* differentiated neurons. Repetitive trains of action potentials in response to depolarizing voltage steps, **i,** Spontaneous post-synaptic currents of >6 weeks *in vivo* differentiated neurons, **j,** Morphological reconstruction of transplanted neuronal progeny. GFP-positive neurons were filled with biocytin, identified via 3,3'-diaminobenzidine staining and reconstructed using the Neurolucida tracing program; scale bar 200 $\mu$m. **k,** Immunofluorescence pictures of iNBSCs differentiated into peripheral neurons. Arrowheads indicate sensory neurons (BRN3a/ Peripherin douple positive); scale bar 50 $\mu$m. **l,** Mesenchymal crest differentiation of NC-primed iNBSC. Stainings of mesenchymal crest differentiations reveal smooth muscle cells (SMA), chondrocytes (Alcian blue) and adipocytes (Oil red); scale bar 100 $\mu$m (SMA) and 250 $\mu$m (Alcian blue/ Oil red).

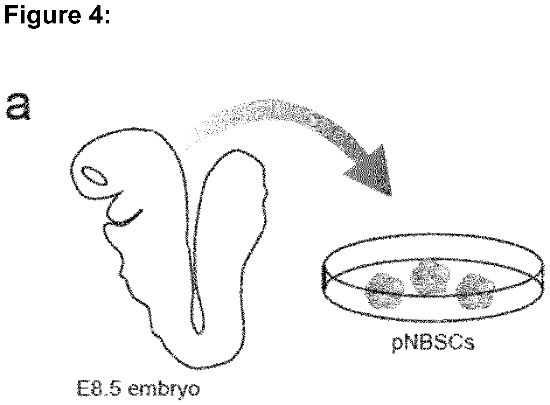

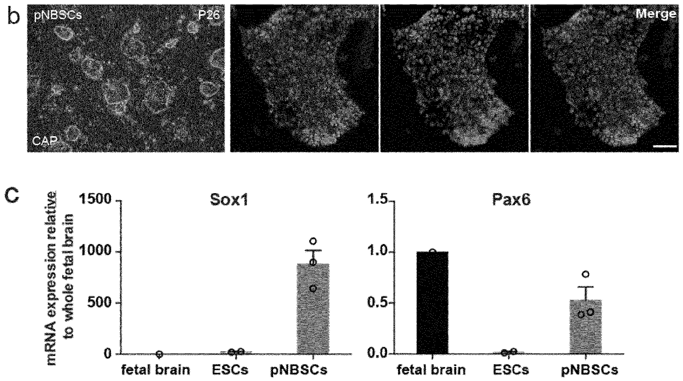

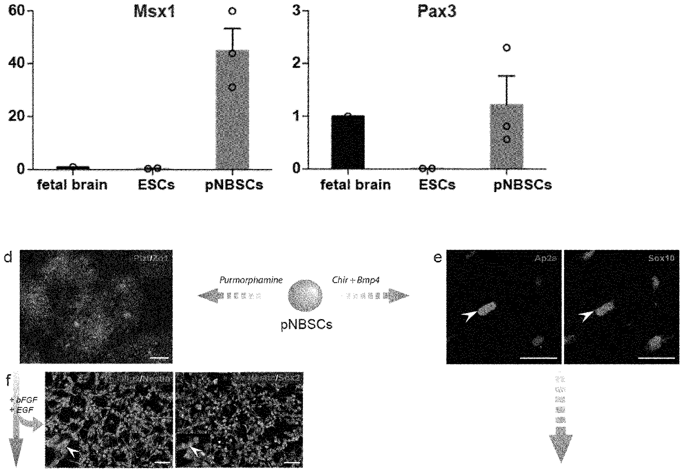

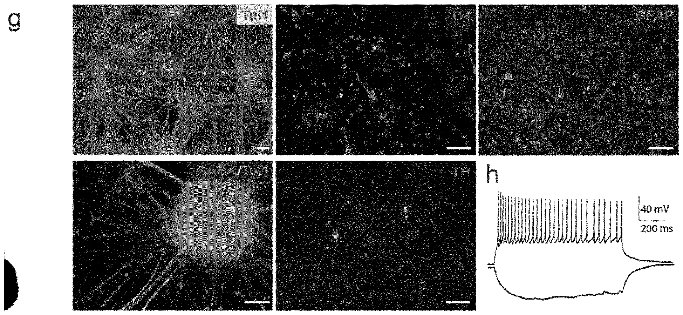

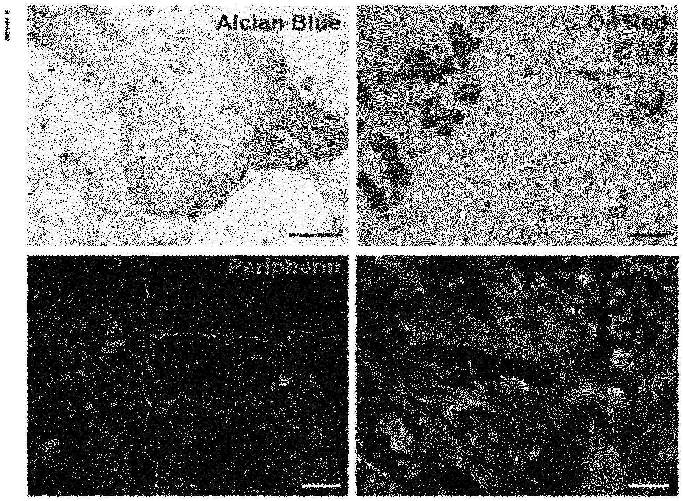

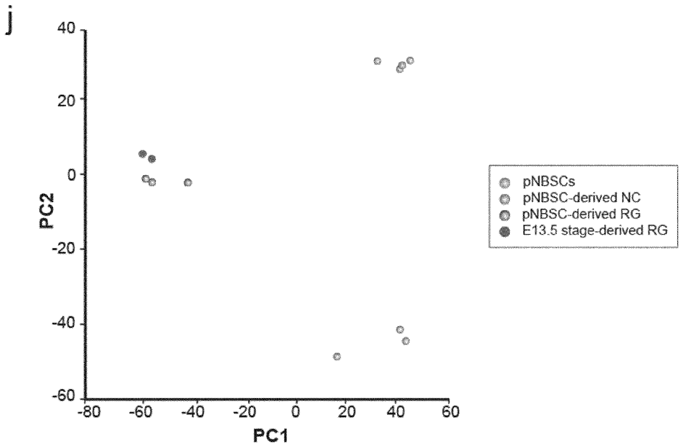

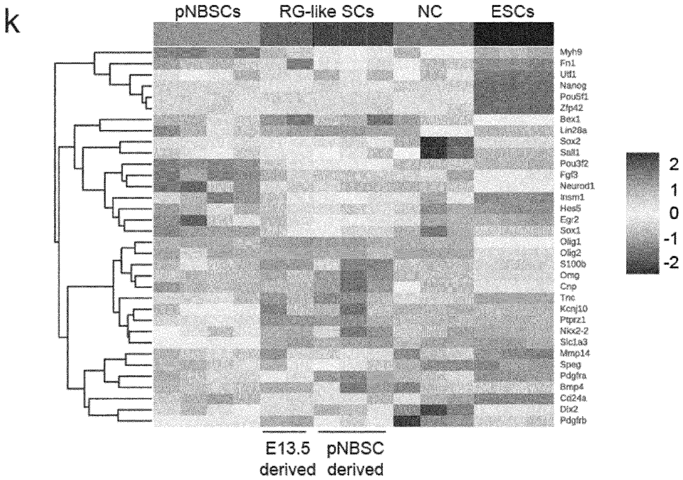

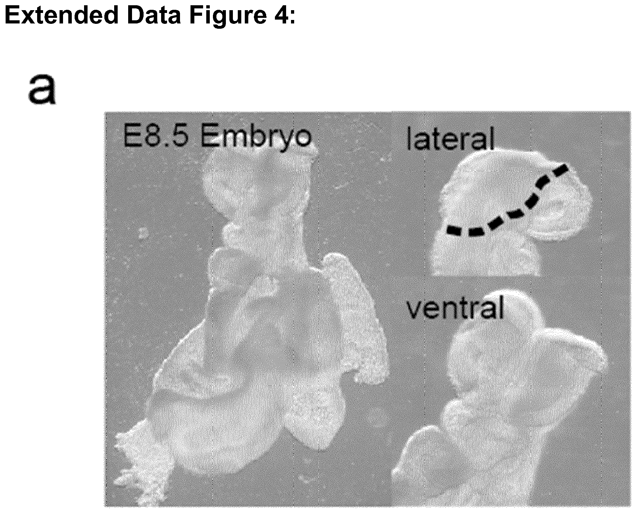

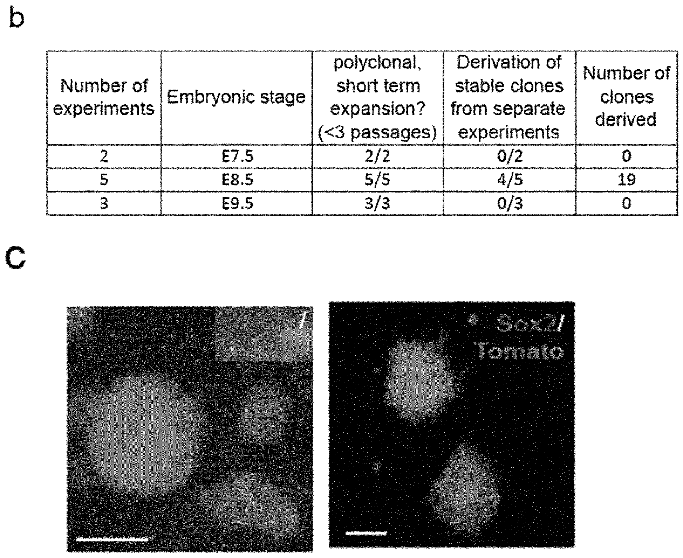

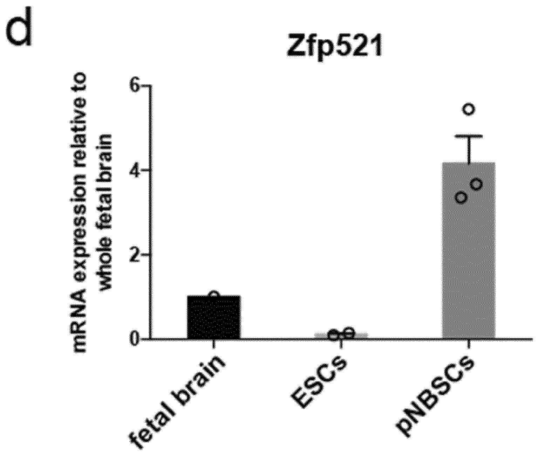

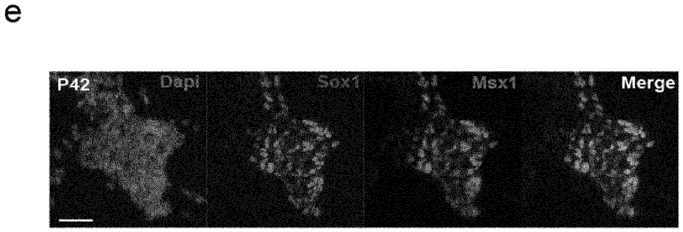

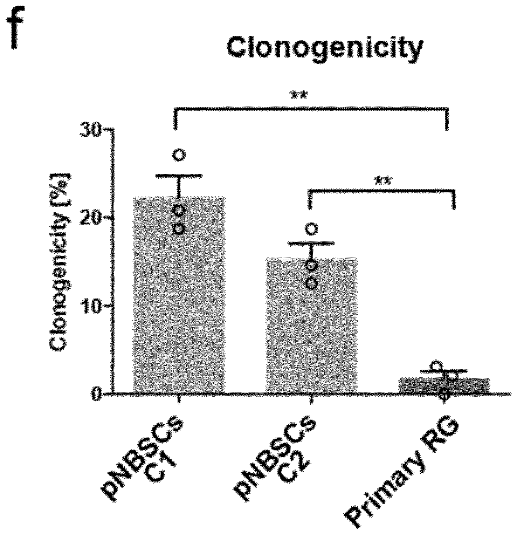

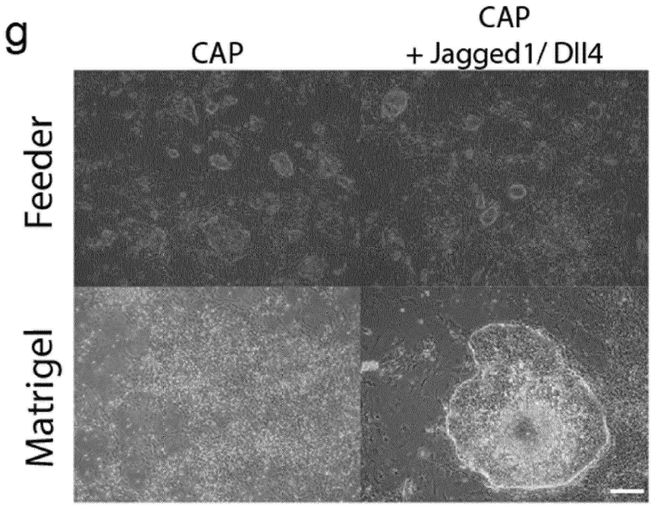

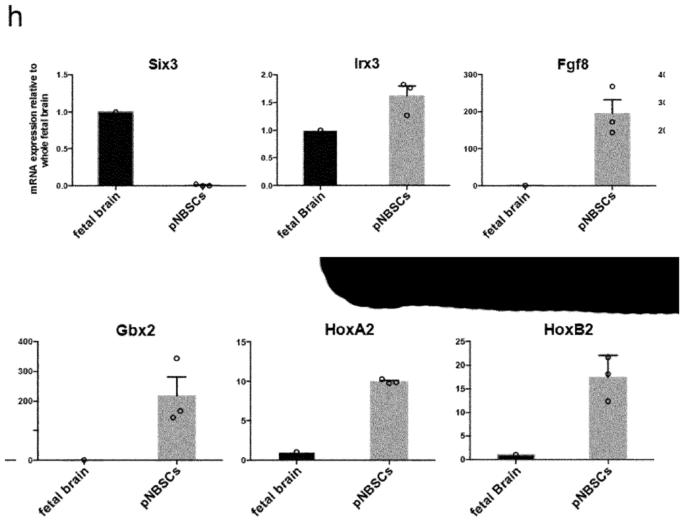

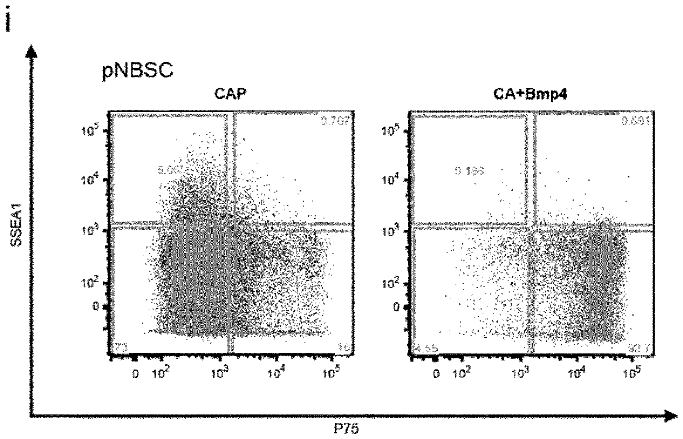

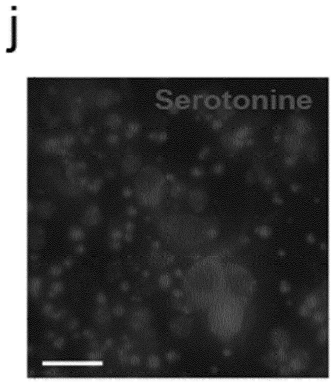

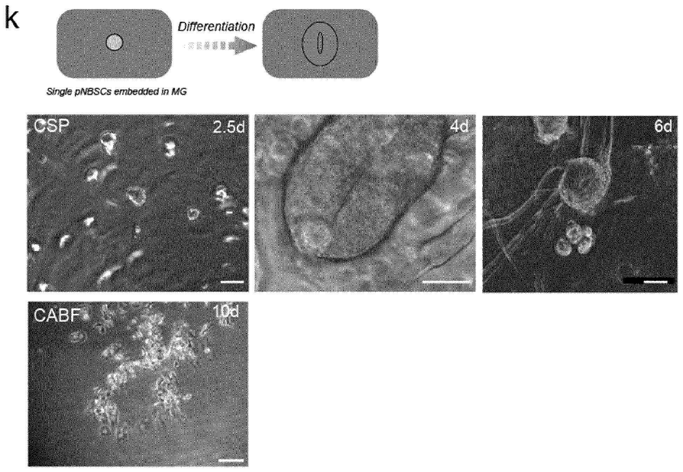

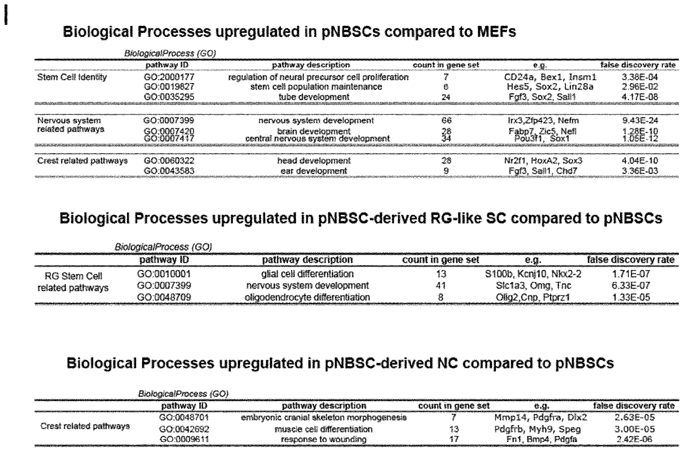

**Figure 4** shows the derivation of primary Neural Border Stem Cells from mouse embryos. **a,** Scheme for the derivation of pNBSCs. **b,** BF image and immunofluorescence stainings of a representative neural progenitor line derived from E8.5 stage embryos. BF picture shows representative morphology after 9 weeks of culture (P26). Immunofluorescence stainings for neural progenitor (Sox1) and border (Msx1) markers. Scale bar 50 $\mu$m. **c,** mRNA expression of neural progenitor and neural border markers in pNBSCs and controls. qRT-PCRs were performed on three independent pNBSCs lines and normalized to whole E13.5 stage fetal brains; mouse ESCs served as negative control. **d,** Immunofluorescence stainings of CNS-primed pNBSCs. pNBSCs were differentiated in presence of Purmorphamine for 3 days and stained for rosette stage markers Plzf, Zo1; scale bar 100 $\mu$m. **e,** Immunofluorescence stainings of neural crest primed pNBSCs. pNBSCs were differentiated in presence of Chir99028 and BMP4 for 2 days and co-stained for neural crest stem cell markers Ap2a and Sox10; scale bar 50 $\mu$m. **f,** Stabilization of radial glia-like stem cells from pNBSCs. CNS-primed pNBSCs were stabilized in radial glia-like stage by culture in medium supplemented with bFGF and EGF. Arrowheads indicate co-expression of radial glia and stem cell markers Olig2/ Nestin and Sox2/ Nestin; scale bar 50 $\mu$m. **g,** Immunofluorescence pictures of pNBSCs differentiated into various neuronal and glial subtypes. Stainings were performed after >2 weeks of differentiation. pNBSC showed high neurogenic potential (Tuj1) and also gave rise to oligodendrocytes (O4) and astrocytes (GFAP). Stainings for Gaba and Th reveal differentiation into specific subtypes; scale bar 100 $\mu$m. **h,** Electrophysiological properties of >3 weeks *in vitro* differentiated neurons. Repetitive trains of action potentials in response to depolarizing voltage steps, i, Differentiation of pNBSCs into neural crest derivates. Stainings of neural crest differentiations reveal chondrocytes (Alcian blue), adipocytes (Oil red), peripheral neurons (Peripherin), and smooth muscle cells (SMA); scale bar 100 $\mu$m. **j,** Microarray-based transcriptional profiling of pNBSCs, pNBSC-derived neural crest, pNBSC-derived radial glia-like stem cells (RG-like SCs), and E13.5 stage-derived RG-like SCs. Principal component analysis shows three clearly distinct clusters, representing pNBSCs and its NC and RG-like SC progeny. Note clustering of E13.5 stage-derived RG-like SCs together with pNBSCs-derived RG-like SCs. **k,** Gene expression heatmap of genes enriched for by GO analysis. Heatmap shows a selection of genes related to GO terms enriched in pNBSCs, its neural crest and RG-like SC progeny as well as selection of pluripotency markers (see also Suppl. Fig. 4I). E13.5 stage-derived RG SCs and ESCs serve as controls.

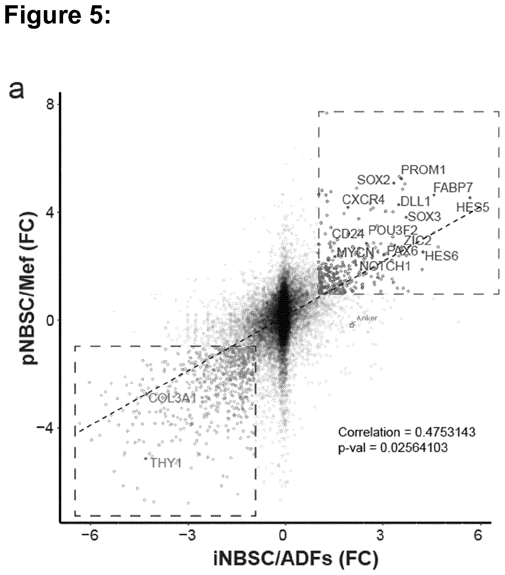

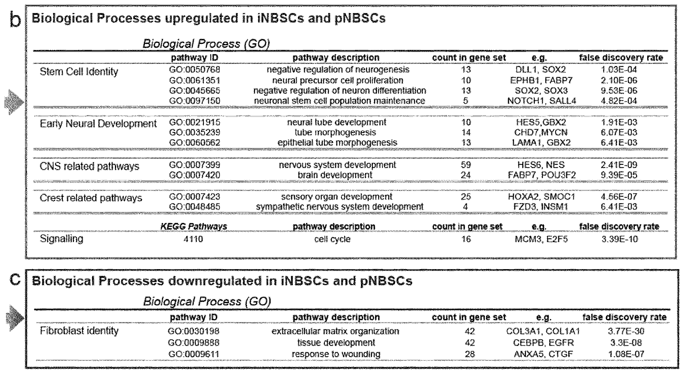

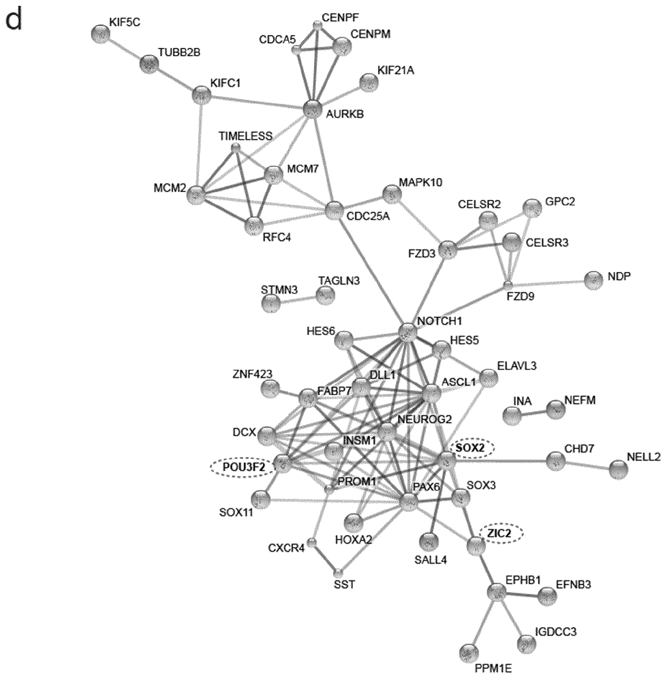

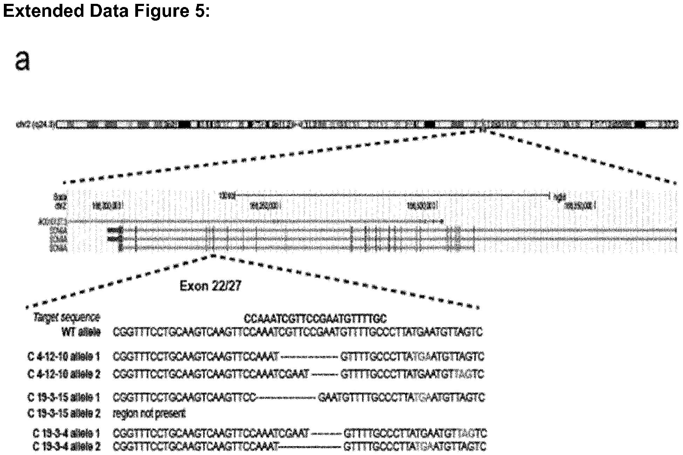

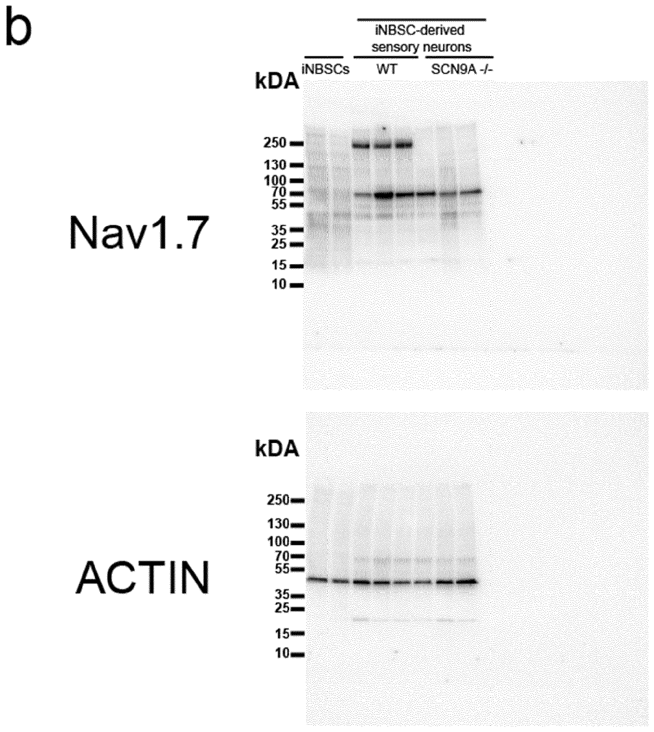

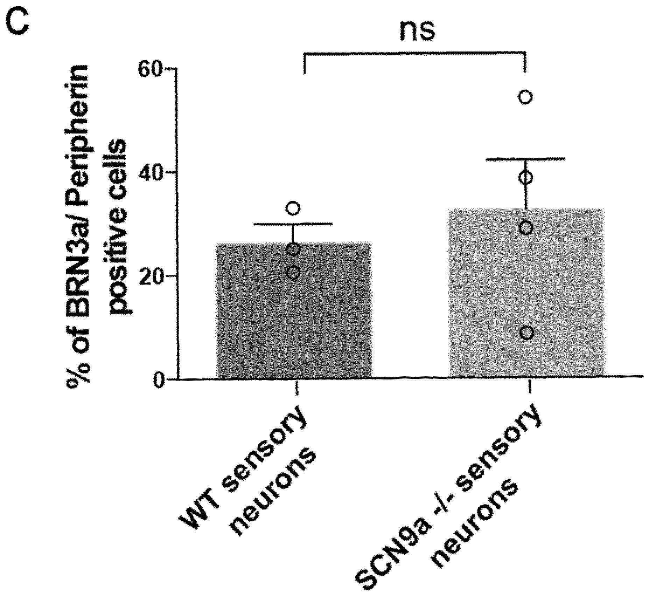

**Figure 5** shows a comparison of iNBSCs, primary mouse NBSCs and primary human progenitors. **a,** Comparison of human iNBSC and mouse pNBSC expression data. Differential gene expression of iNBSCs vs ADFs and pNBSCs vs MEFs was evaluated for similarity applying the agreement of differential expression procedure (AGDEX). The analysis shows a positive correlation of 0.457 (permutated p-value of 0.0256). **b,** Shared biological processes enriched in iNBSCs and pNBSCs. Gene Ontology processes based on upregulated genes with log 2 fold change >1 (258 genes) in iNBSC and pNBSCs lines. c, Biological processes downregulated in iNBSCs and pNBSCs compared to ADFs and MEFs. Gene Ontology processes based on shared 200 top downregulated genes in iNBSC and pNBSCs lines. **d,** Shared core-network of iNBSCs and pNBSCs. Network analysis was performed on shared upregulated genes of iNBSCs and pNBSCs with a log 2 fold change >2 (74 Genes) using the STRING v10 web interface; only connected nodes of the network are displayed.

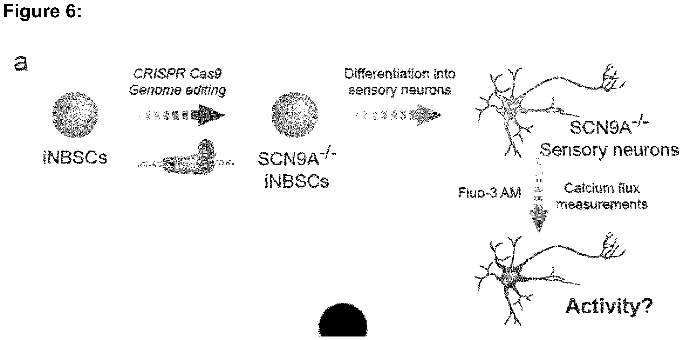

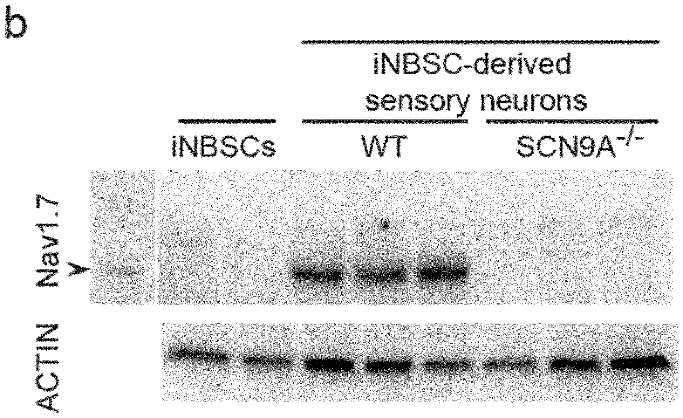

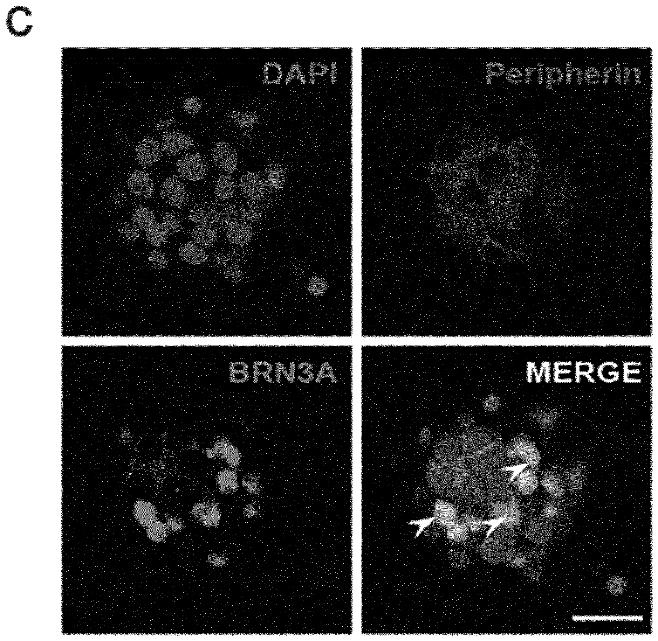

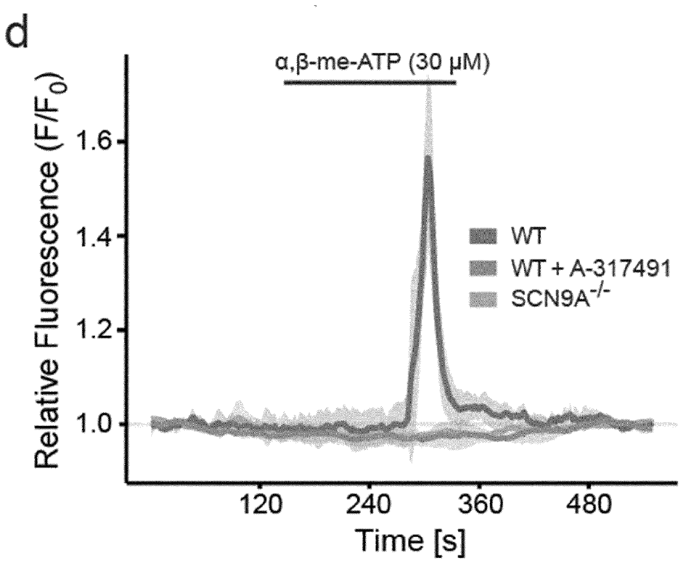

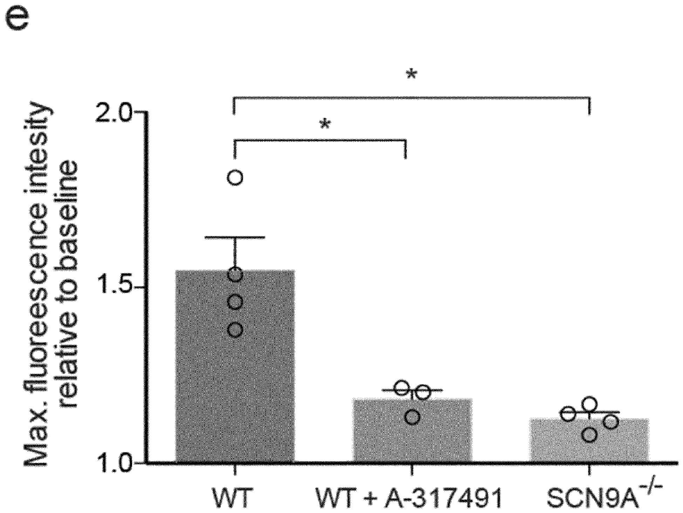

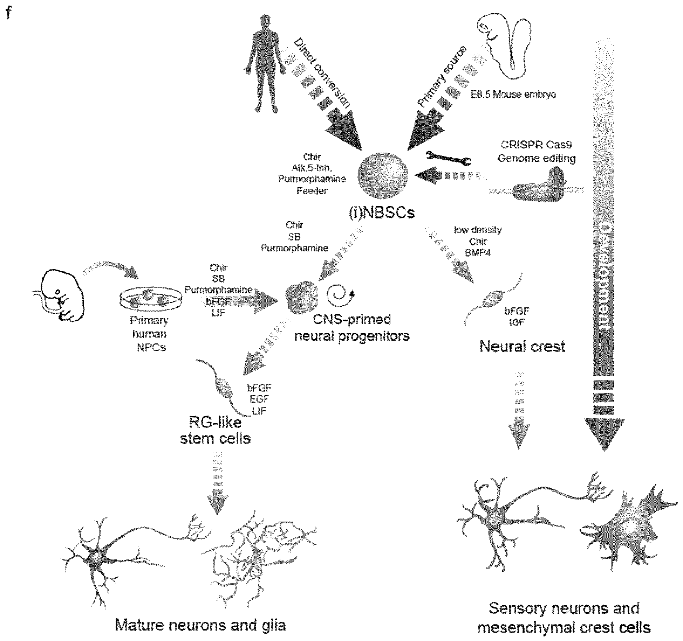

**Figure 6** shows the utility of iNBSCs for mutant gene-related functional studies. **a,** Schematic outline of CRISPR Cas9-mediated knockout of SCN9a and functional analysis by calcium flux measurements. **b,** Western blot analysis of undifferentiated iNBSCs and sensory neurons derived from WT and SCN9 -/- iNBSCs. Blot shows two independent iNBSCs clones and three independent sensory neuron differentiations from three independent iNBSC WT and SCN9a-/- clones. All neuron cultures were harvested after > 4 weeks of differentiation. Arrowhead marks 200 kDa band of protein ladder. Western blot for ACTIN serves as loading control. **c,** Representative confocal image of a sensory neuron differentiation derived from a SCN9a-/- iNBSC clone. Arrow head marks BRN3a/ Peripherin double-positive cells; scale bar 50 $\mu$m. **d,** Calcium flux measurements of sensory neurons derived from WT and SCN9a -/-iNBSCs before and after stimulation with 30 $\mu$M $\alpha,\beta$-me-ATP. Fluorescence intensity (Fluo-3 AM) of the whole picture is shown as fold change relative to baseline measurement. Plot shows mean (dark colored lines) and SD (light color) of measurements for three independent WT sensory neuron differentiations, three independent WT differentiations with additional treatment of the P2X2/3 antagonist A-317491 and three independent SCN9-/- sensory neuron differentiations, **e,** Quantification of calcium flux. Graph shows maximal fluorescence intensities (Fluo-3 AM) after treatment with $\alpha,\beta$-me-ATP relative to baseline of four independent WT sensory neuron differentiations, three independent WT differentiations with additional treatment of the P2X2/3 antagonist A-317491 and four independent SCN9-/- sensory neuron differentiations. (t-test, * P<0.05, Mean with SEM). **f,** A schematic overview of the direct conversion and isolation of NBSCs, its manipulation via CRISPR Cas9 and differentiation into neural progeny from the CNS and neural crest lineages.

**Extended Data Figure 1: a,** Time dependent rate of neural colony induction of three independent experiments. ADF were transduced with BKSZ, followed by induction of the reprogramming process by DOX- and CAPT-treatment one day after. Subsequently, Dox was removed after 8 - 20 days respectively and the efficiency of colony induction was determined at day 20. **b,** Representative BF pictures of reprogramming without molecules or transgenes. To determine role of transgenes and molecules, ADFs were transduced either with BKSZ transgene (left) or were treated with CAPT without transduction (right). In both condition no formation of neural colonies could be observed; scale bar 250 $\mu$m. **c,** No significant increase of OCT4 expression during neural reprogramming. ADFs were transduced with BKSZ and reprogramming was initiated by application of DOX and CAPT. RNA was derived from three independent experiments at time points indicated; untransduced ADFs served as negative control. No significant increase of OCT4 could be detected (t-test, P>0.05, Mean with SEM). **d,** Overview of neural reprogramming from different somatic cell types. Table shows number of colonies picked from >3 independent reprogramming experiments of FPFs, ADFs and PBMCs. After isolation of single colonies cells were grown without DOX in CAP on feeder cells. If cells showed epithelial morphology and could be kept in culture for more than 5 passages they are referred to as expandable lines. Some lines were further analyzed in respect to marker expression, differentiation capacity and expansion potential and are referred to as characterized lines. All lines used in this study could be kept in culture for > 40 passages (> 6 month). **e,** Conversion efficiency of different somatic cell types. PBMCs, ADFs and FPFs were transduced with BKSZ and reprogramming was initiated by DOX and CAPT treatment. Number of colonies was determined at day 19 post transduction. Efficiency was calculated as number of colonies relative to cell number after transduction. Efficiency was calculated based on three independent reprogramming experiments of PBMCs, ADFs and MSCs, respectively. **f,** mRNA expression of neural progenitor and border markers in iNBSCs derived from different cell types. mRNA expression of neural progenitor and border markers was determined from stable lines of ADF-derived (n=3), PBMC-derived (n=4) and iPSC-derived (n=3) (i)NBSC lines. ADFs and hESCs serve as controls. No significant differences of marker expression between different sources could be detected (t-test, P>0.05, Mean with SEM). **g,** Validation of transgene removal on mRNA level. qRT-PCR with a transgene-specific primer were performed on cDNA of three independent iNBSC clones before and after Cre-mediated recombination. Untransduced and BKSZ transduced ADFs were used as reference. **h,** Validation of transgene removal from gDNA. qRT-PCR with a transgene-specific primer were performed on gDNA of three independent iNBSC clones before and after Cre-mediated recombination. Untransduced cells were used as reference. **i,** Representative confocal images of an PBMC-derived neural progenitor line staining positive for neural border and neural stem cell markers even after prolonged culture (P43, >7 month of continuous culture); arrow head marks triple-positive cells; scale bar 50 $\mu$m. **j,** Microarray-based transcriptional profiling of ADF-, Blood- and iPSC-derived (i)NBSCs, PSCs and PBMCs. Principal component analysis shows three clearly distinct clusters, representing (i)NBSCs (ADF-, Blood-, iPSC-derived) and its somatic cell (PBMCs) and PSC (hESCs/ iPSCs) ancestors, respectively. **k,** Representative confocal images of an iPSC-derived neural progenitor line staining positive for neural border and neural stem cell markers; arrow head marks triple-positive cells; scale bar 50 $\mu$m. **l,** Gene expression heatmap of characteristic genes for iNBSCs, PSCs, ADFs and mesoderm. ADFs, PSCs and ADF-, PBMC-, and iPSC-derived (i)NBSCs cluster according to cell type specific gene expression, **m,** Gene set enrichment analysis of hypermethylated promoter sites of iNBSCs compared to ADFs. Terms are ranked by normalized enrichment score (NES).

**Extended Data Figure 2: a,** mRNA expression of TFAP2a after CNS- or NC-priming of iNBSCs. Cells were FACS-sorted for CD133$^+$/ P75$^{neg}$ (CNS-primed) and P75$^+$/CD133$^{neg}$ (NC-primed) and analyzed by qRT-PCR (n=6, t-test, *P<0.05, Mean with SEM). **b,** Representative cytometry data of iNBSCs stained with neural crest surface marker panel. Right panel shows SSEA-1$^{neg}$/CD133$^{neg}$ cell population. **c,** mRNA expression of CNS- and NC-specific markers in SSEA-1$^{neg}$/CD133$^{neg}$/P75$^+$/HNK1$^+$ sorted iNBSC-derived neural crest and iNBSCs. mRNA expression of three independent iNBSC lines and neural crest derivates was analyzed by qRT-PCRs. Expression is normalized to hESCs (t-test, **P<0.01, Mean with SEM). **d,** GO analysis of DEGs from iNBSC-derived neural crest and iNBSCs. Top 100 upregulated genes from iNBSC-derived neural crest and iNBSCs were analyzed by Enrichr analysis tool. Graph shows top results for analysis based on WikiPathways 2016 library. **e,** Representative cytometry data of iNBSCs and iNBSC-derived MSCs stained for MSC surface marker panel. NC-primed iNBSCs were differentiated into MSC-like cells and grown in MSC culture medium for > 2 passages prior to analysis. **f,** Top 20 DEGs between CD13$^+$/CD44$^+$/CD90$^+$/ CD105$^+$/CD146$^+$ sorted MSC-like cells (red) and iNBSCs (grey). **g,** Gene ontology terms enriched in MSC-like cells based on the 150 top upregulated genes in MSC-like cells compared to iNBSC lines. **h,** Expression of neural border markers in iNBSCs and cNPCs. Analysis of mRNA expression in iNBSCs and iNBSC-derived cNPCs by qRT-PCR (t-test, ***P<0.001, Mean with SEM). i, Representative flow cytometry blots of an iNBSC and iNBSC-derived NPC line for P75 and CD133. **j,** Quantification of CD133$^+$/P75$^-$, CD133$^+$/P75$^+$, and CD133$^-$/P75$^+$ population by flow cytometry. Data were derived from independent iNBSC (n=5) and cNPC lines (n=3) (t-test, **P<0.01, ****P<0.0001, Mean with SEM). **k,** Expression of CXCR4 in cNPCs and iNBSCs. Expression levels of CXCR4 were determined by flow cytometry in iNBSCs and iNBSC-derived cNPC. **l,** Expression of the surface markers CD133 and P75 after culture in iNBSC condition. Representative flow cytometry data of iNBSCs and NPCs cultured under iNBSC maintenance condition with CAP medium and culture on feeder cells. **m,** Expression of CXCR4 in independent iNBSC (n=5) and NPC (n=3) lines cultured under iNBSC maintenance condition with CAP medium and growth on feeder cells. Expression levels of CXCR4 were determined by flow cytometry. **n,** Expression of the surface markers CD133 and P75 after culture of NPCs in NC-priming conditions. Representative flow cytometry data of cNPCs cultured in CAB medium for 5 days. **o,** Analysis of P75$^+$/CD133$^-$ and P75$^-$/CD133$^+$ population in iNBSCs and NPCs after culture in NC-priming condition. Three independent iNBSC and cNPC lines were cultured in CAB for 5 days and analyzed by flow cytometry (t-test, *P<0.05, Mean with SEM). **p,** Regional identity of iNBSCs and NPCs. mRNA expression of region specific markers was determined in iNBSCs (n=4) and NPC (n=8) lines. Expression was normalized to hESCs. **q,** Representative cytometry data of iNBSCs stained with a radial glia surface marker panel. Right panel shows SSEA-1$^+$ cell population. **r,** mRNA expression of radial glia-associated markers in independent iNBSC-derived RG-like SCs (n=3) and iNBSCs (n=3); (Mean with SEM).

**Extended Data Figure 3: a,** Confocal images of iNBSCs differentiated into dopaminergic neurons. iNBSCs were differentiated in presence of Chir99028, Purmorphamin and FGF8 for 8 days, followed by culture in Purmrophamin for additional 2 days and matured for another 6 weeks. Arrowheads indicate FOXA2, TH, EN1 triple positive neurons. Scale bar 50 $\mu$m. **b,** Differentiation of RG-like SCs into oligodendrocytes. iNBSC-derived RG-like stem cells were differentiated towards oligodendrocytes and matured for 6 month. Scale bar 50 $\mu$m. c, CNS and crest marker expression in CNS and neural crest primed 3D cultures. Single cell suspension of iNBSCs were embedded in MG and cultured in CNS- or NC-priming conditions for 12 days. mRNA expression of independent CNS (n=3) and NC (n=3) differentiations was determined by qRT-PCR (t-test, **P<0.01, ****P<0.0001, Mean with SEM). **d,** 3D culture of neural crest primed NPCs. Single cell suspension of iNBSC-derived NPCs were embedded in MG and cultured in neural crest priming (CABF) conditions for 12 days. Scale bar 250 $\mu$m. **e,** 3D reconstruction of an oligodendrocyte from confocal images. GFP transduced iNBSCs were differentiated for 8 days in Chir99028, Purmorphamine and FGF8 supplemented medium, followed by transplantation into the striatum of 8 weeks old NOD.*Prkdc$^{scid}$.Il2rg$^{null}$* mice. Mice were analyzed 8 weeks post transplantation. **f,** Confocal images of iNBSCs differentiated towards sensory neuron fate. Cells were analyzed after 4 weeks of differentiation; scale bar 50 $\mu$m.

**Extended Data Figure 4: a,** Preparation of E8.5 stage embryos for isolation of pNBSCs. Representative microscopy pictures of an E8.5 stage embryo prior to dissection of neural tissue. Dashed lines indicate region to be isolated and digested for single cell suspension and subsequent culture. **b,** Table showing results for isolation of pNBSCs from E7.5 to E9.5 stage embryos. **c,** Representative immunofluorescence pictures for neural border and stem cell markers on pNBSCs. pNBSCs were derived from E8.5 tomato mice and cultured for 20 passages in CAP on feeder cells; scale bar 50 $\mu$m. **d,** mRNA expression of Zfp521 in pNBSCs and controls. qRT-PCRs were performed on three independent pNBSCs lines and normalized to whole E13.5 stage fetal brains; mouse ESCs served as negative control. **e,** Expression of neural progenitor and neural border marker after long-term culture of pNBSCs. Representative immunofluorescence pictures of pNBSCs cultured for >4 month; scale bar 50 $\mu$m. **f,** Clonogenicity of pNBSCs. pNBSCs were FACS sorted as single cells and cultured in CAP on inactivated MEFs (pNBSCs) or bFGF/EGF supplemented media on fibronectin (primary RG) for 7 days before analysis. Results are shown for two independent pNBSC clones and E13.5 stage derived RG-like SCs (n=3; t-test, **P<0.01, Mean with SEM). **g,** Culture of pNBSCs on inactivated MEFs (feeder) or Matrigel in presence or absence of Notch-ligands 500ng/ml DII4 and 500 ng/ml Jagged-1. **h,** Regional identity of pNBSCs. mRNA expression of region specific markers was determined in three independent pNBSCs lines. Expression was normalized to whole E13.5 stage fetal brains. **i,** Flow cytometry data of a pNBSC line under maintenance and neural crest priming condition. pNBSCs were either cultured in CAP or CA+BMP4 for 5 days before levels of Ssea1 and P75 were determined by flow cytometry. **j,** Immunofluorescence stainings for serotonin on neuronal differentiation. pNBSCs were differentiated in presence of Purmorphamine for 3 days and matured for additional 2 weeks before stainings were performed; scale bar 50 $\mu$m. **k,** BF pictures of pNBSCs embedded as single cell suspension in MG after CNS- and NC-priming. pNBSCs were cultured in CSP for 6 days (upper panel) or CABF for 10 days (lower panel). Pictures were taken at day 2.5, 4, 6 and 10, respectively. Scale bar 50 $\mu$m. **l,** Gene Ontology (GO) processes enriched in pNBSCs, pNBSC-derived RG-like SCs and pNBSCs-derived NC. GOs are based on top 200 upregulated genes of respective groups.

**Extended Data Figure 5: a,** Targeting of exon 22/27 of SCN9a via CRISPR Cas9. Scheme shows SCN9a locus, the targeting strategy via specific guide RNA and sequencing result for the locus in SCN9-/- clones used in the study. Dashes indicate absent bases; red sequence indicates stop codon. **b,** Western blot analysis of undifferentiated iNBSCs and sensory neurons derived from WT and SCN9 -/- iNBSCs. Complete membranes of blots for Nav1.7 and ACTIN, described in Figure 6b. **c,** Quantification of BRN3a/ Peripherin double-positive neurons in cultures derived from WT and SCN9-/- iNBSC clones. Sensory neuron differentiations from three independent WT iNBSCs and four independent SCN9a -/- iNBSCs were quantified. No significant difference could be measured (t-test, P>0.05, Mean with SEM).

## DETAILED DESCRIPTION OF THE INVENTION

The present invention may be understood more readily by reference to the following detailed description of the invention and the examples included therein.

The present application relates to an *in vitro* method for the direct reprogramming of mature human cells, comprising the step of culturing said mature human cells in the presence of a mixture of transcription factors, wherein said mixture comprises the factors BRN2, SOX2, KLF4 and ZIC3, and wherein said culturing is performed in the presence of a GSK-3 inhibitor, particularly Chir99021; an Alk5 inhibitor, particularly Alk5 inhibitor II; and a hedgehog/smoothened agonist, particularly Purmorphamine. Thus, in a first aspect, the present invention relates to an *in vitro* method for generating induced neural border stem cells by the direct reprogramming of somatic human cells, comprising the step of culturing said somatic human cells in the presence of a mixture of transcription factors, wherein said mixture comprises the factors BRN2, SOX2, KLF4 and ZIC3, and wherein said culturing is performed in the presence of GSK-3 inhibitor Chir99021; Alk5 inhibitor II; and hedgehog/smoothened agonist Purmorphamine. Furthermore, the present application relates to an *in vitro* method for the direct differentiation of pluripotent human stem cells, particularly embryonic stem cells (ESCs) or induced pluripotent stem cells (iPSCs), comprising the step of culturing said pluripotent human stem cells in the presence of a GSK-3 inhibitor, particularly Chir99021; an Alk5 inhibitor, particularly Alk5 inhibitor II; and a hedgehog/smoothened agonist, particularly Purmorphamine.

Furthermore, the present application relates to an *in vitro* method for the direct differentiation of pluripotent non-human stem cells, particularly embryonic stem cells (ESCs) or induced pluripotent stem cells (iPSCs), comprising the step of culturing said pluripotent human stem cells in the presence of a GSK-3 inhibitor, particularly Chir99021; an Alk5 inhibitor, particularly Alk5 inhibitor II; and a hedgehog/smoothened agonist, particularly Purmorphamine.

In particular, the pluripotent non-human stem cells are pluripotent murine stem cells.

In the context of the present invention, the term "hematopoietic stem and progenitor cells", abbreviated HSPCs, collectively refers to hematopoietic stem cells (HSCs) and progenitors thereof, which are the first stages of differentiation of HSCs.

In the context of the present invention, the term "comprises" or "comprising" means "including, but not limited to". The term is intended to be open-ended, to specify the presence of any stated features, elements, integers, steps or components, but not to preclude the presence or addition of one or more other features, elements, integers, steps, components, or groups thereof. The term "comprising" thus includes the more restrictive terms "consisting of" and "consisting essentially of".

To reprogram human adult somatic cells into an early embryonic self-renewing neural stem cell type, we transduced human adult dermal fibroblasts (ADFs) with a variety of combinations of different transcription factors (BRN2, SOX2, KLF4, MYC, TLX, and ZIC3), different small molecules and different cytokines that we hypothesized to potentially allow access to early neural stages. Finally, we identified the combination of four factors BRN2, KLF4, SOX2 and ZIC3 (BSKZ) and the molecules Chir99021 (GSK-3 inhibitor), Alk5 Inhibitor II, and Purmorphamine (hedgehog/ smoothened agonist), and optionally Tranylcypromine (inhibitor of monamine-oxidase (MAO) and CYP2 enzymes: A6, C19, and D6) (CAPT) to enable neural reprogramming.

The present application furthermore describes, that said culturing is performed in the additional presence of an inhibitor of monoamine-oxidase, particularly Tranylcypromine. In a particular embodiment, said culturing is performed in the additional presence of monoamine-oxidase inhibitor Tranylcypromine.

In the context of the present invention, the term "Tranylcypromine" refers to an inhibitor of monoamine-oxidase (MAO) and of CYP2 enzymes: A6, C19, and D6 (CAPT).

In a particular embodiment, Tranylcypromine is only present during an induction phase, particularly in the first 12 to 21 days of said culturing, particularly in the first 12 to 16 days for pMSCs, and 17 to 21 days wherein said somatic cells are PBMCs.

In a particular embodiment, said somatic cells are selected from adult fibroblast cells; pancreas-derived mesenchymal stromal cells; and peripheral blood cells, particularly peripheral blood mononuclear cells.

In particular embodiments, said step of culturing is performed on supportive feeder cells, particularly on murine fibroblast cells.

In particular embodiments, a culture comprising said mature human cells is transduced with said factors BRN2, SOX2, KLF4 and ZIC3.

In a particular embodiment, said factors BRN2, SOX2, KLF4 and ZIC3 are comprised in a vector.

In a particular embodiment, said vector is a polycistronic vector.

In particular embodiments, said vector is a doxycycline-inducible vector, particularly wherein said vector is vector pHAGE2-TetOminiCV-BRN22AKlf4-IRES-Sox2E2AZic3-W according to SEQ ID NO: 1

In a particular embodiment, said culturing is performed in the presence of doxycycline for at least 12 days after transduction, particularly for 12, 13, 14, 15 or 16 days.

In particular embodiments, said method further comprises the step of clonally expanding single colonies.

In a particular embodiment, said colonies are expanded until a day selected from day 19 to day 24 after transduction.

In particular embodiments, said vector further comprises loxP sites flanking the nucleic acid sequence encoding said factors BRN2, SOX2, KLF4 and ZIC3, particularly wherein said vector is vector pHAGE2-TetOminiCV-BRN22AKlf4-IRES-Sox2E2AZic3-W-loxp according to SEQ ID NO: 2.

In a particular embodiment, the nucleic acid sequence encoding said factors BRN2, SOX2, KLF4 and ZIC3 comprised in said vector is excised by Cre recombinase.

In a particular embodiment, said *in vitro* method of the present invention comprises the step of transducing the cells with a plasmid encoding said Cre recombinase.

In a particular embodiment, said Cre recombinase is the Cherry-Cre recombinase.

The present application further describes a nucleic acid sequence encoding BRN2, SOX2, KLF4 and ZIC3.

The present application further describes a polycistronic vector encoding BRN2, SOX2, KLF4 and ZIC3.

In particular, said vector is a polycistronic vector.

In particular embodiments, said vector is a doxycycline-inducible vector, particularly wherein said vector is vector pHAGE2-TetOminiCV-BRN22AKlf4-IRES-Sox2E2AZic3-W according to SEQ ID NO: 1.

In particular embodiments, said vector further comprises a loxP site, particularly wherein said vector is vector pHAGE2-TetOminiCV-BRN22AKlf4-IRES-Sox2E2AZic3-W-loxp according to SEQ ID NO: 2.

The present application furthermore describes a kit comprising at least two, more particularly all three components selected from: a GSK-3 inhibitor, particularly Chir99021; an Alk5 inhibitor, particularly Alk5 inhibitor II; and a hedgehog/smoothened agonist, particularly Purmorphamine.

In particular, said kit is further comprising an inhibitor of monoamine-oxidase, particularly Tranylcypromine.

In particular, said kit further comprises one or more components selected from: a vector as used in a method of the present invention; supportive feeder cells, particularly murine fibroblast cells; and a plasmid encoding a Cre recombinase, particularly the Cherry-Cre recombinase.

The present application furthermore describes an isolated (induced) neural border stem cell line. In the context of the present invention, terms such as "(induced) neural border stem cell line" or "(induced) neural border stem cells" refer either to an induced neural border stem cell line/induced neural border stem cells obtained by reprogramming mature human cells, or to neural border stem cell line/neural border stem cells obtained by differentiating pluripotent stem cells, such as ESCs or iPSCs.

In particular, said isolated (induced) neural border stem cell line is characterized by being positive both (i) for early neural markers, particularly PAX6, ASCL1, BRN2 and SOX1; and (ii) for stem cell markers, particularly NESTIN and SOX2.

In particular, said isolated (induced) neural border stem cell line is further characterized by expressing MSX1, ZIC1 and PAX3.

In particular, said isolated (induced) neural border stem cell line is characterized by being additionally positive for HES5, SOX3 and HOXA2.

In particular, said isolated (induced) neural border stem cell line has been generated by the *in vitro* method of the present invention.

In particular, , the results of a single nucleotide polymorphisms analysis of the isolated (induced) neural border cell line cluster with the results of a single nucleotide polymorphisms analysis of said mature human cells.

In particular, said isolated (induced) neural border stem cell line has been generated by the *in vitro* method of the present invention, wherein the results of a principle component analysis of a comparative global gene expression analysis of the cell line (i) does not cluster, in the case of an isolated induced neural border stem cell line, with the results of a principle component analysis of a comparative global gene expression analysis of said mature human cells, and (ii) does not cluster with the results of a principle component analysis of a comparative global gene expression analysis of human induced pluripotent stem cells.

In an embodiment of the *in vitro* method of the present invention, the method further comprises expanding said induced neural border stem cells or said isolated induced neural border stem cell line, comprising the step of culturing said induced neural border stem cells or cells from said isolated induced neural border stem cell line, particularly wherein said culturing is performed in the presence of a substance selected from DLL1, DLL3 and DLL4, Jagged-1, and Jagged-2, more particularly from DLL4 and JAGGED-1.

The formation of the nervous system initiates with the neural plate stage shortly after gastrulation. Signalling pathways such as WNTs, BMPs and SHH orchestrate the diversification of neural committed cells, which underlie the development of the various brain regions, spinal cord as well as the neural crest ([13], [14], [15]).

In particular said culturing is performed in the presence of GSK-3 inhibitor Chir99021; an Alk5 inhibitor II; and hedgehog/smoothened agonist Purmorphamine, particularly wherein said culturing is performed at 5% $O_2$.

In particular embodiments, said culturing is performed on a layer of supportive feeder cells, particularly on murine fibroblast cells.

In particular embodiments, said culturing is performed for up to 40 passages.

In a next aspect, the present invention relates to an *in vitro* method for generating and differentiating induced neural border stem cells, comprising the steps of (i) performing the *in vitro* method of the present invention for generating induced neural border stem cells, and (ii) of culturing said induced neural border stem cells or cells from said isolated

induced neural border stem cell line in the presence of differentiation factors.

In a particular embodiment, said (induced) neural border stem cells are differentiated to cells of a central nervous system lineage.

In a particular embodiment, said induced neural border stem cells are cultured in the presence of a GSK-3 inhibitor, particularly Chir99021; an ALK 4,5,7 inhibitor, particularly SB431542; and a hedgehog/smoothened agonist, particularly Purmorphamine, and wherein bFGF is added.

In particular embodiments, said method is characterized by an increase in CD133$^+$/ P75 $^{neg}$ cells.

In particular embodiments, said method is characterized by an enrichment of mRNA for CNS-related genes, particularly PAX6, and by a downregulation of neural border-related genes, particularly TFAP2a and SOX10.

In a particular embodiment, the *in vitro* method further comprises the step of isolationg a central nervous system primed neural progenitor cell line of the central nervous system lineage.

The present application further describes an isolated central nervous system primed neural progenitor cell line of the central nervous system lineage, particularly (i) wherein said cell line is of the same development status as primary neural progenitor cells obtainable from embryos of gestation week 8 to 12, and/or (ii) wherein said cell line is characterized by progenitor markers LONRF2, ZNF217, NESTIN, SOX1 and SOX2, particularly LONRF2 and ZNF217, and by being negative for MSX1, PAX3 and TFAP2, and/or (iii) wherein said cell line is characterized by epigenetically corresponding to mature human cells, particularly wherein said cell line has been obtained from said mature human cells in a direct reprogramming method according to the present invention.

The present application further describes an isolated differentiated (induced) neural border stem cell line of the central nervous system lineage, which is generated by the method of the present invention.

In a next aspect, the present invention relates to an *in vitro* method for generating CNS progenitor cells, comprising the steps of (i) performing the *in vitro* method of the present invention for generating induced neural border stem cells, and (ii) culturing induced neural border stem cells or cells from said isolated induced neural border stem cell line, optionally after first differentiating (induced) neural border stem cells in a method of the present invention, in a medium comprising GSK-3 inhibitor Chir99021; ALK inhibitor SB431542; hedgehog/smoothened agonist Purmorphamine; bFGF; and LIF.

In a particular embodiment of the *in vitro* method of the present invention, the culture is maintained on a gelatinous protein mixture secreted by Engelbreth-Holm-Swarm mouse sarcoma cells (Matrigel).

The present application further describes an isolated central nervous system progenitor cell line, particularly wherein said cell line is characterized by epigenetically corresponding to mature human cells, particularly wherein said cell line has been obtained from said mature human cells in a direct reprogramming method according to the present invention.

In particular, the isolated central nervous system progenitor cell line is generated by the method of the present invention.

In particular embodiments, the isolated central nervous system progenitor cell line generated by the method of the present invention is characterized by (ia) downregulation of FGFR3, HES5, ASCL1, CLDN5 und ZIC3, and (ib) maintained expression of PAX6, SOX1, SOX2 and NESTIN; in both cases when compared to (induced) neural border stem cells; and/or (ii) wherein said cell line is characterized by epigenetically corresponding to mature human cells, particularly wherein said cell line has been obtained from said mature human cells in a direct reprogramming method according to the method of the present invention.

In a next aspect, the present invention relates to an *in vitro* method of generating and differentiating central nervous system progenitor cells as described in the present application, comprising the steps of (i) performing the *in vitro* method of the present invention for generating CNS progenitor cells, and (ii) culturing said central nervous system progenitor cells in the presence of differentiation factors.

In a particular embodiment, said method is performed for seven weeks, followed by culturing in an expansion medium supplemented with bFGF, EGF and LIF.

In particular embodiments of the *in vitro* method of the present invention, the resulting cell population is positive for SSEA1, CD133 and Glutamate Aspartate Transporter (GLAST; SLC1A3).

The present application further describes an isolated cell population having a radial glia type stem cell phenotype, particularly wherein said cell population is characterized by epigenetically corresponding to mature human cells, particularly wherein said cell population has been obtained from said mature human cells in a direct reprogramming method according to the present invention.

In a particular embodiment, said method comprises the steps of (i) seeding iNBSCs on plates coated with a gelatinous protein mixture secreted by Engelbreth-Holm-Swarm mouse sarcoma cells (Matrigel) and culturing in basal medium supplemented with 1 $\mu$M Purmorphamine and 10 ng/ml FGF8 for one week, (ii) culturing in basal medium with 1 $\mu$M Purmorphamine for one additional day; (iii) growing the cultures in basal medium containing 10 ng/ml BDNF and 10 ng/ml GDNF for 7 more weeks; and (iv) culturing the cells in radial glia medium, comprised of basal medium, 20 ng/ml bFGF, 20 ng/ml EGF and 10 ng/ml LIF.

In particular, said isolated cell population is characterized by cells (ia) being triple-positive for SSEA1, CD133 and GLAST, (ib) strongly expressing the glial markers VIMENTIN, GFAP and GLAST; and (ic) being positive for the stem cell markers PAX6, NESTIN, SOX1 and BLBP; and/or (ii) wherein said cell line is characterized by epigenetically corresponding to mature human cells, particularly wherein said cell population has been obtained from said mature human cells in a direct reprogramming method according to the present invention.

In particular, said (induced) neural border stem cells are differentiated to cells of a neural crest lineage.

In particular, said (induced) neural border stem cells are cultured in the presence of a GSK-3 inhibitor, particularly Chir99021; an Alk5 inhibitor, particularly Alk5 inhibitor II; and BMP4.

In particular, said method is characterized by an increase in $P75^+/CD133^{neg}$ cells.

In particular, said method is characterized by an enrichment of mRNA for neural crest associated genes, particularly SOX10 and AP2a.

Furthermore, the present application describes an isolated differentiated (induced) neural border stem cell line of the neural crest lineage, particularly wherein said cell line is characterized by epigenetically corresponding to mature human cells, particularly wherein said cell line has been obtained from said mature human cells in a direct reprogramming method according to the present invention.

In a particular, said isolated differentiated (induced) neural border stem stell line of the neural crest lineage is generated by the *in vitro* method of the present invention.

In a next aspect, the present invention relates to an *in vitro* method for generating neural crest progenitor cells, comprising the steps (i) performing the *in vitro* method of the present invention for generating induced neural border stem cells, and (ii) culturing induced neural border stem cells or cells from said isolated induced neural border stem cell line for three days in the presence of GSK-3 inhibitor Chir99021; Alk5 inhibitor II; and BMP4; followed by culturing in the presence of GSK-3 inhibitor Chir99021, FGF8, IGF1 and DAPT.

The present application further describes an isolated neural crest progenitor cell line, particularly wherein said cell line is characterized by epigenetically corresponding to mature human cells, particularly wherein said cell line has been obtained from said mature human cells in a direct reprogramming method according to the present invention.

In particular, said isolated neural crest progenitor cell line is generated by the *in vitro* method of the present invention.

In particular, the isolated neural crest progenitor cell line is characterized by (ia) the induction of migratory crest markers P75 and HNK1; (ib) a decrease in CD133 and SSEA1 levels; (ic) presence of SOX10, and (id) absence of PAX6; in each case when compared to (induced) neural border stem cells; and/or (ii) wherein said cell line is characterized by epigenetically corresponding to mature human cells, particularly wherein said cell line has been obtained from said mature human cells in a direct reprogramming method according to the present invention.

In particular, the neural crest progenitor cell line is further characterized by KANK4, BGN, TFAP2A and SOX10 being among the strongest upregulated genes, with neural progenitor markers, in particular HES5 and PAX6, being downregulated, in each case when compared to (induced) neural border stem cells.

In a next aspect, the present invention relates to an *in vitro* method of generating and differentiating a neural crest progenitor cell line, comprising the step of (i) performing the *in vitro* method of the present invention for generating said neural crest progenitor cell line, and (ii) culturing cells from said neural crest progenitor cell line in the presence of differentiation factors.

In a particular embodiment, said cells are obtained by performing the method comprising the steps of (i) seeding $1\times10^5$/ 6 Well iNBSCs on plates coated with a gelatinous protein mixture secreted by Engelbreth-Holm-Swarm mouse sarcoma cells (Matrigel) and growing in basal medium supplemented with 4 $\mu$M Chir99028, 5 $\mu$M Alk5 Inhibitor II and 10 ng/ml BMP4 for three days; (ii) growing the cells in a basal medium supplemented with 4 $\mu$M Chir99028, 10 ng/ml FGF8, 10 ng/ml IGF1 and 1 $\mu$M DAPT for five days; (iii) puryingin the NCSC-like cells by cell sorting for SSEA-1$^{neg}$CD133$^{neg}$P75$^+$HNK1$^+$; in particular wherein all cultures are grown at 37°C, 5% $CO_2$ and 20% $O_2$..

In particular embodiments of the method, the resulting cell population is positive for first markers shown in Figure 2f, particularly HNK1, P75, and SOX10, and negative for second markers shown in Figure 2f, particularly CD133, SSEA1, HES5, and PAX6.

The present application further describes an isolated cell population having a neural border stem cell phenotype, particularly wherein said cell line is characterized by epigenetically corresponding to mature human cells, particularly wherein said cell population has been obtained from said mature human cells in a direct reprogramming method according to the present invention.

In particular, said isolated cell population is obtained by a method of the present invention.

In particular, said isolated cell population is characterized by (i) the induction of migratory crest markers P75 and HNK1; (ii) a decrease in CD133 and SSEA1 levels; (iii) the presence of SOX10, and (iv) the absence of PAX6; in each case when compared to (induced) neural border stem cells.

In particular, said isolated cell population is further characterized by KANK4, BGN, TFAP2A and SOX10 being among the strongest upregulated genes, with neural progenitor markers, in particular HES5 and PAX6 being downregulated, in each case when compared to (induced) neural border stem cells.

The present application further describes an *in vitro* method for the generation of dopaminergic neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, (ii) changing to a medium that is supplemented with FGF8 and a hedgehog/smoothened agonist, particularly Purmorphamine, on murine fibroblasts; (iii) culturing the cells in the medium according to (ii) for 7 days on a gelatinous protein mixture secreted by Engelbreth-Holm-Swarm mouse sarcoma cells (Matrigel), (iv) changing to a medium that is supplemented with a hedgehog/smoothened agonist, particularly Purmorphamine; (v) culturing the cells in the medium according to (iv) for 2 days, and (vi) changing the medium to maturation medium; and (vii) culturing the cells for 5 weeks in said maturation medium.

The present application further describes an *in vitro* method for the generation of motor neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 2 days on a gelatinous protein mixture secreted by Engelbreth-Holm-Swarm mouse sarcoma cells (Matrigel), (iv) changing to a medium that is supplemented with a hedgehog/smoothened agonist, particularly Purmorphamine, and all-trans retinoic acid; (v) culturing the cells in the medium according to (iv) for 7 days, and (vi) changing the medium to maturation medium; and (vii) culturing the cells for 5 weeks in said maturation medium.

The present application further describes an *in vitro* method for the generation of glutamatergic and gabaergic neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to maturation medium comprising BDNF and GDNF; and (v) culturing the cells for 5 weeks in said maturation medium.

The present application further describes an *in vitro* method for the generation of serotonergic neurons, comprising the steps of (i) culturing (induced) neural border stem cells in basal medium comprising 3 $\mu$M Chir99021, an Alk5 inhibitor, particularly 3 $\mu$M SB431542, and a hedgehog/smoothened agonist, particularly 1 $\mu$M Purmorphamine, on plates covered by a gelatinous protein mixture secreted by Engelbreth-Holm-Swarm mouse sarcoma cells (Matrigel) for one week, (ii) followed by culture in 3 $\mu$M Chir99028, an Alk5 inhibitor, particularly 3 $\mu$M SB431542, and a hedgehog/smoothened agonist, particularly 1 $\mu$M Purmorphamine, and 10 ng/ml FGF4 for another week, (iii) followed by switching to and a hedgehog/smoothened agonist, particularly 1 $\mu$M Purmorphamine, for two days, and (iv) subsequently growing the cells in neuronal maturation medium comprising basal medium, 500 $\mu$M dbcAMP (Sigma), 1 ng/ml TGF$\beta$3, 10 ng/ml BDNF and 10 ng/ml GDNF for at least 5 more weeks.

The present application further describes an *in vitro* method for the generation of astrocytes, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to a maturation medium comprising BDNF, GDNF and 1% FCS, and (v) culturing the cells for 5 weeks in said maturation medium.

The present application further describes an *in vitro* method for the generation of oligodendrocytes, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a hedgehog/smoothened agonist, particularly Purmorphamine; (iii) culturing the cells in the medium according to (ii) for 7 days on a gelatinous protein mixture secreted by Engelbreth-Holm-Swarm mouse sarcoma cells (Matrigel), (iv) changing the medium to a medium comprising T3, IGF, Forskolin, PDGF, and EGF, (v) culturing the cells for 2 weeks in the medium according to (iv), (vi) changing the medium to a medium comprising T3, IGF, Forskolin, PDGF, Dorsomorphin, (vii) culturing the cells for 1 week in the medium according to (vi), (viii) changing the medium to a medium comprising T3, IGF, and Forskolin, and (ix) culturing the cells for 3 weeks in the medium according to (viii).

The present application further describes an *in vitro* method for the generation of neural crest-derived neurons, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a GSK-3 inhibitor, particularly Chir99021; an Alk5 inhibitor, particularly Alk5 inhibitor II, and BMP4; (iii) culturing the cells in the medium according to (ii) for 3 days, (iv) changing the medium to a medium comprising a GSK-3 inhibitor, particularly Chir99021; an FGF inhibitor, particularly SU5402, a Notch inhibitor, particularly DAPT, and NGF, (v) culturing the cells for 10 days in the medium according to (iv), (vi) changing the medium to a maturation medium comprising BDNF, GDNF and NGF, and (vii) culturing the cells for 3 weeks in the maturation medium according to (vi).

The present application further describes an *in vitro* method for the generation of cells having a mesenchymal stem cell phenotype, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) changing to a medium that is supplemented with a GSK-3 inhibitor, particularly Chir99021; an Alk5 inhibitor, particularly Alk5 inhibitor II, and BMP4; (iii) culturing the cells in the medium according to (ii) for 3 days, (iv) changing the medium to a medium comprising a GSK-3 inhibitor, particularly Chir99021; FGF8, IGF, and a Notch inhibitor, particularly DAPT, (v) culturing the cells for 7 days in the medium according to (iv), (vi) changing the medium to a maturation medium comprising bFGF and IGF, (vii) culturing the cells for 2 weeks in the maturation medium according to (vi), (viii) changing the medium to a mesenchymal stem cell medium, and (ix) culturing in said mesenchymal stem cell medium.

The present application further describes an *in vitro* method for the generation of cells having a mesenchymal stem cell phenotype, comprising the steps of (i) seeding iNBSCs on plates coated with a gelatinous protein mixture secreted by Engelbreth-Holm-Swarm mouse sarcoma cells (Matrigel),(ii) culturing the cells in 4 $\mu$M Chir99028, 10 ng/ml BMP4 and 10 $\mu$M DAPT for 7 days; (iii) culturing the cells in basal medium containing 10 ng/ml bFGF and 10 ng/ml IGF-1 for at least 5 passages; (iv) stabilizing the cells by switching the cultures to mesenchymal stem cell medium and culturing for at least 2 passages.

The present application further describes an *in vitro* method for the differentiation of cells having a mesenchymal stem cell phenotype into adipocytes, comprising the steps of (i) generating said cells having a mesenchymal stem cell phenotype by the *in vitro* method of the present invention, (ii) changing the medium to a mesenchymal induction medium comprising 10% FCS; (iii) culturing the cells in the medium according to (ii) for 5 days, (iv) changing the medium to a adipogenesis differentiation medium, and (v) culturing the cells in the medium according to (iv).

The present application further describes an *in vitro* method for the differentiation of cells having a mesenchymal stem cell phenotype into chondrocytes, comprising the steps of (i) generating said cells having a mesenchymal stem cell phenotype by the *in* vitro method as described in the present application, (ii) changing the medium to a mesenchymal induction medium comprising10% FCS; (iii) culturing the cells in the medium according to (ii) for 5 days, (iv) changing the medium to a chondrocyte differentiation medium, and (v) culturing the cells in the medium according to (iv).

The present application further describes an *in vitro* method for the differentiation of cells having a mesenchymal stem cell phenotype into smooth muscle cells, comprising the steps of (i) generating said cells having a mesenchymal stem cell phenotype by the *in vitro* method as described in the present application, (ii) changing the medium to a mesenchymal induction medium comprising10% FCS; and (iii) culturing the cells in the medium according to (ii) for 3 to 5 weeks.

The present application further describes an *in vitro* method for the generation of a neural tube-like 3D culture, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) embedding of a single cell suspension of the cells cultured according to step (i) in a gelatinous protein mixture secreted by Engelbreth-Holm-Swarm mouse sarcoma cells (Matrigel) and adding a medium comprising SB and a hedgehog/smoothened agonist, particularly Purmorphamine; (iii) culturing said single cell suspension according to (ii) for 9 days; (iv) changing the medium to a medium comprising a GSK-3 inhibitor, particularly Chir99021, SB, a hedgehog/smoothened agonist, particularly Purmorphamine, and bFGF and (v) culturing for 4 days.

The present application further describes an *in vitro* method for the generation of a neural crest-like 3D culture, comprising the steps of (i) culturing (induced) neural border stem cells in a medium comprising a GSK-3 inhibitor, particularly Chir99021; an Alk5 inhibitor, particularly Alk5 inhibitor II; a hedgehog/smoothened agonist, particularly Purmorphamine, on murine fibroblasts, (ii) embedding of a single cell suspension of the cells cultured according to step (i) in a gelatinous protein mixture secreted by Engelbreth-Holm-Swarm mouse sarcoma cells (Matrigel) and adding a medium comprising a medium comprising a GSK-3 inhibitor, particularly Chir99021, an Alk5 inhibitor, particularly Alk5 inhibitor II, BMP4, and FGF2, and (iii) culturing for 12 days.

The present application further describes an *in vitro* method for the generation of cells representing a mutant phenotype, comprising the steps of (i) causing or allowing the modification of a gene sequence, the transcription or translation of a gene sequence, and/or of a protein encoded by a gene sequence of cells from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, or cells generated by the method as described in the present application.

In particular, said step (i) is performed by using gene editing, particularly by using a CRISPR Cas9-mediated knockout.

In particular, said step (i) is performed by using gene silencing, particularly by using a DNAzyme, antisense DNA, siRNA, or shRNA.

In particular, said step (i) is performed by using protein inhibitors, particularly by using antibodies directed against a protein.

The present application further describes an *in vitro* method for drug screening, comprising the step of exposing cells from an isolated (induced) neural border stem cell line as described in the present application, an isolated differentiated (induced) neural border stem cell line of the central nervous system lineage as described in the present application, an isolated central nervous system progenitor cell as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application to a drug substance.

In particular, the *in vitro* method further comprises the determination of one of more factors that are potentially affected by interaction with said drug substances.

The present application further describes a pharmaceutical composition comprising cells from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application.

The present application further describes a cell from an isolated (induced) neural border stem cell line as described in the present application, an isolated differentiated (induced) neural border stem cell line of the central nervous system lineage as described in the present application, an isolated central nervous system progenitor cell line as described in the present application, an isolated cell population having a radial glia type stem cell phenotype as described in the present application, an isolated differentiated (induced) neural border stem cell line of the neural crest lineage as described in the present application, an isolated neural crest progenitor cell line as described in the present application, an isolated cell population having a neural border stem cell phenotype as described in the present application, cells generated by the method as described in the present application, or cells representing a mutant phenotype that are obtained according to the method as described in the present application for use in the treatment of a patient suffering from a neural disorder.

## EXAMPLES

### Introduction:

Generation of functional human neuronal cell types from pluripotent iPSCs by directed differentiation is inefficient. Moreover, neural conversion of somatic cells by direct reprogramming typically results in cell types with limited proliferation and/ or differentiation potential. Here we report that ectopic expression of four neural transcription factors (BRN2, SOX2, KLF4 and ZIC3) allows to reprogram human adult fibroblasts or peripheral blood cells into a so far unidentified self-renewing Neural Border Stem Cell population (iNBSC). Human iNBSCs share molecular and functional features with a corresponding mouse NBSC population we could isolate from neural folds of E8.5 embryos. Upon differentiation, iNBSCs pass through successive developmental stages and can give rise to either (1) CNS-primed progenitors, radial glia-type stem cells, dopaminergic and serotonergic neurons, motoneurons, astrocytes and oligodendrocytes or (2) neural crest lineage including peripheral neurons. We demonstrate direct reprogramming of human adult cells into expandable naturally occurring and multipotent embryonic iNBSCs that define a novel embryonic neural stem cell population in human and mouse. Furthermore, we provide evidence that CRISPR/Cas9 edited iNBSCs, carrying a mutant SCN9a gene, can be expanded and differentiated into sensory neurons. These show impaired functional properties mimicking a human pain syndrome. Hence iNBSCs open novel possibilities for patient-specific and mechanism-based research, which can be associated to high throughput drug screens or cell based regenerative medicine.

The goal of this study was to overcome these limitations of the prior art and to explore the possibility of reprogramming human adult somatic cells into early, defined and self-renewing neural progenitors with broad but specific differentiation potential.

## Results

The formation of the nervous system initiates with the neural plate stage shortly after gastrulation. Signalling pathways such as WNTs, BMPs, and SHH orchestrate the diversification of neural committed cells, which underlie the development of the various brain regions, spinal cord as well as the neural crest ([13], [14], [15]). We hypothesized that overexpression of stage-specific transcription factors, in combination with adequate signalling cues provided by the growth medium, might allow for the direct reprogramming of adult somatic cells to early embryonic neural progenitors with stem cell features including self-renewal and multipotency.

To reprogram human adult somatic cells into an early embryonic self-renewing neural stem cell type, we transduced human adult dermal fibroblasts (ADFs) with various combinations of transcription factors (BRN2, SOX2, KLF4, MYC, TLX, ZIC3) and small molecules that we hypothesized to allow access to early neural stages. To this end, we identified the combination of four factors BRN2, KLF4, SOX2 and ZIC3 (BKSZ) and the four small molecules Chir99021 (GSK-3 inhibitor), Alk5 Inhibitor II, Purmorphamine (hedgehog/ smoothened agonist), and Tranylcypromine (inhibitor of monamine-oxidase (MAO) and CYP2 enzymes: A6, C19, and D6) (CAPT) to enable neural reprogramming.

In short, ADFs were transduced with a polycistronic, doxycycline (DOX)-inducible vector containing BKSZ, and subsequently cultured in the presence of CAPT. We also derived a modified version containing a loxP site (Fig. 1a,b) to allow subsequent Cre-mediated transgene removal. At day 14 post transduction, colony formation was observed. Upon DOX withdrawal, the colonies continued to grow and expressed the early neural markers PAX6 and SOX1 (Fig 1b). Individual colonies were clonally expanded resulting in stable lines 19-24 days after transduction (Fig. 1b).

Detailed time-course experiments of the reprogramming process revealed that the minimum time period of BKSZ-activity, in constant presence of CAPT, was 12 days, while the efficiency of reprogramming further increased with prolonged DOX application (Extended Data Fig. 1a). Importantly, overexpression of BKSZ and growth without small molecules or treatment with CAPT alone did not result in colony formation (Extended Data Fig. 1b). The following results suggest that BKSZ-mediated reprogramming does not involve a pluripotent intermediate state. First, induction of the transgenes did not result in a significant increase of OCT4 expression (Extended Data Fig. 1c). Second, iPSCs could not be derived by BKSZ reprogramming of ADFs, even after switching to pluripotent stem cell medium (data not shown).

Next, we investigated whether other adult cell types could be used as a source for neural reprogramming. Indeed, we were able to successfully convert human fetal pancreas fibroblasts (FPFs) and peripheral blood mononuclear cells (PBMCs), and established more than 30 stable neural progenitor lines (Extended Data Fig. 1d). In concordance with earlier reports, the conversion efficiency varied with the degree of maturity of the cell of origin, being highest for the FPFs (0.166%) and lowest for PBMCs (0.015%) ([16]) (Extended Data Fig. 1e). Importantly, once stable lines were established, the cell type of origin had no apparent impact on proliferation, neural marker expression or differentiation capacity (Extended Data Fig. 1 d, f, i).

A prerequisite of stable reprogramming is the silencing of the ectopically expressed transgenes to guarantee normal differentiation as shown for iPSCs ([17]). Importantly, uncontrolled transgene reactivation bears the risk of tumor induction in *vivo* ([18], [19]). After DOX withdrawal, stable neural lines with sustained >1000 fold down-regulation of the polycistronic trangene cassette could be established (Extended Data Fig. 1h). To rule out potential effects of system-intrinsic leakiness of the Tet-On system on the differentiation or self-renewal capacity of the clones, we used the LoxP-modified version of the BKSZ vector mentioned above (Fig. 1a). After derivation of clonal lines, cells were transfected with a plasmid encoding a Cherry-Cre recombinase ([20]), sorted for Cherry-positivity and seeded at clonal density. In doing so, we derived subclones that showed neither expression of the transgene at the mRNA level (Extended Data Fig. 1g) nor genomic integration (Extended Data Fig. 1h). Taken together these results show that the derived neural clones are transgene independent.

Converted clonal neural cell lines derived from ADFs, FPFs and PBMCs could be cultured on a layer of supportive feeders and in medium containing Chir99021, Alk5 Inhibitor II and Purmorphamine (CAP) for more than 40 passages (> 7 months), without loss of proliferative potential and maintenance of high expression of early neural markers such as PAX6 and SOX1 (Fig. 1d, Extended Data Fig. 1i). Moreover, the cultures were homogenously positive for the stem cell markers NESTIN and SOX2, and expressed also MSX1, ZIC1 and PAX3, suggesting a neural border-like identity of the converted cells (Fig. 1d, Extended Data Fig 1i). To formally prove that the iNBSCs were indeed derived from PBMCs and ADFs respectively, clones were analysed for single nucleotide polymorphisms (SNPs) demonstrating that they originated from their respective human donor (data not shown). In concordance with their sustained self-renewal capacity and expression of neural border markers, we named these cells "induced Neural Border Stem Cells" (iNBSCs).

To gain further insight into the molecular identity of iNBSCs, we performed comparative global gene expression analysis of iNBSCs, iPSCs and the cell type of origin (i. e. ADFs, PBMCs). Principle component analysis revealed that each cell type clustered distinctly in separate expression clusters (Fig. 1e, Extended Data Fig. 1j). All iNBSC clones clustered closely, indicating that the cell of origin had no major impact on the iNBSC identity. Next, we explored whether NBSCs can also be obtained through directed differentiation from iPSCs, as this would exclude an artificial state that occurs only during the reprogramming process. To this end, we analysed neural border marker expression in clonal lines from differentiated iPSCs and found that it was indeed similar to that of ADF- and PBMC-derived iNBSCs (Extended Data Fig 1f, Extended Data Fig. 1k). Moreover, as the expression profile of iPSC-derived clones also clustered with that of iNBSCs, we conclude that the NBSC state can be established also during *in vitro* differentiation from pluripotent stem cells (Fig. 1e, Extended Data Fig. 1j).

Comparison of the expression profiles of iNBSCs with that of ADFs revealed 10917 differentially expressed genes (DEGs) ($p < 0.05$) reflecting the robust change of cellular identity. Gene ontology (GO) analysis of the top 200 up-regulated genes in iNBSCs unveiled an enrichment for stem cell related processes such as *neuronal stem cell population maintenance* and *positive regulation of neural precursor cell proliferation,* as well as processes related to central nervous system (CNS)- and neural crest (NC)-identity such as *brain development* and *head development* respectively (Fig 1f). In agreement with their proliferative nature, the *cell cycle* and *Notch signalling pathway* was also enriched in iNBSCs compared to ADFs (Fig 1f). Thus, numerous neural genes were among the genes that contributed strongest to the segregation of iNBSCs from ADFs and pluripotent stem cells (Fig. 1g). These include the neural stem cell markers NESTIN, PAX6 and HES5, as well as neural border and neural crest markers including MSX1, TPAP2a, SOX3 and HOXB2 (Fig. 1g, Extended Data Fig. 1l). In contrast, pluripotency markers (OCT4/ POU5F1 and NANOG) were not up-regulated (Fig. 1g, Extended Data Fig. 1l). Interestingly, while most mesoderm and fibroblast markers (i.e. BRACHYURY/ T, THY1) were strongly down-regulated, some residual expression of COL3A1 was detected in ADF- but not PBMC-derived iNBSCs, which might be indicative of some epigenetic memory (Extended Data Fig. 1l).

In contrast to the transcriptome, the methylation pattern of cells is rather stably associated with the identity and fate of the cell and is independent on certain cellular states (i.e. actively dividing or quiescent). Comparison of the DNA methylation profiles of ADF-converted and iPSC-derived (i)NBSCs with ADFs and hESCs revealed three distinct clusters (Fig 1h). ADF-converted and iPSC- differentiated (i)NBSCs clustered closely, confirming high similarity also at the level of the methylome. The comparison of ADFs with ADF-derived and iPSC-differentiated (i)NBSCs resulted in 9067 differentially methylated promoter regions (FDR adjusted p-val $< 0.05$). Gene set enrichment analysis of hypomethylated promoter sites of iNBSCs compared to ADFs revealed an enrichment for gene ontology terms such as *pattern specification, skeletal system development* and *neuron fate commitment. These data* are consistent with the reprogramming into an NBSC identity also at the epigenetic level (Fig 1i). The analysis of hypermethylated promoter sites revealed processes related to fibroblast identity such as *cell-cell adhesion via plasma membrane adhesion molecules, calcium dependent cell-cell adhesion, innate immune response* and *collagene fibril organisation* (Extended Data Fig 1m), which became apparently silenced. Together, these results demonstrate robust changes in the methylation and transcriptional landscape after reprogramming of ADFs into iNBSCs. In summary, we present a strategy to directly convert various adult human cell types into iNBSCs that are characterized by high expression of neural border markers, sustained self-renewal capacity and the acquisition of an expression and epigenomic landscape consistent with a neural border-like phenotype.

**iNBSCs give rise to central nervous system and neural crest progenitors**

During early post-gastrulation neural development, signaling molecules such as Wnts, BMPs, FGFs and SHH finely orchestrate the development and patterning of the neural plate thereby guiding diversification into central nervous system and neural crest. Hence we examined whether iNBSCs represent a developmental stage prior to the separation into the CNS and NC lineage. To this end, iNBSCs clones were cultured either in the presence of (1) Chir99021, SB431542 (ALK 4,5,7 Inhibitor) and Purmorphamine (CSP) followed by addition of bFGF to induce CNS differentiation or (2) medium containing Chir99021, Alk-5 Inhibitor and BMP4 (CAB) to promote differentiation towards a NC fate (Fig 2a).

To monitor the induction of CNS versus NC fate, we analyzed the expression of CD133 and P75, respectively by flow cytometry. Indeed, when seeding iNBSCs at low density in the presence of CAB, there was a significant increase in $P75^+/CD133^{neg}$ cells compared to CNS-primed cultures, indicative of NC differentiation (Fig 2 a, b). In contrast, CSP+bFGF cultures contained only a minor fraction of $P75^+$ cells, but showed instead a strong and robust increase in $CD133^+/P75^{neg}$ cells (Fig 2a, b). Interestingly, though iNBSC-lines had been established from cultures seeded at clonal density (see also above), iNBSC clones showed a fraction of $P75^+/CD133^{neg}$ cells in CAP medium, indicating that some spontaneous differentiation also occurs under maintenance condition. Quantitative PCRs on sorted $CD133^+/P75^{neg}$ or $P75^+/CD133^{neg}$ confirmed the enrichment for CNS related genes (e.g. PAX6) or NC associated genes (e.g. SOX10 and AP2a), respectively (Fig. 2c, Extended Data Fig. 2a). These data suggest that iNBSCs are able to generate progeny with CNS or NC identity. Although the data are consistent with the hypothesis that iNBSCs are multipotent, and likely mimic a progenitor state prior to CNS and NC lineage commitment, future single cell analysis will be necessary to prove this point formally.

We next investigated whether lineage-committed CNS or NC progenitors can be derived downstream of iNBSCs. Towards this aim, we first cultured iNBSCs for three days in NC-priming conditions, followed by culture in NC stem cell (NCSC) medium containing Chir99021, FGF8, IGF1 and DAPT. Robust induction of migratory crest markers P75 and HNK1 was observed that was paralleled by a decrease in CD133 and SSEA1 expression levels (Fig 2d, Extended Data Fig. 2b). Immunofluorescence and quantitative PCR analysis of $SSEA-1^{neg}/CD133^{neg}/P75^+/HNK1^+$ sorted cells revealed expression of SOX10, P75 and HNK1 triple-positive cells, but not of PAX6, which is indicative for a NCSC-like identity (Fig. 2e, Extended Data Fig. 2c). Furthermore, comparison of expression profiles of iNBSCs and SSEA-1[neg]/CD133[neg]/P75[+]/HNK1[+] NCSC-like cells revealed migratory crest and NC stem cell markers, such as KANK4, BGN, TFAP2A and SOX10 to be among the top regulated genes, while neural progenitor markers such as HES5 and PAX6 were robustly down-regulated (Fig 2f). Gene ontology analysis of the top 100 up-regulated differentially expressed genes in NCSC-like cells versus iNBSCs confirmed this finding and pointed to biological processes such as *neural crest differentiation,* further indicating a robust development of iNBSC identity towards NC lineage (Extended Data Fig. 2d) ([21]).

NC-primed cells could also be differentiated to phenotypically defined CD105[+]/CD44[+]/CD13[+]/CD90[+]/CD146[+] mesenchymal stem cells (Extended Data Fig. 2e). The analysis of gene expression profiles of MSC-like cells compared to iNBSCs demonstrated an up-regulation of genes involved in maintenance and differentiation of MSCs such as COL1A1, GREMLIN1 and TAGLN and was further supported by gene ontology analysis implying biological processes such as *extracellular matrix organization* and *skeletal system development* (Extended Data Fig. 2f, g). Taken together these data demonstrate that NC-primed differentiation of iNBSCs recapitulates physiological stages of NC development and that respective progenitors can be stabilized when using specific culture conditions.

To examine the iNBSCs to CNS differentiation axis and test whether a restricted CNS progenitor could be isolated, we cultured iNBSCs in CSP, and subsequently added bFGF and LIF (CSPFL) while maintaining them on Matrigel™ (MG) (Fig. 2g). Using this approach we obtained mixed cultures that contained highly proliferative, rosette-like colonies (Fig. 2g). Picking of these colonies resulted in the establishment of subclones, which could be maintained in the presence of CSPFL for >20 passages. Intriguingly, compared to iNBSCs, in these subclones of neural progenitors (NPCs) there was a significant down-regulation of the neural border markers TFAP2a, PAX3, PAX7 and MSX1 (Fig 2h, i, Extended Data Fig 2h). In contrast, the neural stem cell markers PAX6, SOX1, SOX2 and NESTIN remained expressed while the neural border marker MSX1 was not detectable anymore (Fig. 2i). Gene set enrichment analysis performed on global expression data of iNBSCs versus NPCs further confirmed this finding. While processes such as *neural crest differentiation, Notch signalling* and *WNT beta catenin signalling* appear to be dominant in iNBSCs, in NPCs *mTORC1 signalling, epithelial mesenchymal transition* and *IL-6 signalling* prevail (Fig 2 J). Taken together this demonstrates a robust change of transcriptional programs and indicates a transition from a neural border-like to a CNS progenitor identity. In addition, FACS analysis for the neural stem cell marker CD133, the early neural progenitor marker CD184 (CXCR4) and P75 revealed robust expression differences between iNBSCs and NPCs. While iNBSCs were positive for CD133 and showed intermediate levels of P75 (CD133[+]P75[int]), NPCs did not express significant level of P75 (CD133[+]P75[neg]) (Extended Data Fig. 2i, j). Interestingly, the neural multipotent progenitor marker CD184 was strongly and homogenously expressed in iNBSCs, while NPCs showed reduced levels, ranging from low to intermediate expression (Extended Data Fig 2k). To explore whether the differentiation of iNBSCs into NPCs was reversible, NPCs were seeded on feeders in CAP medium (without FGF2 and LIF). Since NPCs remained negative for P75 and also retained low expression levels of CD184/CXCR4 (Extended Data Fig 2l, m) no evidence for back-differentiation into iNBSCs could be obtained. To address if NPCs also harbour the potential to give rise to the NC lineages, we cultured NPCs in NC-priming condition. In striking contrast to iNBSCs, the NPCs showed only a very small fraction of P75[+]/ CD133[neg] cells (Extended Data Fig. 2n, o), indicating that NPCs represent neural progenitors with robust CNS-priming (cNPCs) .

To investigate the regional identity of iNBSCs and iNBSC-derived cNPCs, the expression of region specific transcription factors was analyzed. While iNBSCs expressed the anterior hindbrain markers GBX2, IRX3, HOXA2 and HOXB2, cNPCs preferentially expressed GBX2, EN1, FGF8 and PAX2 (Extended Data Fig 2p). There was either no detectable or only low expression of forebrain markers (EMX1, FOXG1) and the more anterior midbrain marker OTX2 in both populations (Extended Data Fig. 2p). Along the dorso-ventral axis, cNPCs expressed the ventral marker NKX6.1 but not NKX2.2, while dorsal markers such as MSX1, PAX3 and PAX7 were much lower expressed compared to iNBSCs (Suppl. Fig 2 h, p). Taken together, the transcription factor landscape of iNBSCs is mostly compatible with a dorsal anterior hindbrain fate, while cNPCs exhibit a ventral mid-hindbrain identity.

To explore whether more mature developmental stages could be derived from iNBSCs, we differentiated CNS-primed iNBSCs for 7 weeks in absence of small molecules before changing to bFGF, EGF and LIF supplemented expansion medium. In doing so, we could obtain a sub-population of SSEA1[+], CD133[+] and glutamate-aspartate-transporter (GLAST; SLC1A3) positive cells, i.e. a phenotype associated with radial glia-like stem cell (RG-like SC) identity ([7], [22]) (Fig 2k, Extended Data Fig. 2q). Cells, triple-positive for SSEA1[+], CD133[+] and GLAST[+], strongly expressed both the glial markers VIMENTIN, GFAP and GLAST, and the stem cell markers PAX6, NESTIN, SOX1 and BLBP (Fig. 2l, m, Extended Data Fig. 2r).

To study the developmental stage of RG-like stem cells compared to their parental iNBSCs, we made use of a recently described machine learning framework (CoNTExT), which was developed to match *in vitro* derived neural cultures with the spatiotemporal transcriptome atlas of the human brain ([23]). This analysis revealed a clear maturation from iNBSCs to RG-like SCs. While iNBSCs mapped to embryonic and early fetal stages, our RG-like SCs mapped more towards later developmental stages (early/late fetal and childhood) (Fig. 2n). This is also reflected in the principal component analysis when combining expression data of iNBSCs and their progeny (Fig. 2o). Thus, iNBSCs, cNPCs, RG-like SCs and NCSCs clustered separately and showed an increase in distance with respect to the NBSC-state. Taken together these data indicate that the iNBSC population behaves in a multipotent manner as these embryonic neural progenitors that can be directed towards a CNS or NC fate. Notably, within each lineage even more restricted progenitors that correspond to previously defined development stages can be generated and stabilized.

## Differentiation of iNBSC into mature CNS and NC cells

To examine the differentiation potential of iNBSCs towards mature cell types, they were subjected to specific differentiation cues. To derive neurons with CNS identity, cells were first cultured in presence of Purmorphamine for 7 days, supplemented with either FGF8 (dopaminergic neurons), all-trans retinoic acid (ATRA) (motor neurons) or without additional cues (GABAergic/ glutamatergic differentiation). Subsequently cultures were switched to maturation medium and analyzed five weeks later (Fig 3a).

iNBSCs displayed a high neurogenic potential and differentiated rapidly upon removal of CAP medium. Immunofluorescence staining and qPCR analysis revealed the presence of glutamatergic (vGLUT2), dopaminergic (FOXA2, TH, EN1), motor neurons (CHAT, ISLET1) and GABAergic neurons (GABA, GAD67) (Fig 3a, b; Extended Data Fig. 3a). Recently the first *in vitro* derivation of functional serotonin neurons from pluripotent stem cells was reported ([24]). Interestingly, applying an adapted protocol, we could derive also serotonergic neurons as demonstrated by co-expression of THP2 and serotonin. Positivity for SYN1 reflected the formation of synapses, which is associated with mature neurons (Fig 3a). In addition to neuronal subtypes, we detected astrocytes and oligodendrocytes after 10 weeks of differentiation, as shown by GFAP/ S100ß. and Olig2/ MBP expression, respectively (Fig. 3a).

To corroborate the neuronal phenotype of *in vitro* differentiated iNBSC-derived neurons, we performed whole cell patch-clamp recordings of 10 weeks old neural cultures. This revealed repetitive trains of action potentials in response to depolarizing voltage steps (12 cells in 3 cultures; Fig. 3c). Moreover, patched neurons also exhibited spontaneous post-synaptic currents, in line with a mature and functional neuronal phenotype (7 cells in 3 cultures; Fig 3 D).

Three-dimensional and organoid cultures have been recently appreciated as a valuable system to spatially model neural development and recapitulate the underlying differentiation processes *in vitro* ([25], [26], ([27], [28]). Hence we embedded iNBSCs as single cell suspension in matrigel and expanded them in CNS- or NC-priming culture conditions, respectively (Fig. 3e). iNBSCs were first cultured in SP for nine days, and subsequently switched to CSP supplemented with bFGF. Of note, single iNBSCs efficiently gave rise to three-dimensional spheres, some of which developed a central lumen during the differentiation process resembling the morphology of a neural tube (Fig. 3e and see below). Immunofluorescence analysis revealed that 3D cultures were positive for the neural progenitor markers SOX1 and NESTIN (Fig. 3e). The expression of PAX6 and SOX2 further confirmed a CNS progenitor identity (Extended Data Fig. 3c). In contrast, when embedded iNBSCs were grown in NC-priming conditions and bFGF (CABF), no spheres were obtained. Instead, iNBSCs differentiated in loosely attached clusters of mesenchymal-type cells that migrated throughout the matrix (Fig. 3e). Cells in 3D cultures were either double-positive for AP2a and SOX10 or expressed SOX10 only, which reflects different stages of NC development (Fig. 3e). Expression analysis of NC-primed 3D cultures confirmed down-regulation of PAX6 and SOX1 and concomitant induction of SOX10 and SOX9. Furthermore there was an up-regulation of the crest-associated EMT marker SNAIL (Extended Data Fig. 3c). Thus, there was a clear divergence in phenotype and gene expression of iNBSC-derived 3D cultures grown in CNS- or NC-priming conditions.

Next, 3D cultures of cNPCs (CSP plus bFGF) were prepared. Interestingly, this approach resulted in the growth of spheres with frequent formation of a neural tube-like structure, including the formation of a central lumen. Immunofluorescence analysis demonstrated SOX1 and luminal PROMININ expression, resembling a neural tube-like structure (Fig. 3f). Interestingly, cNPCs grown in NC-priming condition did not give rise to migratory cells present in the iNBSC differentiations, in line with their more restricted developmental potential characterized above (Extended Data Fig. 3d).

To assess the *in vivo* differentiation potential of iNBSCs, we pre-differentiated GFP-labelled iNBSCs for 8 days, and transplanted them into the striatum of 6 weeks old NOD.*Prkdc^scid^.Il2rg^null^* (NSG) mice. Six weeks post transplantation, mice were sacrificed and the engrafted cells were detected by immunofluorescence. GFP-positive cells included NeuN-positive neurons, GFAP-positive astrocytes and MBP-positive oligodendrocytes demonstrating the differentiation potential of iNBSCs to all three major neural linages and survival of the progeny *in vivo* (Fig. 3g, Extended Data Fig.3e). To confirm that iNBSC-derived neurons exhibit mature functional properties *in vivo,* whole cell patch-clamp analysis of transplanted neurons in acute brain slices was performed. Indeed, GFP-positive neurons exhibited repetitive action potentials upon depolarizing current injection (6 cells from 3 mice; Fig. 3h). The neuronal identity was further confirmed by morphological reconstructions of biocytin-filled patched neurons (Fig. 3i).

To investigate the potential of iNBSCs towards differentiated NC cell types, they were first cultured in NC induction medium and then switched to neural ([29]) or mesenchymal maturation media. After four weeks differentiation was initiated, BRN3a/PERIPHERIN/NAV1.7 triple positive neurons, characteristic for peripheral sensory neurons, could be detected (Fig 3j, Extended Data Figure 3f). In contrast, differentiation of iNBSCs in mesenchymal differentiation media produced smooth muscle actin (SMA) positive cells, alcian blue positive cartilage formation and oil red positive fat cells (Fig 3k). Collectively, our data demonstrate that iNBSCs can differentiate into functional neurons both in vitro and in vivo, show broad neuronal differentiation capacity and can give rise to glial and mesenchymal cells of the CNS and NC.

**Derivation of primary Neural Border Stem Cells from mouse embryos**

The existence of iNBSC and the ability to stabilize them from pluripotent cells *in vitro,* raise the question whether "primary Neural Border Stem Cells" (pNBSC) also emerge during normal embryogenesis. To this end we isolated E7.5 to E10.5 mouse embryos. After mechanic removal of optic and non-neural tissue and enzymatic digestion, the resulting single cell suspension was seeded onto a layer of supportive mouse embryonic fibroblasts and cultured in the presence of CAP (Fig 4a, Extended Data Fig. 4a). Two to three days after plating, clonal lines were established by picking single colonies. Notably, although all embryonic stages gave rise to a variety of neural precursors, only from E8.5 embryos stably proliferating lines could be established, which indicates that the cells are present in the embryo only during a tight developmental time window (Extended Data Fig. 4b). Expression of neural markers was similar to that of human iNBSCs described above. Thus, we detected in pNBSCs Sox1, Sox2, Pax6 and Zfp521 as well as the neural border markers Msx1 and Pax3 (Fig 4b, c, Extended Data Fig 4c, d). Furthermore, pNBSCs showed sustained self-renewal and could be kept in culture for more than 40 passages (> 4 month) without loss of early marker expression (Extended Data Fig. 4e). Moreover, upon single cell sorting pNBSCs showed an up-to 10-times higher clonogenic capacity compared to cultured primary radial glia stem cells isolated from E13.5 stage embryos (Extended Data Fig 4f) ([1]). Interestingly, the culture of pNBSC was also feeder-dependent, although they differentiated more rapidly in the absence of feeders compared to human iNBSCs. The rapid differentiation in the absence of feeders could be partially halted when pNBSCs were cultured on MG in the presence of the Notch-ligands DlI4 and Jagged1 (Extended Data Fig. 4g). This indicates that the feeder-mediated maintenance of the NBSC niche is at least in part mediated by Notch signalling.

To investigate the regional identity of pNBSCs, the expression of region specific transcription factors was analyzed. This analysis revealed that they expressed the anterior hindbrain markers GBX2, IRX3, HOXA2 and HOXB2 (Extended Data Fig. 4h). In contrast to human iNBSCs, the mid-hindbrain marker Fgf8 was already expressed in pNBSCs, but forebrain markers, such as Six3, were also not detected (Extended Data Fig. 4h). Taken together, mouse pNBSCs show a similar regionalization as human iNBSCs, a scenario that is compatible with the notion that they share a dorsal mid-hindbrain to anterior hindbrain identity.

We next addressed the differentiation capability of pNBSCs. When pNBSCs were induced to differentiate in the presence of purmorphamine, Plzf/ Zo1 double-positive rosettes formed within two days, suggesting CNS progenitor activity (Fig 4d). The switch to Fgf2 and Egf at this point led to the stable expansion of Olig2, Sox2 and Nestin positive RG-like cells (Fig 4f). In contrast, treatment of pNBSCs with Chir99021 and BMP4 led to the induction of P75 expression, and gave rise to Ap2a/Sox10 double positive NC cells (Fig 4e, Extended Data Fig. 4i). These data suggest that primary mouse NBSCs have the potential to give rise to CNS, RG-like stem cells and NC progeny, and thus constitute self-renewing, multipotent neural progenitors, akin to human iNBSCs obtained by direct reprogramming.

When pNBSCs were allowed to further differentiate, subsequent to CNS priming by purmorphamine, robust neuronal differentiation (Tuj1) prevailed, but glial progeny, such as oligodendrocytes (O4) and astrocytes (Gfap), was also detected. Within the neural cultures we identified GABA-, TH- and serotonin-positive neurons (Fig 4g, Extended Data Fig. 4j), indicating a broad neuronal differentiation potential. Electrophysiological recordings from neuronal cultures that had matured for three weeks provided functional evidence for the neuronal identity. Patch-clamped cells held in the whole-cell mode exhibited repetitive trains of action potentials in response to depolarizing voltage steps, thus clearly showing that pNBSCs differentiated into mature neurons (Fig. 4h). Upon NC-primed differentiation, peripheral neurons, smooth muscle cells, oil-red positive fat and alcian blue positive cartilage could be observed (Fig. 4i). Finally, we embedded pNBSCs as a single cell suspension in Matrigel and switched them to CNS- or NC-priming culture conditions, respectively (Extended Data Fig. 4k). When pNBSCs were cultured in CSP, small epithelial clusters became apparent after 2 days, some of which formed neural tube-like structures within two additional days (Extended Data Fig 4k). In contrast, as reported above for iNBSCs, continuous culturing in CABF did not result in neural tube-like structures, but instead gave rise to loosely connected mesenchymal-like cells that started migrating throughout the matrix (Extended data Fig. 4k).

To obtain more information regarding gene expression in pNBSCs and their progeny, we performed comparative global gene expression analysis. To this end pNBSCs were differentiated and subsequently FACS-sorted for RG-like SCs (Ssea1$^+$, Glast$^+$) or NC (P75$^+$, Glast-, Ssea1$^-$). Principal component analysis revealed that pNBSCs and their RG-like and NC progeny clustered separately (Fig. 4j). Notably, RG-like SC isolated from E13.5 stage embryos showed great overlap with RG-like SCs derived from pNBSC, confirming their identity (Fig. 4j). In line with the results from human iNBSCs, gene ontology analysis of pNBSCs compared to MEFs showed that processes such as *tube development, regulation of neural precursor cell proliferation, brain development* and *head development* were significantly enriched (Extended data Fig. 4l). Comparison of RG-like SCs or NC with pNBSCs showed an up-regulation of processes such as *gliogenesis, oligodendrocyte differentiation, nervous system development* and *central nervous system development* for RG-like SCs, and processes related to *embryonic cranial skeleton morphogenesis, regulation of cell migration and tissue development* for the neural crest derivates (Extended Data Fig. 4I). The analysis of genes identified by GO analysis in pNBSCs and their progeny also corroborated their respective identity. While pNBSCs showed a specific up-regulation of progenitor markers such as Hes5, Sox1 and Lin28, RG-like cells expressed glia-related markers such as Olig 2, Omg and S100b (Fig. 4k). The NC progeny on the other hand showed up-regulation of NC-associated genes such as Dlx2 and Pdgf receptors and down-regulation of neural progenitor and glia markers respectively (Fig. 4k). Of note, neither pNBSCs nor their progeny showed expression of pluripotency associated markers such as Nanog, Oct4 or Utf1 (Fig. 4k).

In summary, these results suggests that mouse embryo derived pNBSCs are highly similar to directly reprogrammed human iNBSCs. This is reflected by the requirement of the same signalling cues for maintenance, early marker expression, stable long-term proliferation and the potential to recapitulate early neural development by giving rise to naive as well as mature progeny of the CNS and NC lineage. Notably, pNBSCs represent a so far unidentified neural progenitor cell population active during early mouse brain organogenesis and also representing a unique stable pre-rosette population with sustained expansion potential.

### Comparison of iNBSCs and mouse pNBSCs

Human iNBSCs and mouse pNBSCs share many characteristics, including long-term self-renewal capacity, expression of specific markers, and developmental potential. To further extend the comparison to the molecular expression landscape, we analysed the global gene expression profile of mouse pNBSCs and human iNBSCs.

To this end, differential gene expression of iNBSCs versus ADFs and pNBSCs versus MEFs was evaluated for similarity applying the agreement of differential expression procedure (AGDEX), developed to enable cross-species comparisons ([30]). This analysis revealed a positive correlation of 0.457 for differential gene expression of iNBSCs and pNBSCs (Fig. 5a). Gene ontology analysis of the top shared up-regulated genes (log 2 fold change >1; 248 genes) identified processes such as *neural precursor cell proliferation, nervous system development* and *head development to* be up-regulated, while down-regulated DEGs were related to *response to wound healing, tissue development* and *extracellular matrix organization* (Fig. 5b, c). Network analysis of the shared top up-regulated genes (log 2 fold change >1.75; 74 genes) using the STRING database revealed the core-network of pNBSCs and iNBSCs (Fig. 5d) ([31]). This comprises members of the Notch signalling pathway such as *HES5, DLL1* and *NOTCH1* as well a prominent neural transcription factors such as *SOX2, ASCL1* and *PAX6*. Notably, the network comprised two of the four factors used for iNBSC reprogramming, such as POU3f2 (BRN2), SOX2 as well as the ZIC family member ZIC2.

Taken together, pNBSCs and iNBSCs show high similarities in their global expression signature and share an embryonic neural progenitor network, further supporting the notion that pNBSCs reflect a physiological, embryonic counterpart to NBSCs.

### Modelling of SCN9A mediated pain sensitivity syndrome by gene editing of iNBSCs

The self-renewal and extensive differentiation potential of iNBSCs make them a powerful tool to model genetically based human neuronal syndromes. To directly demonstrate this, we used CRISPR/Cas9 mediated gene editing to mutate the voltage-gated sodium-channel Nav1.7, a nociceptor encoded by the *SCN9a* gene. This channel is expressed in the peripheral nervous system and while gain of function mutations in *SCN9a* lead to primary erythromelalgie and paroxysmal pain disorder, loss of function mutations result in congenital insensitivity to pain. To model the loss-of-function situation in human iNBSCs derived sensory neurons, we targeted SCN9a with specific guide RNAs that resulted in mutations in exon 22/27 leading to a truncated version of the gene (Fig. 6a and Extended Data Fig. 5a). $SCN9^{-/-}$ iNBSC subclones harbouring the deleted allele were expanded and subsequently differentiated into sensory neurons. Western blot analysis confirmed the absence of SCN9a in neurons derived from SCN9a$^{-/-}$ iNBSCs but not controls (Fig 6b, Extended Data Fig. 5b). Staining of > 3 weeks old neuronal cultures for the sensory neuron markers BRN3a and Peripherin, ruled out that the mutations in *SCN9A* interfered with sensory neuron differentiation (Fig. 6c). Quantification of Peripherin / BRN3a double-positive neurons showed no significant difference between control and SCN9a$^{-/-}$ derived cultures (Extended Data Fig. 5c). Next, we assessed neuronal activity by calcium flux measurements upon stimulation with $\alpha,\beta$-Methylene-ATP, a selective agonist of P2RX3-receptors ([29]), which are expressed in sensory neurons and whose activation mediates inflammatory pain. As expected addition of $\alpha,\beta$-Methylene-ATP resulted in a robust increase of activity associated with a synchronous calcium flux in most cells (Fig. 6d). In contrast, SCN9a$^{-/-}$ cultures exhibited less activity and paucity of synchronous calcium flux (Fig. 6d, e). To confirm that $\alpha,\beta$-Methylene-ATP was mediated by P2RX3 receptors, we performed $\alpha,\beta$-Methylene-ATP-induced calcium flux measurements in the presence of the selective P2RX3 antagonist A-317491 and as expected, neuronal activity was significantly reduced compared to controls (Fig. 6d, e). In sum, the data show the requirement of SCN9A for pain mediated signalling and more importantly demonstrate the great potential of using human iNBSCs for future modelling or genetic rescue as well associated functional screens in the context of genetically caused neuronal diseases in man.

## Discussion

Here we demonstrate the direct conversion of human skin fibroblasts and blood cells into clonal, multipotent iNBSCs, a thus far unidentified neural progenitor with sustained self-renewal and CNS- and NC-lineage differentiation capacity. We provide evidence that human iNBSCs mimic a mouse neural progenitor cell type derived from E8.5 embroynic neural folds. Both human and mouse cell types share a similar expression profile as well as differentiation and proliferation potential. Therefore NBSCs represent a novel bona fide embryonic, multipotent and self-renewing progenitor, that can be derived either by direct reprogramming of adult somatic cells or by isolation from primary embryonic brain tissue (Fig. 6f).

We and others have previously described direct conversion from mouse embryonic fibroblasts into neural progenitor cells ([32], [33], [11]). In contrast, although attempts to reprogram human somatic cells towards neural progenitors have been reported, it has typically led to heterogeneous and developmentally undefined cell types ([34], [35], [36]). Possible reasons for this phenomenon include (1) the lack of clonal cell line analysis resulting in functional and cellular heterogeneity, (2) the use of pluripotency-factors for conversion with associated persistent expression of the pluripotency makers or (3) absence of in-depth linage analysis. Finally, in these studies the equivalence of the converted cells to naturally existing embryonic brain cell types remains to be shown.

To overcome these issues, we derived iNBSCs by over-expression of a specific set of transcription factors (BKSZ) with chemically defined, serum-free culture medium and a collection of small molecules. They are transgene-independent, show sustained self-renewal, high clonogenicity and a neural border-like expression signature. In contrast to previous reports, we demonstrate the stabilization and characterization of a broad variety of CNS- and NC-lineage progenitors downstream of iNBSCs such as cNPCs, RG-like SCs, NCSCs and MSC-like cells. Moreover, iNBSCs can differentiate into mature progeny of the CNS and NC and also recapitulate early neural development when embedded into a 3D matrix. Thus iNBSCs represent a neural progenitor with well-defined molecular and functional features.

Beside direct conversion there have been various attempts to differentiate and stabilize neural progenitors from human pluripotent stem cells. Notably, NBSCs can also be generated by directed differentiation starting from pluripotent stem cells. The combination of SHH and Notch ligands Jagged-1 and Dll4 has been reported to stabilize rosette-type NSC (R-NSCs), i.e. early progenitors capable of giving rise to CNS and NC progeny ([37]). However, the R-NSC state is transient and long-term culture results in a heterogeneous population. More recently, the use of small molecules has opened new possibilities to stabilize expandable NPCs. The combination of Chir99028, SB431542 and LIF has been reported to stabilize an early mid-hindbrain precursor that retains high neurogenic potential and shows sustained proliferation in culture ([4]). These precursors, similar to iNBSC-derived cNPCs characterized here, show multi-CNS lineage contribution, but their potential to give rise to cell of the NC lineage is very low. Reinhardt and colleagues described neural progenitors with CNS- and NC-differentiation competence that can be maintained in presence of Chir99028 and Purmorphamine on Matrigel-coated plates ([6]). These progenitors show some overlapping functional properties to iNBSCs but display a distinct molecular signature and culture requirements (data not shown).

Considering these and other reports, (i)NBSCs present a thus far unidentified self-renewing neural progenitor with broadest differentiation capacity. However, in the pertinent literature, the term 'neural progenitor' has been used ambiguously to describe a variety of populations that differ in developmental stage, region of origin and expansion capacity ([37], [3], [4], [6]). Therefore a molecular and functional comparison to the corresponding *in vivo* cell type represents an essential step for the proper characterization, especially in case of ectopically reprogrammed cells.

The direct comparison of (i)NBSCs to naturally occurring embryonic neural progenitors in our study clarifies the nature of the reprogrammed cells and directly links reprogramming to development. pNBSCs can be stabilized from embryonic neural tissue, hence ruling out the possibility that the NBSC identity reflects an artificial state accessible only through directed differentiation of pluripotent cells or transcription factor mediated reprogramming. Notably, pNBSCs represent one of the most plastic neural progenitors in mouse neural development described so far and at the same time show sustained proliferation potential. pNBSCs originate from a tight time window of development (E8.5), which raises the possibility that it represents a progenitor only transiently present within the embryonic brain. In the future, it will be interesting to use single cell technologies to further uncover the series of different transient progenitors stages that occur during embryonic brain development.

The close similarity between mouse primary NBSCs and human iNBSCs regarding developmental potential, global gene expression and core regulatory networks indicate that this developmental state is conserved across species. Future studies of NBSCs in other species, particularly in primates, will shed light on conserved and species-specific aspects of these early neural progenitors and will help to gain further insight to human neural development.

(i)NBSCs are highly clonogenic, proliferative and multipotent and can be derived from any individual, healthy or diseased. These characteristics make them an ideal cell population for gene editing approaches. As exemplified here, CRISPR/Cas9-mediated loss-of-function-mutations in SCN9a render sensory neurons insensitivity to $\alpha,\beta$-Methylene-ATP, a P2RX3 agonist involved in inflammatory pain. The pathophysiological response in vitro mimics the functional deficit of SCN9a in patients with congenital insensitivity to pain, who do not experience any modality of pain except for

neuropathic pain ([38]). Hence, iNBSCs, in conjunction with CRISPR/Cas9 gene editing, serve as an ideal tool to model and/ or repair genetic defects underlying neural diseases.

Collectively, we show that (i)NBSCs and progenitors derived thereof, can be generated from various somatic cells, thus offering alternative approaches in obtaining defined and scalable neural progenitors. This strategy not only avoids the generation of pluripotent and cancer prone iPSCs, but also shortcuts inefficient differentiation steps from iPSCs into neural progenitors. Moreover, iNBSCs when used in conjunction with genome editing, constitute a useful tool to study both neural development and, as exemplified in this study, mature cell types with a disease related phenotype. Hence, (i) NBSCs might proof valuable to model and/ or repair genetic defects underlying neural diseases such as for example Parkinson's disease, ataxia or neuropathic diseases and will eventually open new options for regenerative medicine.

**Methods:**

**Example 1: Virus production and reprogramming into induced neural border stem cells**

For the production of lentiviral particles plasmids encoding for pHAGE2-TetO-BKSZ-flox or m2RTTA ([39], Addgene plasmid #20342) were transfected together with helper plasmids psPAX2 (Addgene plasmid #12260) and pMD2.G (Addgene plasmid #12259) into 293FT cell lines (Life technologies) as described elsewhere ([40]).

Lentiviral supernatants containing BKSZ-flox and m2RTTA were mixed in a ratio of 2:1, supplemented with 5 $\mu$g/ml polybrene (Sigma) and used freshly for transduction of $8\times10^5$/ 6 Well primary ADF and pancreas fibroblasts. For reprogramming of PBMCs the lentiviral supernatant was concentrated via ultracentrifugation and PBMCs were transduced in QBSF-60 Stem Cell Medium (Quality Biological) containing 50 $\mu$g/ml Ascorbic Acid (Sigma), 50 ng/ml SCF (R&D Systems), 10 ng/ml IL-3 (R&D Systems), 2 U/ml EPO (R&D Systems), 40 ng/ml IGF-1 (Peprotech), 1 $\mu$M Dexamethasone (Sigma) and 5 $\mu$g/ml polybrene (for details see [41]). The other day $2\times10^5$ transduced PBMCs were transferred onto one well of a 6 Well plate, coated with inactivated MEFs and reprogramming was initiated one day thereafter.

For reprogramming transduced cells were cultured in DMEM/F-12 Glutamax (Life Technologies) containing 64 $\mu$g/ml L-Ascorbic acid 2-phosphate (LAAP, Sigma), ITS-X (1:100, Life Technologies), NEAA (1:100, Life Technologies), 2% FCS, 8% Serum Replacement (Life Technologies) supplemented with 4 $\mu$M Chir99021 (Sigma), 5 $\mu$M Alk5 Inhibitor II (Enzolifesciences), 0.5 $\mu$M Purmorphamine (Sigma), 5$\mu$M Tranylcypromine (Sigma) and 1 $\mu$g/ml Doxycycline (Sigma) and incubated at 37°C in 5% $O_2$ and 5% $CO_2$. During reprogramming medium was changed every other day. When first colonies became visible or latest after 19 days, medium was changed to NBSC maintenance medium. NBSC maintenance medium is composed of DMEM/ F-12 Glutamax (for iNBSCs) or Adv. DMEM/ F12 Glutamax (for pNBSCs) and Neurobasal Medium (1:1) containing 64 $\mu$g/ml LAAP, N2 Supplement (1:100, Life Technologies), B27 without RA (1:50, Life Technologies), 18 $\mu$g/ml Albumax I (Life Technologies) and Glutamax (1:200, Life Technologies), referred to as 'basal medium', and 4 $\mu$M Chir99028, 5$\mu$M Alk5 Inhibitor II and 0.5 $\mu$M Purmorphamine.

Once visible, distinct colonies were manually picked and cultured in NBSC maintenance medium on a layer of inactivated mouse embryonic fibroblasts (feeder) at 37°C in 5% $O_2$ and 5% $CO_2$. Established iNBSC lines were tested for mycoplasma, Squirrel monkey retrovirus, and Epstein-Barr virus contamination prior to analysis. iNBSC lines were routinely split after 4-5 days by treatment with Accutase (Life Technologies) and transferred onto fresh feeders.

**Example 2: Deletion of BSKZ flox**

For the excision of the transgene cassette $5\times10^5$ iNBSCs were seeded onto fresh feeders and transfected with a plasmid encoding for a Cherry-Cre as previously described ([42]). 48 hours later cells were harvested and sorted for Cherry fluorescence. 5000 cherry positive cells were seeded onto a 10 cm dish coated with feeders and incubated until colonies became apparent. Single colonies were picked and checked for transgene removal by transgene-specific PCRs on genomic DNA.

**Example 3: Derivation of human PBMCs and ADFs**

PBMCs were derived from healthy male donors (Age 24-30) and isolated using the Ficoll gradient procedure. PBMCs were used either directly or frozen in 90% Serum Replacement/ 10% DMSO as previously described (for details see [41]).

Adult dermal fibroblasts were derived from skin biopsies of healthy male donors (Age 24-30) as described in Meyer et al. 2015 ([43]). ADFs were expanded until passage 4 and frozen in 90% Serum/ 10% DMSO prior to use.

All cultures were grown at 37°C in 5% $CO_2$ and 20% $O_2$.

Cells were derived under informed consent from all donors and handled in accordance with Ethics Committee II of Heidelberg University approval no. 2009-350N-MA.

## Culture of human fetal pancreas fibroblasts

Human Primary Pancreatic Fibroblast were obtained from Vitro Biopharma and cultured in MSC-Gro™ medium (Vitro Biopharma) supplemented with 10% FCS. Cells were expanded for 4 passages prior to use. Cells were grown at 37°C in 5% $CO_2$ and 20% $O_2$.

## Example 4: Culture of human iPSCs and differentiation into NBSCs

Human iPSCs were routinely grown on feeder cells in DMEM/ F12, 64 $\mu$g/mL LAAP, 1x NEAA, 15% Serum Replacement and 20 ng/ml bFGF. Prior to the differentiation into NBSCs, human IPSCs were cultured for one passage on Matrigel™-coated plates in basal medium supplemented with 20 ng/ml bFGF and 1 ng/ml TGFß1.

When cells reached confluency, iPSCs were harvested by treatment with 1 mg/ml Collagenase II (Life Technologies) and cell clumps were transferred into uncoated petri dishes containing NBSC maintenance medium. At this point the culture was switched from 20% $O_2$ to 5% $O_2$ and subsequently grown under this condition. After five days spheres were plated onto feeder cells and grown in NBSC maintenance medium. The other day, attached spheres were split by treatment with Accutase and $3\times 10^4$ cells were seeded on feeder containing 10 cm dishes. Single colonies were manually picked and clonal lines established.

## Example 5: Differentiation of iNBSCs

## Example 5.1: Differentiation towards cNPCs

Differentiation towards cNPCs was initiated by seeding NBSCs on Matrigel™-coated plates (1:36, growth factor reduced, BD Biosciences) and switch from NBSC maintenance medium to basal medium with 4 $\mu$M Chir99028, 3 $\mu$M SB431542 (Sigma), 0.5 $\mu$m Purmorphamine. After 5 days 10 ng/ml bFGF (Peprotech) and 10 ng/ml LIF was added to the medium (Peprotech) (CSPFL).

After one passage in CSPFL, 5000 cells/ 6 Well were seeded on Matrigel-coated plates and single colonies were manually picked. cNPC subclones could be maintained on MG-coated plates in CSPFL for > 30 passages (>3 months) and were routinely split after 3 days by treatment with Accutase. All cultures were incubated at 37°C, 5% $CO_2$ and 5% $O_2$.

## Example 5.2: Differentiation towards RG-like cells

NBSCs were seeded on Matrigel™-coated plates and cultured in basal medium supplemented with 1 $\mu$M Purmorphamine and 10 ng/ml FGF8 for one week, followed by culture in basal medium with 1 $\mu$M Purmorphamine for one additional day. Thereafter cultures were grown in basal medium containing 10 ng/ml BDNF and 10 ng/ml GDNF for 7 more weeks. Subsequently, cells were cultured in radial glia medium, comprised of basal medium, 20 ng/ml bFGF, 20 ng/ml EGF and 10 ng/ml LIF. When proliferative, RG-like cells became apparent, cultures were treated with Accutase and expanded on Matrigel-coated plates in radial glia medium. Finally, cultures were enriched for RG-like cells by cell sorting for CD133/2, SSEA1 and GLAST. All cultures were grown at 37°C, 5% $CO_2$ and 20% $O_2$.

## Example 5.3: Differentiation towards NCSC-like cells

To initiate crest differentiation $1\times 10^5$/ 6 Well iNBSCs were seeded on Matrigel-coated plates and grown in basal medium supplemented with 4 $\mu$M Chir99028, 5 $\mu$M Alk5 Inhibitor II and 10 ng/ml BMP4 (CAB) (Peprotech) for three days. Thereafter, medium was switched to basal medium supplemented with 4 $\mu$M Chir99028, 10 ng/ml FGF8 (Peprotech), 10 ng/ml IGF1 (Peprotech) and 1 $\mu$M DAPT (Sigma). Five days later NCSC-like cells were purified by cell sorting for SSEA-1$^{neg}$CD133$^{neg}$P75$^+$HNK1$^+$. All cultures were grown at 37°C, 5% $CO_2$ and 20% $O_2$.

## Example 5.4: Differentiation towards MSC-like cells and neural crest progeny

To derive mesenchymal crest cells, iNBSCs were first seeded on Matrigel-coated plates and cultured in 4 $\mu$M Chir99028, 10 ng/ml BMP4 and 10 $\mu$M DAPT for 7 days. Thereafter, cells were cultured in basal medium containing 10 ng/ml bFGF and 10 ng/ml IGF-1 for > 5 passages. Subsequently, MSC-like cells were stabilized by switching cultures to mesenchymal stem cell medium (Gibco). MSC-like cells were cultured for > 2 passages prior to analysis.

In order to derive mature mesenchymal crest cells NC-primed cultures were treated for 5 days in mesenchymal induction medium comprising DMEM/ F12, 64 $\mu$g/ml LAAP and 10% FCS. Thereafter, cells were cultured in StemPro® Adipogenesis Differentiation Kit (Life Technologies), StemPro® Chondrocyte Differentiation Kit (Life Technologies) or kept in mesenchymal induction medium to generate adipocytes, chondrocytes or smooth muscle, respectively.

To induce sensory neurons, iNBSCs were grown on MG-coated plates in basal medium in presence of 3 μM Chir99021, 10 μM DAPT (Sigma) and 10 μM SU5402 (Sigma) for 10 days. Subsequently cultures were grown in maturation medium comprising basal medium, 10 ng/ml BDNF, 10 ng/ml GDNF, 10 ng/ml NT-3 (Peprotech) and 25 ng/ml NGF (Peprotech) for at least another three weeks ([44]).

**Example 5.5: Differentiation towards mature CNS progeny**

To induce neuronal differentiation iNBSCs were seeded on MG-coated plates and iNBSCs maintenance medium was switched to neural induction medium containing 1 μM Purmorphamine (undirected differentiation), 1 μM Purmorphamine and 10 ng/ml FGF8 (dopaminergic differentiation) or 1 μM Purmorhamine and 1 μM all-trans retinoic acid (Sigma) (motoneural differentiation) for one week. Serotonergic differentiation was initiated by culture of iNBSCs in basal medium, 3 μM Chir99028, 3 μM SB431542 and 1 μM Purmorphamine for one week, followed by culture in 3 μM Chir99028, 3 μM SB431542, 1 μM Purmorphamine and 10 ng/ml FGF4 for another week and finally switching to 1 μM Purmorphamine for two days.

Subsequent to neural induction, cultures were grown in neuronal maturation medium comprising basal medium, 500 μM dbcAMP (Sigma), 1 ng/ml TGFß3, 10 ng/ml BDNF and 10 ng/ml GDNF for at least 5 more weeks. Astrocytes and oligodendrocytes could be found within neuronal cultures, starting after 5 weeks of differentiation.

**Example 5.6: 3D Differentiation of iNBSCs**

In order to differentiated iNBSCs and cNPCs as three-dimensional cultures, a single cell suspension of $2 \times 10^4$ was resuspended in 10 μl of basal medium and mixed with 150 μl of matrigel on ice. Next, 30 μl drops of the mix were distributed on cover slips and incubated at 37°C for 10 minutes. After gelling the differentiation medium was applied onto the embedded cells.

For CNS-primed differentiation, embedded iNBSCs cultures were first treated with basal medium supplemented with 3 μM SB431542 and 1 μM Purmorphamine for nine days. Thereafter cultures were switched to basal medium supplemented with 3 μM Chir99028, 3 μM SB431542, 1 μM Purmorphamine and 20 ng/ml bFGF and cultured for 4-6 days. Embedded cNPCs did not have to undergo CNS-priming and were directly cultured in basal medium supplemented with 3 μM Chir99028, 3 μM SB431542, 1 μM Purmorphamine and 20 ng/ml bFGF.

For neural crest-primed differentiation, embedded iNBSC cultures were cultured in basal medium supplemented with 4 μM Chir99028, 5 μM Alk5-Inh., 10 ng/ml BMP4 and 20 ng/ml bFGF for at least 12 days.

Cultures were either fixed with 4% paraformaldehyde and analyzed by confocal microscopy or total RNA was extracted using the ARCTURUS PicoPure RNA Isolation Kit.

**Example 6: In vivo experiments in mice**

**Mice:**

Six- to 12-week-old mice were used throughout the study. Embryos at different gestation stages were derived from Tomato-mice (Gt(ROSA)26Sor<sup>tm4(ACTB-tdTomato,-EGFP)Luo</sup>). Transplantation were performed into female NOD.*Prkdc<sup>scid</sup>.Il2rg<sup>null</sup>* (NSG) mice. All mice were maintained at the DKFZ under specific pathogen-free (SPF) conditions in individually ventilated cages (IVCs). No statistical methods were used to estimate sample size and mouse experiments were not randomised nor blinded.

Animal procedures were performed according to protocols approved by the German authorities, Regierungspräsidium Karlsruhe (Nr.Z110/02, DKFZ 299 and G184-13).

**Transplantation**

Prior to transplation iNBSCs were allowed to initiate differentiation by cultivation in basal medium supplemented with 1 μM Purmorphamine and 10 ng/mL FGF8 on MG coated plates for 8 days. On the day of transplantation primed cultures were dissociated to a single cell suspension and resuspended in medium at a concentration of $5 \times 10^4$ cells per μl. 6 weeks old female NSG mice were anesthetized with isoflurane, mounted in a stereotactic apparatus and kept under isoflurane anaesthesia during surgery. 3 μl of neural cell suspension was bilaterally transfused into the striatum using a glass micropipette. The following coordinates were used for transplantation: from bregma and the brain surface, anterior/posterior: 0 mm; medial/lateral ± 2.5; dorsal/ventral -2.5 mm.

The scalp incision was sutured, and post-surgery analgesics were given to aid recovery (0.03 mg/kg KG Metamizol).

Electrophysiological experiments were performed 8-12 weeks after the treatments.

**Derivation of pNBSCs**

Male and female tomato mice were paired and checked for vaginal plug the following morning. Positive plug test was considered 0.5 days postcoitum. Embryos were collected at day 8.5 postcoitum and optic and non-neural tissue was removed mechanically. Neural tissue was digested with Accutase and the resulting single cell suspension was seeded onto a layer of mouse embryonic fibroblasts and cultured in NBSC maintenance medium in 5% $CO_2$, 5% $O_2$ at 37°C. Two to three days after seeding, single colonies were mechanically picked and clonal pNBSC lines established. pNBSC lines were routinely split every three days on fresh feeder cells by treatment with Accuase and could be maintained in culture for > 40 passages (> 4 months).

**Differentiation of pNBSCs**

In order to differentiate pNBSCs towards the CNS, pNBSCs were seeded on matrigel-coated plates and cultured in basal medium supplemented with 1 $\mu$M Purmorphamine for 3 days. Rossette-like structures could be observed 2-4 days after differentiation was initiated. RG-like SCs were stabilized by switching culture medium of rosette-like cells to basal medium, supplemented with 20 ng/ml bFGF and 20 ng/ml EGF. Cells were expanded for > 3 passages before RG-like SCs were further enriched by FACS sorting for Ssea1$^+$Glast$^+$. Mature progeny such as neurons, astrocytes and oligodendrocytes were derived by culturing pNBSCs in basal medium supplemented with 1 $\mu$M Purmorphamine for 3 days and subsequent switch to basal medium containing 10 ng/ml Bdnf and 10 ng/ml Gdnf for > 3 weeks.

To derive NCSC-like cells, $1\times10^4$ pNBSCs were seeded onto a matrigel-coated 6 well plate and cultured in basal medium supplemented with 4 $\mu$M Chir99028 and 10 ng/ml BMP4 for 3 days. Thereafter, cells were cultured in basal medium supplemented with 10ng/ml bFGF and 10 ng/ml EGF for 4 days and enriched for NCSC-like cells by FASC sorting for P75$^+$Glast$^-$Ssea1$^-$. Oil red positive adipocytes were obtained by culture of pNBSCs in 4 $\mu$M Chir99028 and 10 ng/ml BMP4 for 3 days, followed by addition of 10 ng/ml bFGF to the medium for 2 days and switch to basal medium supplemented with 10 ng/ml bFGF and 10 ng/ml EGF for another 3 weeks. Chondrocytes and smooth muscle cells were derived by culture of pNBSCs in 4 $\mu$M Chir99028 and 10 ng/ml BMP4 for 3 days, addition of 10 ng/ml bFGF to the medium for 2 days and subsequent switch to basal medium supplemented with 10% FCS. Peripheral neurons were differentiated from pNBSCs by culture in Chir99028 and 10 ng/ml BMP4 for 3 days, followed by switching to basal medium supplemented with 10 ng/ml BDNF and 10 ng/ml GDNF for two weeks.

**Derivation of primary RG from mouse embryos**

Male and female tomato mice were paired and checked for vaginal plug the following morning. Positive plug test was considered 0.5 days postcoitum. Embryos were collected at day 13.5 postcoitum and medial and lateral ganglionic eminences were mechanically isolated. Neural tissue from individual embryos was digested by treatment with Accutase. The resulting single cell suspension was transferred to petri-dishes and cultured in basal medium supplemented with 20 ng/ ml bFGF and 20 ng/ml EGF for 5 days. The resulting spheres were plated onto fibronectin-coated cell culture dishes and expanded for at least 3 passages before FACS sorting for Glast+Ssea1+ cells was performed and used for downstream analysis.

**3D Differentiation of pNBSCs**

A single cell suspension of $2\times10^4$ pNBSCs was resuspended in 10 $\mu$l of basal medium and mixed with 150 $\mu$l of matrigel on ice. Next, 30 $\mu$l drops of the mix was distributed on cover slips and incubated at 37°C for 10 minutes. After gelling the differentiation medium was applied onto the embedded cells.

For CNS-primed differentiation, embedded pNBSCs cultures were treated with basal medium supplemented with 4 $\mu$M Chir99028, 3 $\mu$M SB431542 and 1 $\mu$M Purmorphamine for six days until neural tube-like structures had formed.

For neural crest-primed differentiation, embedded pNBSC cultures were cultured in basal medium supplemented with 4 $\mu$M Chir99028, 5 $\mu$M Alk5-Inh., 10 ng/ml BMP4 and 20 ng/ml bFGF for at least 10 days.

**Electrophysiology**

**Slice preparation**

Electrophysiological recordings were performed from 6 to 12 weeks old female mice. We recorded from acute coronal slices (300 $\mu$m) containing striatum.

Mice were deeply anaesthetized with inhaled isoflurane, followed by transcardially perfusion with ~30 ml ice-cold sucrose solution containing (in mM) 212 sucrose, 26 NaHCO, 1.25 $NaH_2PO_4$, 3 KCl, 7 $MgCl_2$, 10 glucose and 0.2 CaCl2,

**EP 3 418 378 B1**

oxygenated with carbogen gas (95% $O_2$/ 5% $CO_2$, pH 7.4). Sections were cut in ice-cold oxygenated sucrose solution, followed by incubation in oxygenated extracellular solution containing (in mM) 12.5 NaCl, 2.5 NaHCOs, 0.125 $NaH_2PO_4$, 0.25 KCl, 2 $CaCl_2$, 1 $MgCl_2$ and 25 glucose. Cells in striatum were visualized with DIC optics and epifluorescence was used to detect GFP fluorescence.

## Whole-cell recordings

Whole-cell patch-clamp recordings were performed at 30 to 32°C bath temperature. Individual slices or coverslips were placed in a submerged recording chamber mounted on an upright microscope (Olympus BW-X51) and continuously perfused with oxygenated extracellular solution. Recording pipettes were pulled from borosilicate glass capillaries and had tip resistances of 5-8 MΩ. Liquid junction potentials were not corrected. Series resistance was maximally compensated and continuously monitored during the recordings. Cells were discarded if no "Giga seal" was initially obtained or series resistance changed more than 20% or was higher than 40 MΩ.

The following intracellular solutions were used: low Cl⁻ potassium-based solution containing (in mM) 130 K-gluconate, 10 Nagluconate, 10 Hepes, 10 phosphocreatine, 4 NaCl, 4 Mg-ATP and 0.3 GTP, pH adjusted to 7.2 with KOH for firing patterns. High Cl⁻ solution containing (in mM) 127.5 KCl, 11 EGTA, 10 Hepes, 1 $CaCl_2$, 2 $MgCl_2$ and 2 Mg-ATP (pH 7.3) for spontaneous activity in cell culture. Cs⁺-based solution containing (in mM) 120 Cs⁺-gluconate, 10 CsCl, 10 Hepes, 0.2 EGTA, 8 NaCl, 10 phosphocreatine, 2 Mg-ATP and 0.3 GTP, pH 7.3 adjusted with CsOH for spontaneous activity in transplanted neurons.

For subsequent morphological and immunocytochemical characterization of patched cells, biocytin (circa 10 mg/ml; Sigma) was added to the respective intracellular solution.

Cells were initially kept in cell-attached mode. After achieving a "Giga seal", whole-cell configuration was established and firing patterns were analyzed in current-clamp mode at resting membrane potential by applying 1 s current pulses, starting from -30 pA in 5 pA steps until maximal firing frequency was reached for cell culture and from -200 pA in 20 pA steps for acute slices. Individual traces upon -30 pA/-200 pA current injection, at actionpotential threshold (for intermediated excitatory cell types) were selected for illustration of firing pattern.

Spontaneous activity of the neurons was recorded at a holding potential of -70 mV.

All recordings were made using HEKA PatchMaster EPC 10 amplifier and signals were filtered at 3 kHz, sampled at 10 or 20 kHz. Data analysis was done offline using HEKA software FitMaster and Clampfit (Molecular Devices, USA).

## Cell identification and reconstruction

Acute slices with biocytin-filled cells in the MEC were fixed overnight in 4% paraformaldehyde, followed by extensive washing with PBS.

For morphological reconstructions, biocytin-filled MEC cells were identified via 3,3'-diaminbenzidine (DAB) staining. Sections quenched in 1% H2O2 for 5 min. After renewed washing, sections were permeabilized in PBS with 1% Triton X-100 for 1 hr. Subsequently, sections were incubated with avidin-biotin-horseradish-peroxidase complex in PBS for 2 hrs at room temperature. Following washing in PBS, sections were developed in DAB and mounted in Mowiol. Labeled cells were reconstructed using the Neurolucida (MBF bioscience, Willston, VT, USA) tracing program.

## Calcium Imaging

Cell cultures were incubated in a solution containing the cell-permeable fluorescent Ca2+ indicator Fluo-3 AM (2 μM; F1242 Thermo ScientificTM) and Cell Tracker Red CMTPX (1 μM; C34552, Thermo ScientificTM) to delineate cell membranes. Dye loading was carried out in an incubator at 37°C, 5% $CO_2$ for 30 min.

Imaging of fluorescently labeled cells was performed on a TCS SP5 microscope (Leica) equipped with a 20x (1 numerical aperture) water-immersion objective. Images (512×512 pixels) were acquired at 1000 Hz speed every 0.8-1.6 seconds with 0.5 μm per pixel resolution in the xy dimension, and 3-4 μm steps in the z dimension. Argon and HeNe-543 lasers were used to excite Fluo-3 AM and Cell Tracker Red CMTPX dyes, respectively. Artificial cerebrospinal fluid (ACSF) containing (in mM): 120 NaCl, 3.5 KCl, 2.5 $CaCl_2$, 1.3 $MgSO_4$, 1.25 $NaH_2PO_4$, 25 $NaHCO_3$, 25 glucose (pH 7.2) was applied by a pump perfusion system with a constant flux (1.5 ml/min) that continuously renewed the buffer in the recording chamber. After recording baseline fluorescence for 2 minutes, $\alpha$, $\beta$ Methylene ATP was applied (30 μM in ACSF; COMPANY) for 2 minutes. Recordings continued for up to 10 minutes in total. We added a non-nucleotide P2X3 and P2X2/3 receptor antagonist (1 μM; A-317491, COMPANY) together with the dye loading and were also present in ACSF during the whole recording. Leica Application Suite AF software and FIJI software ([45]) was used to record and measure fluorescent activity, respectively, in 3-5 independent experiments per group.

We obtained relative fluorescence changes (Fi/F0), where F0 is the fluorescence image formed by averaging the first 50 frames of the sequence, and F(i) represents each (i) frame of the recording. Curves were normalized by subtracting a linear regression line fitted through the first and last 50 values and maximum peak intensity was aligned at respective time points were applicable (WT measurements).

We then studied global calcium activity by averaging fluorescence intensity in the whole image before and in response to stimulation with $\alpha,\beta$ Methlynene ATP in WT- (n=3) and SCN9-/- (n=4) neuronal cultures.

Statistical analyses were performed using Prism 6. Differences between groups were examined using Student's t test. Values of p < 0.05 were considered statistically significant for the rest of the analyses.

**Flow Cytometry**

Cells were harvested by treatment with Accutase and washed twice with unsupplemented DMEM/ F12. Thereafter cells were resuspended in medium and stained with for 30 minutes at 4°C using the following antibodies: anti-SSEA1 (MC480)-V450 (Becton Dickinson), anti-CD133/2 (293C3)-FITC (Miltenyi Biotec), anti-CD271 (ME20.4-1.H4)-PE (Miltenyi Biotec), anti-CD271 (ME20.4-1.H4)-APC (Miltenyi Biotec), anti-GLAST (ACSA-1)-APC (Miltenyi Biotec), anti-HNK1 (TB01)-PeCy7 (eBioscience), ms anti-HNK1 (clone VC1.1, Sigma), Donkey Anti-Mouse Alexa Fluor 488 (Abcam, ab150105), anti-CXCR4 (12G5)-PeCy5 (BioLegend), anti-Cxcr4(L276F12)-BV421 (BioLegend), anti-PSA-NCAM (clone: 2-2B)-PE (Miltenyi Biotec), anti-CD44(G44-26)-PE (BD Pharmingen), anti-CD105 (43A3)-FITC (BioLegend), anti-CD90 (5E10)-BV421 (BioLegend), anti-CD146-Alexa647 (Biolegend), anti-CD13 (WM15)-APCCy7 (BioLegend). FACS analysis was performed on LSRII or LSR Fortessa flow cytometers (Becton Dickinson, San Jose, CA). Data were analyzed using the FlowJo software (Tree Star, Ashland, OR). Cell sorting experiments were carried out on a BD FACSAria™ III sorter (Becton Dickinson, San Jose, CA). The following sort parameters were used: 100 $\mu$m nozzle; ca. 2000 events/second.

**Immunofluorescence**

Cells were fixed in PBS with 4% paraformaldehyde (Electron Microscopy Sciences, 19208) for 15 minutes. Fixed cells were then washed three times with PBS and blocked for one hour in PBS containing 0.1% Triton X-100 and 1% BSA. Primary antibodies were applied in 0.1% Triton X-100 and 1% BSA at 4°C over night. For list of primary antibodies and dilutions used in this study see extended Data Table 1. Subsequent to three times washing with PBS, cells were incubated with secondary antibodies for two hours at room temperature.

Following DAPI (Sigma, D9542) staining, cells were mounted (DAKO, S3035) and analyzed on a LSM 710 ConfoCor 3 confocal microscope (Zeiss).

**Immunohistochemical analysis**

For IHC analysis, transplanted mouse brains were fixed with 4% paraformaldehyde over night and consecutively dehydrated using 20% sucrose in PBS. Thereafter, brains were embedded in 3% agarose and 100 $\mu$m coronal sections were prepared using a Leica VT1000S sliding microtome. Brain slices were blocked in 3% goat serum, 0.25% Triton-X in TBS for one hour at 4°C. Primary antibodies (Extended Data Table 1) were applied in 3% goat serum, 0.25% Triton-X in TBS for 72 hours at 4°C. After washing slices in TBS for three times, secondary antibodies were incubated in 3% goat serum, 0.25% Triton-X in TBS for two hours at room temperature. Following DAPI staining, brain slices were mounted and analyzed on a LSM 710 ConfoCor 3 confocal microscope (Zeiss).

**Oil Red o staining**

Adipocyte differentiations were fixed in 4% paraformaldehyde for 15 minutes at room temperature. After washing twice with ddH$_2$0, cells were incubated with 60% isopropanol for 5 minutes and completely dried afterwards. Fresh Oil Red staining solution, consisting of 3.5 mg/ml Red O (Sigma) in 60% isopropanol, was applied on cells for 10 minutes at room temperature. After washing cells 4 times with ddH$_2$O microscope images (Nikon Eclipse Ti-E) were acquired.

**Alcian blue staining**

Chondrocyte differentiations were fixed in 4% paraformaldehyde for 15 minutes and rinsed three times with PBS. Alcian blue solution, consisting of 10 mg/ml Alcian Blue GX (Sigma) in 3% acetic acid, was applied for 1 hour and rinsed twice with 0.1 M HCl. After washing cells twice with PBS microscope images (Nikon Eclipse TiE) were acquired.

**Western Blot**

WT and SCN9a -/- iNBSCs were differentiated into sensory neurons for at least three weeks before whole cell lysates were prepared using RIPA buffer (Cell Signaling Technology), 1 mM PMSF (Sigma), 1 mM EDTA and Halt Protease-Phosphates Inhibitor Cocktail (Pierce). After denaturing lysates in 5% SDS at 95°C, protein samples were resolved on 4-12% TGX gels (Criterion, Bio-Rad) with TGS (Tris-Glycine-SDS) running buffer (Bio-Rad) and blotted on PVDF membranes (Trans-Blot TURBO, Bio-Rad). Membranes were blocked with TBS containing 0.3% (vol/vol) Tween-20 and 5% (wt/vol) BSA powder for 1 hour. Primary antibodies (Extended Data Table 1) were incubated over night at 4°C on a shaker. After thorough washing, Secondary HRP-coupled antibodies were incubated in TBS containing 0.3% Tween-20 for 1 hour at RT. Membranes were washed and immunocomplexes were visualized using the ECL kit (Amersham Internationsal).

**CRISPR-Cas9-mediated knockout**

Guide RNAs were ordered as DNA oligos (Sigma) and cloned into the pSpCas9(BB)-2A-Puro vector (PX459) (A gift from Feng Zhang, via Addgene) as described elsewhere ([46]). $0.5-2\times10^6$ iNBSCs were nucleofected with 0.5-2ng of plasmid DNA using Nucleofector™ Kits for Mouse Neural Stem Cells (Lonza) with program A-033 and seeded on fresh feeder cells. Transfected cultures were harvested after 48 hours, sorted for GFP fluorescence and plated onto feeders. Individual colonies were manually isolated 5-7 days later. Genomic DNA was extracted using the QIAamp DNA mini kit (QIAGEN) and guide RNA target sites were amplified and analyzed by Sanger sequencing (GATC). Biallelic sequences were deconvoluted using CRISP-ID ([47])

**Gene Expression Analysis by Quantitative PCR with Reverse Transcription**

Total RNA was isolated using the ARCTURUS PicoPure RNA Isolation Kit (Life Technologies, Invitrogene) including on-column DNA digestion (Qiagen, 79254). Reverse transcription was performed using the high capacity cDNA synthesis kit (Applied Bioystems). Real-time quantitative PCRs were run in ABI Power SYBR Green Mastermix (Applied Bioystems, 4309155) on a ViiA7 machine. Results were analyzed using the ViiA7-software V1.2.4. Expression was normalized to the housekeeping gene GAPDH. All PCR reactions were carried out as technical triplicates. Samples showing low RNA quality and/ or detection of the house keeping gene for amplification cycles >25 were excluded from the study. For primers used in this study see Extended Data Table 2.

**Microarray analysis**

Total RNA was isolated using the ARCTURUS PicoPure RNA Isolation Kit including on-column DNA digestion. RNA expression profiling using the humanHT-12 v4 Expression BeadChip (Illumina) or Mouse 430 V2.0 GeneChips (Affymetrix) was performed according to the manufacturer's instructions at the DKFZ Genomics and Proteomics Core Facility (Heidelberg, Germany).

Raw data were obtained from DKFZ Genomics and Proteomics Core Facility and merged with publically available expression data from the Gene Expression Omnibus database. Datasets used in this study:

GSE40838

> GSE40838_Patient1_repl1
> GSE40838_Patient1_repl2
> GSE40838_Patient2_repl1
> GSE40838_Patient2_repl2
> GSE40838_Patient3_repl1

GSE51980

> GSE51980_FIB_y
> GSE51980_ESC_H9_y
> GSE51980_ESC_H9_y.1

GSE69486
GSE69486_FIB

GSE34904

> GSE34904_ESC_H1_1

GSE34904_ESC_H1_2

The whole dataset was quantile normalized (self-written function) and log2-transformed.

```
def quant_norm(df):
df_num = df._get_numeric_data()
df_anno = df.drop(df_num.columns, axis=1)
df_ranks = df.apply(rankdata, axis = 0)
df_ranks = df_ranks.applymap(int)
df_sorted = df.apply(np.sort, axis = 0)
means = np.mean(df_sorted, axis = 1)
output = df_ranks.applymap(lambda x: helper_function(means, x))
output_2 = pd.concat([df_anno, output], axis=1)

return output_2
```

Principal component analyses were computed using the PCA function from sklearn.decomposition and visualized (seaborn).

Gene expression heatmaps were visualized as clustered heatmap (seaborn, clustermap). In order to generate PCA-based expression heatmaps, the 200 probes showing highest contribution were extracted applying the formula

$$\sqrt[2]{(PC1^2 + PC2^2)}.$$

Differentially expressed genes between two samples were identified using ttest_ind from the scipy.stats package, p-values were corrected using the Benjamini-Hochberg false discovery control (stats, R-package).

Gene ontology analysis and geneset enrichment analysis were performed using STRING Version 10.0 [48], EnrichR [49] and the Broad Institute GSEA software [50]. Geneset enrichment analyses were run on 'Hallmark gene sets' and gene set from the 'Wikipathway2017' library.

In order to map neural populations with the spatiotemporal atlas of the human brain, iNBSCs-derived RG-like SCs and iNBSCs were submitted to the online tool of the machine-learning framework CoNTExT [51]. Data were uploaded as suggested by the authors, using an identifier file and the respective subset of identifiers from the expression data.

To compare human and mouse data, iNBSCs/ADFs and pNBSCs/Mefs were analyzed using the AGDEX package [52] using homology information from BioMart (R). Log2 expression fold changes were correlated and genes that were differentially expressed in both comparisons (p < 0.05) to an absolute fold change of > 1 were analyzed for gene ontology enrichment.

## Methylation analysis

Genomic DNA was extracted using the DNeasy Blood and Tissue Kit (Qiagen) according to the manufacturer's instructions. DNA methylation profiling using the Illumina Infinium HumanMethylation450 BeadChip array was performed according to the manufacturer's instructions at the DKFZ Genomics and Proteomics Core Facility (Heidelberg, Germany). From the GEO repository, reference data from the following datasets was acquired:

Reference data for hESCs and human ADFs were obtained from the Gene Expression Omnibus database:

GSE52025:

GSM1257669: GM02704
GSM1257670: GM02706
GSM1257671: GM01650
GSM1257672: GM01653

GSE61461:

GSM1505345 B105-ES
GSM1505346 B152-ES
GSM1505347 B160-ES
GSM1505348 B209-ES
GSM1505349 B220-ES
GSM1505350 B312-ES

All methylation data was processed using the minfi package ([53]) from the bioconductor suite. Probes with a detection p-value < 0.01 or coinciding with known SNPs and all probes on X- and Y-chromosomes were excluded. The dataset was normalized using the preprocessIllumina function and visualized using classical multidimensional scaling.

For differential methylation analysis, promoter methylation was analyzed using the RnBeads ([54]) package (Bioconductor). Promoters were ranked based on their combined rank statistic (RnBeads) and ordered from most significantly hyper- to most significantly hypomethylated and used as input for GSEA with a gene set file reduced to genes covered by the 450k array. GSEA was run with the "classical" enrichment statistic ([50]).

### Statistical analysis

In all experiments at least three biological replicates were used. Quantitative results were analyzed by two-sided unpaired Student's t-test using GraphPad Prism 6.

Estimation of variation within each group was determined by s.e.m. unless otherwise indicated. Levels of significance were determined as follows: * $p<0.05$; ** $p<0.01$; *** $p<0.001$; **** $p<0.0001$.

### Data availability

Gene expression and DNA methylation data that support the findings of this study has been deposited at Gene Expression Omnibus database and is available at ArrayExpress.

**Extended Data Table 1:**

| Human Antibodies | | | | |
|---|---|---|---|---|
| **Antigen** | **Dilution** | **Host species** | **company** | **clone/ Product-ID** |
| BRN3A | 500x | rb | LifeSpan BioSciences | LS-C12182 |
| CHAT | 500x | gt | Merck Millipore | AB144P |
| EN1 | 100x | gt | Santa Cruz, USA | D-20 |
| FOXA2 | 100x | ms | Abcam | ab60721 |
| GABA | 500x | rb | Sigma-Aldrich | A-2052 |
| GAD67 | 500x | ms | Millipore | MAB5406 |
| GFAP | 100x | rb | Dako | Z0334 |
| GFP | 1000x | gt | Abcam | ab5450 |
| GLAST | 100x | rb | abcam | ab416 |
| HNK1 | 50x | ms | Sigma-Aldrich | C6680-50TST |
| ISLET | 30x | ms | DSHB | 40.2D6 |
| MBP | 25x | rt | Abcam | ab7349 |
| MSX1 | 30x | ms | DSHB | 4G1 |
| NAV1.7 | 1000x | ms | Abcam | ab85015 |
| NESTIN | 200x | rb | Abcam | ab22035 |
| NEUN | 1000x | bio | Millipore | MAB377B |
| OLIG2 | 100x | gt | R&D Systems | AF2418 |
| PAX6 | 200x | rb | Life Technologies | 42-6600 |
| PERIPHERIN | 300x | ck | Abcam | ab39374 |
| PROMININ | 30x | bio | Miltenyi Biotec | 130-101-851 |
| S100B | 1000x | ms | Sigma-Aldrich | S2532 |
| SMA | 100x | ms | Dako | M0851 |
| SOX1 | 200x | gt | R&D Systems | AF3369 |
| SOX10 | 200x | gt | R&D | AF2864-SP |

(continued)

| Human Antibodies | | | | |
|---|---|---|---|---|
| **Antigen** | **Dilution** | **Host species** | **company** | **clone/ Product-ID** |
| Sox2 | 100x | ms | R&D Systems | MAB2018 |
| SYN1 | 500x | ms | Synaptic Systems | 106 011 |
| TFAP2A | 50x | ms | DSHB | 3B5 |
| TH | 200x | rb | Abcam | ab112 |
| TPH2 | 2000x | rb | Novus Biologicals | NB100-74555 |
| TUJ1 | 1000x | ms | Abcam | ab18207 |
| VGLUT2 | 200x | rb | Abcam | ab18105 |
| ZIC1 | 30x | ms | DSHB | PCRP-ZIC1-1E3 |
| | | | | |
| **Mouse** | | | | |
| **Antigen** | **Dilution** | **Host species** | **company** | **clone/ Product-ID** |
| GABA | 1000x | rb | Sigma-Aldrich | A-2052 |
| GFAP | 1000x | rb | DAKO, Germany | Z0334 |
| MBP | 25x | rt | Abcam | ab7349 |
| MSX1 | 30x | ms | DSHB | 4G1 |
| Nestin | 100x | ms | Millipore | rat-401 |
| O4 | 100x | ms | R&D Systems | MAB1326 |
| Olig2 | 200x | rb | Millipore | AB9610 |
| PAX3 | 100x | ms | DSHB | Pax3 (concentrate) |
| Peripherin | 1000x | rb | Abcam | ab4666 |
| PLZF | 25x | ms | Calbiochem | OP128 |
| Serotonin | 500x | rb | Sigma-Aldrich | S5545 |
| SMA | 1000x | ms | Dako | M0851 |
| Sox1 | 200x | gt | R&D Systems | AF3369 |
| Sox10 | 200x | ms | Santa Cruz, USA | sc-17342 |
| Sox2 | 100x | ms | R&D Systems | MAB2018 |
| TFAP2A | 50x | ms | DSHB | 3B5 |
| TH | 200x | rb | Abcam | ab112 |
| TUBB3 | 1000x | rb | Covance | PRB-435P |
| TUBB3 | 1000x | ms | Covance | MMS-435P |
| ZO1 | 100x | ms | Thermo Fisher Scientific | 61-7300 |

**Secondaries**

| Antigen/ color | Dilution | company | clone/ Product-ID |
|---|---|---|---|
| rb-555 | 1000x | Abcam | ab150075 |
| m s-647 | 1000x | Abcam | ab150115 |

(continued)

| Antigen/ color | Dilution | company | clone/ Product-ID |
|---|---|---|---|
| gt-488 | 1000x | Abcam | ab150129 |
| ms-555 | 1000x | Abcam | ab150106 |
| ms-488 | 1000x | Abcam | ab150117 |
| rb-488 | 1000x | Cell Signaling | 4412S |
| rb-647 | 1000x | Abcam | ab150075 |
| rt-647 | 1000x | Abcam | ab150155 |
| SAV-555 | 30x | eBioscience | 12431787 |
| ck-488 | 1000x | Abcam | ab150169 |
| SAV-488 | 30x | eBioscience | 11431787 |

**Western Blot**

| Antigen/ color | Dilution | company | clone/ Product-ID |
|---|---|---|---|
| Actin-qt | 10000x | Santa Cruz, USA | **sc-1616** |
| SCN9A-ms | 2000x | abcam | ab85015 |
| HRP-ms | 10000x | DAKO, Germany | P0447 |
| HRP-gt | 10000x | Pierce, Thermo Scientific | Catalog: 31402 |

**Extended Data Table 2**

| Sequence-fwd | Sequence-rev | Source |
|---|---|---|
| CTGGAGAAGGAGGTGGTGAG | GAGAAGGACGGGAGCAGAG | This study |
| CCTGTCGCACATGGGTAGT | GATTCAATCCCATGCCTGCA | This study |
| GCCTCCCAGTGGTATTTGAA | AGCAGGTAACCGGAACCTTT | Kim, Y. J. et al. (2014). Cell Stem Cell, 15(4), 497-506 |
| ACCTCGAAGTACAAGGTCACG | GATCTTCCTCCATTTTTAGACTTCG | Reinhardt, P. et al. (2013). PLoS ONE, 8(3), e59252 |
| CCAGAAAGGATGCCTCATAAA | TCTGCGCGCCCCTAGTTA | Li, W. et al. (2011). PNAS, 108(20), 8299-8304 |
| AGAGATCACCTCTTGCTTGAGAACG | GGAGCCTGTGCTGTAGCAATCA | Reinhardt, P. et al. (2013). PLoS ONE, 8(3), e59252 |
| ACCCCTGCCTAACCACATC | GCGGCAAAGAATCTTGGAGAC | MGH PrimerBank; Primer-Bank ID: 207029245c1 |
| TGGCGAACCATCTCTGTGGT | CCAACGGTGTCAACCTGCAT | Reinhardt, P. et al. (2013). PLoS ONE, 8(3), e59252 |
| AGGTCCATGTGGAGCTTGAC | GCCATTGCCTCATACTGCGT | MGH PrimerBank; Primer-Bank ID: 334688843c2 |
| GGCTTTGCCACTAGGCAGG | TGACCACTTTGTCTCCTTCTTGA | MGH PrimerBank; Primer-Bank ID: 36054052c1 |

(continued)

| Sequence-fwd | Sequence-rev | Source |
|---|---|---|
| TGCGAGCAGAGAGGGAGTAG | TGAGTTCCATGAAGGCAGGATG | Reinhardt, P. et al. (2013). PLoS ONE, 8(3), e59252 |
| AACCGCTACATCACGGAGCA | GATCTTGGCGCGCTTGTTCT | Reinhardt, P. et al. (2013). PLoS ONE, 8(3), e59252 |
| GCAGAGCAGCAGGAAGCTGA | TTCTGCCGAGGAGGCTAAGTG | Reinhardt, P. et al. (2013). PLoS ONE, 8(3), e59252 |
| TGCTTCAGGAGCAGCAAACAA | GATCCAACGCCCTTCCAGAG | Reinhardt, P. et al. (2013). PLoS ONE, 8(3), e59252 |
| AAGCAGCCGGAGAAGACCAC | TCCTTCATGAGCGCATCTGG | Reinhardt, P. et al. (2013). PLoS ONE, 8(3), e59252 |
| GGTGGAGGAAGCTGACAACA | ATCTGCTGCAGTGTGGGTTT | This study |
| CAGCAGTGTCGCTCCAATTCA | GCCAAGCACCGAATTCACAG | Reinhardt, P. et al. (2013). PLoS ONE, 8(3), e59252 |
| CGTTCGGAGCACTATGCTG | TGTTGCACGACTTTTTGGGGT | MGH PrimerBank; Primer-Bank ID: 70778758c2 |
| GGCGCTCAGTTTCCTAACTAC | CTGCCGCATATAACGGAAGAA | MGH PrimerBank; Primer-Bank ID: 194272159c2 |
| AGACGCAGGTGAAGGTGTGG | CAGGCAGGCAGGCTCTCC | Koch, P. et al. (2009). PNAS, 106(9), 3225-3230 |
| TGGGAGATAGGAAAGAGGTGAAAA | GCACCAGGCTGTTGATGCT | Reinhardt, P. et al. (2013). PLoS ONE, 8(3), e59252 |
| CAAAGTGAGACCTGCCAAAAAGA | TGGACAAGGGATCTGACAGTG | MGH PrimerBank; Primer-Bank ID: 27436932c1 |
| CCGCCTTCAGCATAGACTCG | GGTAGCCGGTGTAGACGAAAT | Reinhardt, P. et al. (2013). PLoS ONE, 8(3), e59252 |
| CCTGGGACCCATTTCGGAC | TGGAGCCAGACTCAGGACC | MGH PrimerBank; Primer-Bank ID: 226371734c3 |
| CTCTGCTTCCAAAGGTGTCC | CAGGTCCTGGCCAACAAG | Li, W. et al. (2011). PNAS, 108(20), 8299-8304 |
| TCAAGTCGAGTCTATCTGCATCC | CATGTCACGACCAGTCACAAC | MGH PrimerBank; Primer-Bank ID: 152963638c3 |
| GCTCGCTGAGTGCCTGACAT | GGAGGAGGAATCAGTGTCGAGTG | Reinhardt, P. et al. (2013). PLoS ONE, 8(3), e59252 |
| TTTAGCCGTTCGCTTAGAGG | CGGATAGCTGGAGACAGGAG | Reinhardt, P. et al. (2013). PLoS ONE, 8(3), e59252 |
| GCCTCGGAGAACGAGGAAGA | CGCTGCTGGACTTGTGCTTC | Reinhardt, P. et al. (2013). PLoS ONE, 8(3), e59252 |
| GACAACTGGTGGCAGATTTCGCTT | AGCCACAAAGAAAGGAGTTGGACC | Wang, J. et al. (2014). Proc Natl Acad Sci USA. 111(28):E2885-94. |
| AGCAGGGAGTCCGTAAACG | AGCATTCCGAAACAGGTAACTTT | MGH PrimerBank; Primer-Bank ID: 262359915c1 |
| GACTAGAGTCTGAGATGCCC | AGACATTGTTAAATTGCCCG | Lu, H. et al (2013) Dev Cell, 27(5):560-73. |
| AGCGAACGCACATCAAGAC | CTGTAGGCGATCTGTTGGGG | MGH PrimerBank; Primer-Bank ID: 182765453c1 |

(continued)

| Sequence-fwd | Sequence-rev | Source |
|---|---|---|
| CTGGGCTACACTGAGCACC | AAGTGGTCGTTGAGGGCAATG | MGH PrimerBank; Primer-Bank ID: 378404907c3 |
| CGAGAGGACCCCGTGGATGCAGAG | GGCGGCCATCTTCAGCTTCTCCAG | Reinhardt, P. et al. (2013). PLoS ONE, 8(3), e59252 |
| | | |
| | | |

| Sequence-fwd | Sequence-rev | Source |
|---|---|---|
| TTT CAG CTA GCA TCC TGT ACT CC | GAC CCT GGT CTT GTC GTT AGA | This study |
| CCCATTCTCGGCCTTGACTGT | GTGGAGATTGTTGCCATCAACGA | Scognamiglio, R et al. (2016). Cell, 164(4), 668-680. |
| CGGAGGACAAAAGACAAAAACC | AAGTTACAGAGCCGGCAGTCA | Iwafuchi-Doi, M. et al. (2012). Development, 139(24), 4675-4675 |
| ATGGGCATTGGTATGTTATAATGAAG | AACACAGATCCGCGATCCA | Iwafuchi-Doi, M. et al. (2012). Development, 139(24), 4675-4675 |
| TGCTGCTATGACTTCTTTGCC | GCTTCCTGTGATCGGCCAT | MGH Primerbank ID: 6754754a1 |
| CCGGGGCAGAATTACCCAC | GCCGTTGATAAATACTCCTCCG | MGH PrimerBank; Primer-Bank ID: 226958471c1 |
| TCCCCCGCCAAACTTCA | GTACCACCCATCCCTTCGAA | Iwafuchi-Doi, M. et al. (2012). Development, 139(24), 4675-4675 |
| TGCAGGATCCCTACGCCAAC | CCTGATGCTGGAGCCTGTTCTT | This study |
| TCTGGGTCCCTATCCAATGTG | GGTCCCCGAACTGGTACTG | This study |
| CCGAGGAGGGATCTAAGGAAC | CTTCCAAAAGTATCGGTCTCCAC | MGH PrimerBank; Primer-Bank ID: 22094093a1 |
| CAGCGACCACCTTCCCATAC | CGCAGTGTTTGTCCTTGTGTCT | Iwafuchi-Doi, M. et al. (2012). Development, 139(24), 4675-4675 |
| TGCCTCGGCCACAAAGAATC | GTTGGTGTACGCGGTTCTCA | This study |
| ACCAAGAAACCCAGCCAATCCG | GCAATCCGGGGCCATCTGA | This study |
| | | |
| | | |

| Sequence-fwd | Sequence-rev | Source |
|---|---|---|
| ATGGGGGAATCTTTAATGCTGGT | AGCACTCATGAAATGGGACACT | This study |

## REFERENCES

1. Conti, L. et al. Niche-independent symmetrical self-renewal of a mammalian tissue stem cell. Plos Biol 3, e283 (2005).
2. Elkabetz, Y. & Studer, L. Human ESC-derived neural rosettes and neural stem cell progression. Cold Spring Harb Symp Quant Biol 73, 377-387 (2007).

3. Koch, P., Opitz, T., Steinbeck, J. A., Ladewig, J. & Brüstle, O. A rosette-type, self-renewing human ES cell-derived neural stem cell with potential for in vitro instruction and synaptic integration. Proc Natl Acad Sci USA 106, 3225-3230 (2009).

4. Li, W. et al. Rapid induction and long-term self-renewal of primitive neural precursors from human embryonic stem cells by small molecule inhibitors. Proc Natl Acad Sci USA 108, 8299-8304 (2011).

5. Tailor, J. et al. Stem cells expanded from the human embryonic hindbrain stably retain regional specification and high neurogenic potency. J. Neurosci. 33, 12407-12422 (2013).

6. Reinhardt, P. et al. Derivation and expansion using only small molecules of human neural progenitors for neurodegenerative disease modeling. PLoS ONE 8, e59252 (2013).

7. Gorris, R. et al. Pluripotent stem cell-derived radial glia-like cells as stable intermediate for efficient generation of human oligodendrocytes. Glia 63, 2152-2167 (2015).

8. Efe, J. A. et al. Conversion of mouse fibroblasts into cardiomyocytes using a direct reprogramming strategy. Nat Cell Biol 13, 215-222 (2011).

9. Kim, J. et al. Direct reprogramming of mouse fibroblasts to neural progenitors. Proc Natl Acad Sci USA 108, 7838-7843 (2011).

10. Margariti, A. et al. Direct reprogramming of fibroblasts into endothelial cells capable of angiogenesis and reendothelialization in tissue-engineered vessels. Proc Natl Acad Sci USA 109, 13793-13798 (2012).

11. Thier, M. et al. Direct conversion of fibroblasts into stably expandable neural stem cells. Cell Stem Cell 10, 473-479 (2012).

12. Bung, R. et al. Partial Dedifferentiation of Murine Radial Glia-Type Neural Stem Cells by Brn2 and c-Myc Yields Early Neuroepithelial Progenitors. J. Mol. Biol. 428, 1476-1483 (2016).

13. Artinger, K. B., Fraser, S. & Bronner-Fraser, M. Dorsal and Ventral Cell Types Can Arise from Common Neural Tube Progenitors. Developmental Biology 172, 11-11 (1995).

14. Garnett, A. T. A., Square, T. A. T. & Medeiros, D. M. D. BMP, Wnt and FGF signals are integrated through evolutionarily conserved enhancers to achieve robust expression of Pax3 and Zic genes at the zebrafish neural plate border. Development 139, 4220-4231 (2012).

15. Le Dréau, G. & Martí, E. Dorsal-ventral patterning of the neural tube: a tale of three signals. Dev Neurobiol 72, 1471-1481 (2012).

16. Eminli, S. et al. Differentiation stage determines potential of hematopoietic cells for reprogramming into induced pluripotent stem cells. Nat Genet 41, 968-976 (2009).

17. Brambrink, T. et al. Sequential Expression of Pluripotency Markers during Direct Reprogramming of Mouse Somatic Cells. Stem Cell 2, 9-9 (2008).

18. Nori, S. et al. Long-term safety issues of iPSC-based cell therapy in a spinal cord injury model: oncogenic transformation with epithelial-mesenchymal transition. Stem Cell Reports 4, 360-373 (2015).

19. Galat, V. et al. Transgene Reactivation in Induced Pluripotent Stem Cell Derivatives and Reversion to Pluripotency of Induced Pluripotent Stem Cell-Derived Mesenchymal Stem Cells. Stem Cells and Development 25, 1060-1072 (2016).

20. Scognamiglio, R. et al. Myc Depletion Induces a Pluripotent Dormant State Mimicking Diapause. Cell 164, 668-680 (2016).

21. Chen, E. Y. et al. Enrichr: interactive and collaborative HTML5 gene list enrichment analysis tool. BMC Bioinformatics 14, 128 (2013).

22. Johnson, M. B. et al. Single-cell analysis reveals transcriptional heterogeneity of neural progenitors in human cortex. Nat Neurosci 18, 637-646 (2015).

23. Stein, J. L. et al. A quantitative framework to evaluate modeling of cortical development by neural stem cells. Neuron 83, 69-86 (2014).

24. Lu, J. et al. Generation of serotonin neurons from human pluripotent stem cells. Nat Biotechnol 34, 89-94 (2016).

25. Lancaster, M. A. et al. Cerebral organoids model human brain development and microcephaly. Nature (2013). doi:10.1038/nature12517.

26. Meinhardt, A. et al. 3D reconstitution of the patterned neural tube from embryonic stem cells. Stem Cell Reports 3, 987-999 (2014).

27. Qian, X. et al. Brain-Region-Specific Organoids Using Mini-bioreactors for Modeling ZIKV Exposure. Cell 165, 1238-1254 (2016).

28. Jo, J. et al. Midbrain-like Organoids from Human Pluripotent Stem Cells Contain Functional Dopaminergic and Neuromelanin-Producing Neurons. Cell Stem Cell 19, 248-257 (2016).

29. Chambers, S. M. et al. Combined small-molecule inhibition accelerates developmental timing and converts human pluripotent stem cells into nociceptors. Nat Biotechnol 30, 715-720 (2012).

30. Pounds, S. et al. A procedure to statistically evaluate agreement of differential expression for cross-species genomics. Bioinformatics 27, 2098-2103 (2011).

31. Szklarczyk, D. et al. STRING v10: protein-protein interaction networks, integrated over the tree of life. Nucleic Acids Research 43, D447-52 (2015).

32. Lujan, E., Chanda, S., Ahlenius, H., Südhof, T. C. & Wernig, M. Direct conversion of mouse fibroblasts to self-renewing, tripotent neural precursor cells. Proc Natl Acad Sci USA 109, 2527-2532 (2012).

33. Han, D. W. et al. Direct Reprogramming of Fibroblasts into Neural Stem Cells by Defined Factors. Cell Stem Cell 8 (2012). doi:10.1016/j.stem.2012.02.021

34. Cairns, D. M. et al. Expandable and Rapidly Differentiating Human Induced Neural Stem Cell Lines for Multiple Tissue Engineering Applications. Stem Cell Reports 0, 557-570 (2016).

35. Hou, P.-S. et al. Direct Conversion of Human Fibroblasts into Neural Progenitors Using Transcription Factors Enriched in Human ESC-Derived Neural Progenitors. Stem Cell Reports 8, 54-68 (2016).

36. Lee, J. H. et al. Single Transcription Factor Conversion of Human Blood Fate to NPCs with CNS and PNS Developmental Capacity. Cell Reports 11, 1367-1376 (2015).

37. Elkabetz, Y. et al. Human ES cell-derived neural rosettes reveal a functionally distinct early neural stem cell stage. Genes & Development 22, 152-165 (2008).

38. Cox, J. J. et al. An SCN9A channelopathy causes congenital inability to experience pain. Nature 444, 894-898 (2006).

39. Hockemeyer, D. et al. A Drug-Inducible System for Direct Reprogramming of Human Somatic Cells to Pluripotency. Cell Stem Cell 3, 346-353 (2008).

40. Brambrink, T. et al. Sequential Expression of Pluripotency Markers during Direct Reprogramming of Mouse Somatic Cells. Stem Cell 2, 9-9 (2008).

41. Sommer, A. G. et al. Generation of human induced pluripotent stem cells from peripheral blood using the STEMCCA lentiviral vector. J Vis Exp e4327-e4327 (2012). doi:10.3791/4327.

42. Scognamiglio, R. et al. Myc Depletion Induces a Pluripotent Dormant State Mimicking Diapause. Cell 164, 668-680 (2016).

43. Meyer, S., Wörsdörfer, P., Günther, K., Thier, M. & Edenhofer, F. Derivation of Adult Human Fibroblasts and their Direct Conversion into Expandable Neural Progenitor Cells. J Vis Exp e52831-e52831 (2015). doi:10.3791/52831.

44. Chambers, S. M. et al. Combined small-molecule inhibition accelerates developmental timing and converts human pluripotent stem cells into nociceptors. Nat Biotechnol 30, 715-720 (2012).

45. Schindelin, J. et al. Fiji: an open-source platform for biological-image analysis. Nat Meth 9, 676-682 (2012).

46. Ran, F. A. et al. Genome engineering using the CRISPR-Cas9 system. Nat Protoc 8, 2281-2308 (2013).

47. Dehairs, J., Talebi, A., Cherifi, Y. & Swinnen, J. V. CRISP-ID: decoding CRISPR mediated indels by Sanger sequencing. Sci Rep 6, 28973 (2016).

48. Szklarczyk, D. et al. STRING v10: protein-protein interaction networks, integrated over the tree of life. Nucleic Acids Research 43, D447-52 (2015).

49. Chen, E. Y. et al. Enrichr: interactive and collaborative HTML5 gene list enrichment analysis tool. BMC Bioinformatics 14, 128 (2013).

50. Subramanian, A., Kuehn, H., Gould, J., Tamayo, P. & Mesirov, J. P. GSEA-P: a desktop application for Gene Set Enrichment Analysis. Bioinformatics 23, 3251-3253 (2007).

51. Stein, J. L. et al. A quantitative framework to evaluate modeling of cortical development by neural stem cells. Neuron 83, 69-86 (2014).

52. Pounds, S. et al. A procedure to statistically evaluate agreement of differential expression for cross-species genomics. Bioinformatics 27, 2098-2103 (2011).

53. Aryee, M. J. et al. Minfi: a flexible and comprehensive Bioconductor package for the analysis of Infinium DNA methylation microarrays. Bioinformatics 30, 1363-1369 (2014).

54. Assenov, Y. et al. Comprehensive analysis of DNA methylation data with RnBeads. Nat Meth 11, 1138-1140 (2014).

## SEQUENCE LISTING

Full sequence of vector pHAGE2-TetOminiCV-BRN22AKlf4-IRES-Sox2E2AZic3-W (SEQ ID NO:1):

tggaagggctaattcactcccaaagaagacaagatatccttgatctgtggatctaccacacacaaggctacttccctga
ttagcagaactacacaccagggccaggggtcagatatccactgacccttggatggtgctacaagctagtaccagttga
gccagataaggtagaagaggccaataaaggagagaacaccagcttgttacaccctgtgagcctgcatgggatgga
tgacccggagagagaagtgttagagtggaggtttgacagccgcctagcatttcatcacgtggcccgagagctgcatc
cggagtacttcaagaactgctgatatcgagcttgctacaagggactttccgctggggactttccagggaggcgtggcct
gggcgggactggggagtggcgagccctcagatcctgcatataagcagctgcttttgcctgtactgggtctctctggtta
gaccagatctgagcctgggagctctctggctaactagggaacccactgcttaagcctcaataaagcttgccttgagtgc
ttcaagtagtgtgtgcccgtctgttgtgtgactctggtaactagagatccctcagaccctttagtcagtgtggaaaatctct
agcagtggcgcccgaacagggacttgaaagcgaaagggaaaccagaggagctctctcgacgcaggactcggctt
gctgaagcgcgcacggcaagaggcgaggggcggcgactggtgagtacgccaaaaattttgactagcggaggcta
gaaggagagagatgggtgcgagagcgtcagtattaagcggggggagaattagatcgcgatgggaaaaaattcggtt
aaggccaggggggaaagaaaaaatataaattaaaacatatagtatgggcaagcagggagctagaacgattcgcag
ttaatcctggcctgttagaaacatcagaaggctgtagacaaatactgggacagctacaaccatcccttcagacaggat
cagaagaacttagatcattatataatacagtagcaaccctctattgtgtgcatcaaaggatagagataaaagacacca
aggaagctttagacaagatagaggaagagcaaaacaaaagtaagaccaccgcacagcaagcggccggccgct
gatcttcagacctggaggaggagatatgagggacaattggagaagtgaattatataaatataaagtagtaaaaattg
aaccattaggagtagcacccaccaaggcaaagagaagagtggtgcagagagaaaaaagagcagtgggaatag
gagctttgttccttgggttcttgggagcagcaggaagcactatgggcgcagcgtcaatgacgctgacggtacaggcca
gacaattattgtctggtatagtgcagcagcagaacaatttgctgagggctattgaggcgcaacagcatctgttgcaactc
acagtctggggcatcaagcagctccaggcaagaatcctggctgtggaaagatacctaaaggatcaacagctcctgg
ggatttggggttgctctggaaaactcatttgcaccactgctgtgccttggaatgctagttggagtaataaatctctggaac
agatttggaatcacacgacctggatggagtgggacagagaaattaacaattacacaagcttaatacactccttaattg
aagaatcgcaaaaccagcaagaaaagaatgaacaagaattattggaattagataaatgggcaagtttgtggaattg
gtttaacataacaaattggctgtggtatataaaattattcataatgatagtaggaggcttggtaggtttaagaatagttttgc
tgtactttctatagtgaatagagttaggcagggatattcaccattatcgtttcagacccacctcccaaccccgaggggac
ccgacaggcccgaaggaatagaagaagaaggtggagagagagacagagacagatccattcgattagtgaacgg
atctcgacggtatcgccgaattcacaaatggcagtattcatccacaattttaaaagaaaaggggggattggggggtac
agtgcaggggaaagaatagtagacataatagcaacagacatacaaactaaagaattacaaaaacaaattacaaa
aattcaaaattttcgggtttattacagggacagcagagatccagtttggactagtccacaccctaactgacacactcga
gtttaccactccctatcagtgatagagaaaagtgaaagtcgagtttaccactccctatcagtgatagagaaaagtgaa
agtcgagtttaccactccctatcagtgatagagaaaagtgaaagtcgagtttaccactccctatcagtgatagagaaaa
gtgaaagtcgagtttaccactccctatcagtgatagagaaaagtgaaagtcgagtttaccactccctatcagtgataga
gaaaagtgaaagtcgagtttaccactccctatcagtgatagagaaaagtgaaagtcgagctcggtacccgggtcga
ggtaggcgtgtacggtgggaggcctataagcagagctcgtttagtgaaccgtcagatcgcctggagacgccatcc
acgctgttttgacctccatagaagacaccgggaccgatccagcctgcggccgccATGGCGACCGCAGCG
TCTAACCACTACAGCCTGCTCACCTCCAGCGCCTCCATCGTGCACGCCGAGCC
GCCCGGCGGCATGCAGCAGGGCGCGGGGGGCTACCGCGAAGCGCAGAGCCT
GGTGCAGGGCGACTACGGCGCTCTGCAGAGCAACGGACACCCGCTCAGCCAC
GCTCACCAGTGGATCACCGCGCTGTCCCACGGCGGCGGCGGCGGGGGCGGT
GGCGGCGGCGGGGGGGCGGGGGCGGCGGCGGGGGCGGCGGCGACGGCT
CCCCGTGGTCCACCAGCCCCCTGGGCCAGCCGGACATCAAGCCCTCGGTGGT
GGTGCAGCAGGGCGGCCGCGGAGACGAGCTGCACGGGCCAGGCGCCCTGCA
GCAGCAGCATCAGCAGCAGCAACAGCAACAGCAGCAGCAACAGCAGCAACAGC
AGCAGCAGCAGCAGCAACAGCGGCCGCCGCATCTGGTGCACCACGCCGCTAA
CCACCACCCGGGACCCGGGGCATGGCGGAGCGCGGCGGCTGCAGCGCACCT

CCCACCCTCCATGGGAGCGTCCAACGGCGGCTTGCTCTACTCGCAGCCCAGCT
TCACGGTGAACGGCATGCTGGGCGCCGGCGGGCAGCCGGCCGGTCTGCACCA
CCACGGCCTGCGGGACGCGCACGACGAGCCACACCATGCCGACCACCACCCG
CACCCGCACTCGCACCCACACCAGCAGCCGCCGCCCCGCCGCCCCGCAGG
GTCCGCCTGGCCACCCAGGCGCGCACCACGACCCGCACTCGGACGAGGACAC
GCCGACCTCGGACGACCTGGAGCAGTTCGCCAAGCAGTTCAAGCAGCGGCGG
ATCAAACTGGGATTTACCCAAGCGGACGTGGGGCTGGCTCTGGGCACCCTGTA
TGGCAACGTGTTCTCGCAGACCACCATCTGCAGGTTTGAGGCCCTGCAGCTGA
GCTTCAAGAACATGTGCAAGCTGAAGCCTTTGTTGAACAAGTGGTTGGAGGAGG
CGGACTCGTCCTCGGGCAGCCCCACGAGCATAGACAAGATCGCAGCGCAAGG
GCGCAAGCGGAAAAAGCGGACCTCCATCGAGGTGAGCGTCAAGGGGGCTCTG
GAGAGCCATTTCCTCAAATGCCCCAAGCCCTCGGCCCAGGAGATCACCTCCCT
CGCGGACAGCTTACAGCTGGAGAAGGAGGTGGTGAGAGTTTGGTTTTGTAACA
GGAGACAGAAAGAGAAAGGATGACCCCTCCCGGAGGGACTCTGCCGGGCGC
CGAGGATGTGTACGGGGGGAGTAGGGACACTCCACCACACCACGGGGTGCAG
ACGCCCGTCCAGggaagtggcgtgaaacagactttgaattttgaccttctcaagttggcgggagacgtggagt
ccaacccagggcccatgGCTGTCAGCGACGCTCTGCTCCgtccttctccacgttcgcgtccggcccg
gcgggaagggagaagacactgcgtccagcaggtgccccgactaaccgttggcgtgaggaactctctcacatgaag
cgacttcccccacttcccggccgcccctacgacctggcggcgacggtggccacagacctggagagtggcggagct
ggtgcagcttgcagcagtaacaacccggccctcctagcccggagggagaccgaggagttcaacgacctcctggac
ctagactttatcctttccaactcgctaacccaccaggaatcggtggccgccaccgtgaccacctcggcgtcagcttcatc
ctcgtcttccccagcgagcagcggccctgccagcgcgccctccacctgcagcttcagctatccgatccgggccgggg
gtgacccgggcgtggctgccagcaacacaggtggagggctcctctacagccgagaatctgcgccacctcccacgg
ccccccttcaacctggcggacatcaatgacgtgagccctcgggcggcttcgtggctgagctcctgcggccggagttgg
acccagtatacattccgccacagcagcctcagccgccaggtggcgggctgatgggcaagtttgtgctgaaggcgtct
ctgaccaccctggcagcgagtacagcagcccttcggtcatcagtgttagcaaaggaagcccagacggcagccac
cccgtggtagtggcgccctacagcggtggcccgccgcgcatgtgcccaagattaagcaagaggcggtcccgtcct
gcacggtcagccggtccctagaggcccatttgagcgctggaccccagctcagcaacggccaccggcccaacacac
acgacttcccctggggcggcagctccccaccaggactacccctacactgagtcccgaggaactgctgaacagca
gggactgtcaccctggcctgcctcttcccccaggattccatccccatccggggcccaactaccctcctttcctgccagac
cagatgcagtcacaagtcccctctctccattatcaagagctcatgccaccgggttcctgcctgccagaggagcccaag
ccaaagaggggaagaaggtcgtggcccccggaaaagaacagccacccacacttgtgactatgcaggctgtggcaa
aacctataccaagagttctcatctcaaggcacacctgcgaactcacacaggcgagaaaccttaccactgtgactggg
acggctgtgggtggaaattcgcccgctccgatgaactgaccaggcactaccgcaaacacacagggcaccggccctt
tcagtgccagaagtgtgacagggcctttccaggtcggaccaccttgccttacacatgaagaggcacttttaaagatcc
ctccccccccccctaacgttactggccgaagccgcttggaataaggccggtgtgcgtttgtctatatgttattttccaccatat
tgccgtcttttggcaatgtgagggcccggaaacctggccctgtcttcttgacgagcattcctaggggtctttcccctctcgc
caaaggaatgcaaggtctgttgaatgtcgtgaaggaagcagttcctctggaagcttcttgaagacaaacaacgtctgt
agcgaccctttgcaggcagcggaacccccacctggcgacaggtgcctctgcggccaaaagccacgtgtataagat
acacctgcaaaggcggcacaaccccagtgccacgttgtgagttggatagttgtggaaagagtcaaatggctctcctc
aagcgtattcaacaaggggctgaaggatgcccagaaggtaccccattgtatgggatctgatctggggcctcggtgca
catgctttacatgtgtttagtcgaggttaaaaaaacgtctaggcccccccgaaccacggggacgtggttttcctttgaaaa
acacgatgataatatggccacacatatgatgtataacatgatggagacggagctgaagccgccgggcccgcagca
agcttcggggggcggcggcggaggaggcaacgccacggcggcggcgaccggcggcaaccagaagaacagcc
cggaccgcgtcaagaggcccatgaacgccttcatggtatggtcccggggggcagcggcgtaagatggcccaggaga
accccaagatgcacaactcggagatcagcaagcgcctgggcgcggagtggaaacttttgtccgagaccgagaagc
ggccgttcatcgacgaggccaagcggctgcgcgctctgcacatgaaggagcacccggattataaataccggccgc
ggcggaaaaccaagacgctcatgaagaaggataagtacacgcttcccggaggcttgctggcccccggcgggaac
agcatggcgagcggggttggggtgggcgccggcctgggtgcgggcgtgaaccagcgcatggacagctacgcgca
catgaacggctggagcaacggcagctacagcatgatgcaggagcagctgggctacccgcagcacccgggcctca
acgctcacggcgcggcacagatgcaaccgatgcaccgctacgacgtcagcgccctgcagtacaactccatgacca

gctcgcagacctacatgaacggctcgcccacctacagcatgtcctactcgcagcagggcacccccggtatggcgct
gggctccatgggctctgtggtcaagtccgaggccagctccagcccccccgtggttacctcttcctcccactccagggcg
ccctgccaggccggggacctccgggacatgatcagcatgtacctccccggcgccgaggtgccggagcccgctgcg
cccagtagactgcacatggcccagcactaccagagcggcccggtgcccggcacggccattaacggcacactgccc
ctgtcgcacatgggtagtgggcaatgtactaactacgctttgttgaaactcgctggcgatgttgaaagtaaccccggtcc
tatgacgatgctcctggacggaggcccgcagttccctgggttggggagtgggcagcttcggtgctccgcgccaccacg
agatgcccaaccgcgagcctgcaggcatgggattgaatcccttcggggactcaacccacgctgcggccgccgccg
ctgccgccgctgccttcaagctgagcccagccaccgctcacgatctgtcttcgggccagagctcagcgttcacaccgc
agggttcaggttatgccaatgccctgggccaccatcaccaccaccatcaccaccatcacgccagccaggtgcccac
ctacggcggcgctgcctccgccgctttcaactccacgcgcgactttctgttccgtcagcgcggttctgggctcagcgag
gcagcctccggggggcgggcagcacgggcttttcgctggctcggcgagcagtcttcacgctccagctggtattcctgag
cctcctagctacttgctctttcctgggcttcatgagcagggcgctgggcacccgtcgcccacagggcacgtggacaac
aaccaggtccatctggggctgcgcggggagctatttggccgtgcagacccataccgccccgtggctagcccgcgca
cggacccctacgcggccagtgcgcagttccctaactatagccccatgaacatgaacatgggcgtgaacgtggcggc
ccaccacgggccgggcgccttcttccgttacatgcggcagcccatcaagcaggagctgtcctgtaagtggatcgagg
aggctcagctgagccggcccaagaagagctgcgaccggaccttcagcaccatgcatgagttggttacgcatgttacc
atggagcatgtggggggcccggagcagaacaaccacgtctgctattgggaggaatgtccccgcgaaggcaagtcc
ttcaaggcgaagtacaaactggtcaaccatatccgagtgcacactggcgagaaacccttcccgtgtcccttcccgggc
tgcgggaagatttttgcccgctctgagaacctcaagatccacaagaggacccatacaggtgagaaacctttcaaatgt
gaattcgaaggctgtgacagacggtttgccaacagcagcgaccgcaagaagcacatgcatgtgcacacctcggac
aagccctatatctgtaaagtgtgcgacaagtcctacacacacccgagctccctgcgcaaacacatgaaggttcatga
atctcaagggtcagattcctcccctgctgccagttcaggctatgaatcttccactccacccgctatagcttctgcaaacag
taaagataccactaaaacccttctgcagttcaaactagcaccagccacaaccctggacttcctcccaattttaacgaa
tggtacgtctgaatcgatagatcctaatcaacctctggattacaaaatttgtgaaagattgactggtattcttaactatgttg
ctcctttacgctatgtggatacgctgctttaatgcctttgtatcatgctattgcttcccgtatggctttcattttctcctccttgtata
aatcctggttgctgtctctttatgaggagttgtggcccgttgtcaggcaacgtggcgtggtgtgcactgtgtttgctgacgca
acccccactggttggggcattgccaccacctgtcagctcctttccgggactttcgctttccccctccctattgccacggcg
gaactcatcgccgcctgccttgcccgctgctggacaggggctcggctgttgggcactgacaattccgtggtgttgtcgg
ggaaatcatcgtcctttccttggctgctcgcctgtgttgccacctggattctgcgcgggacgtccttctgctacgtcccttcg
gccctcaatccagcggaccttccttcccgcggcctgctgccggctctgcggcctcttccgcgtcttcgccttcgccctcag
acgagtcggatctccctttgggccgcctccccgcctggtacctttaagaccaatgacttacaaggcagctgtagatctta
gccactttttaaaagaaaaggggggactggaagggctaattcactcccaacgaagacaagatcacctgcaggaca
ggcgcgccctgctttttgcttgtactgggtctctctggttagaccagatctgagcctgggagctctctggctaactagggaa
cccactgcttaagcctcaataaagcttgccttgagtgcttcaagtagtgtgtgcccgtctgttgtgtgactctggtaactag
agatccctcagacccttttagtcagtgtggaaaatctctagcacccgggcgattaaggaaagggctagatcattcttga
agacgaaagggcctcgtgatacgcctattttttataggttaatgtcatgataataatggtttcttagacgtcaggtggcacttt
tcggggaaatgtgcgcggaacccctatttgtttattttctaaatacattcaaatatgtatccgctcatgagacaataaccct
gataaatgcttcaataatattgaaaaaggaagagtatgagtattcaacatttccgtgtcgcccttattcccttttttgcggca
ttttgccttcctgttttttgctcacccagaaacgctggtgaaagtaaaagatgctgaagatcagttgggtgcacgagtgggt
tacatcgaactggatctcaacagcggtaagatccttgagagttttcgccccgaagaacgttttccaatgatgagcactttt
aaagttctgctatgtggcgcggtattatcccgtgttgacgccgggcaagagcaactcggtcgccgcatacactattctca
gaatgacttggttgagtactcaccagtcacagaaaagcatcttacggatggcatgacagtaagagaattatgcagtgc
tgccataaccatgagtgataacactgcggccaacttacttctgacaacgatcggaggaccgaaggagctaaccgcttt
tttgcacaacatgggggatcatgtaactcgccttgatcgttgggaaccggagctgaatgaagccataccaaacgacg
agcgtgacaccacgatgcctgtagcaatggcaacaacgttgcgcaaactattaactggcgaactacttactctagcttc
ccggcaacaattaatagactggatggaggcggataaagttgcaggaccacttctgcgctcggcccttccggctggctg
gtttattgctgataaatctggagccggtgagcgtgggtctcgcggtatcattgcagcactggggccagatggtaagccct
cccgtatcgtagttatctacacgacggggagtcaggcaactatggatgaacgaaatagacagatcgctgagataggt
gcctcactgattaagcattggtaactgtcagaccaagtttactcatatatactttagattgatttaaaacttcattttaatttaa
aaggatctaggtgaagatcctttttgataatctcatgaccaaaatcccttaacgtgagttttcgttccactgagcgtcagac
cccgtagaaaagatcaaaggatcttcttgagatccttttttttctgcgcgtaatctgctgcttgcaaacaaaaaaaccacc

gctaccagcggtggtttgtttgccggatcaagagctaccaactctttttccgaaggtaactggcttcagcagagcgcag
ataccaaatactgttcttctagtgtagccgtagttaggccaccacttcaagaactctgtagcaccgcctacatacctcgct
ctgctaatcctgttaccagtggctgctgccagtggcgataagtcgtgtcttaccgggttggactcaagacgatagttacc
ggataaggcgcagcggtcgggctgaacggggggttcgtgcacacagcccagcttggagcgaacgacctacaccg
aactgagatacctacagcgtgagctatgagaaagcgccacgcttccccgaagggagaaaggcggacaggtatccg
gtaagcggcagggtcggaacaggagagcgcacgagggagcttccaggggggaaacgcctggtatctttatagtcctg
tcgggtttcgccacctctgacttgagcgtcgatttttgtgatgctcgtcaggggggcggagcctatggaaaaacgccag
caacgcggcctttttacggttcctggcctttttgctggcctttttgctcacatgttctttcctgcgttatcccctgattctgtggataa
ccgtattaccgcctttgagtgagctgataccgctcgccgcagccgaacgaccgagcgcagcgagtcagtgagcgag
gaagcggaagagcgcccaatacgcaaaccgcctctccccgcgcgttggccgattcattaatgcagcaagctcatgg
ctgactaattttttttatttatgcagaggccgaggccgcctcggcctctgagctattccagaagtagtgaggaggcttttttg
gaggcctaggcttttgcaaaaagctccccgtggcacgacaggtttcccgactggaaagcgggcagtgagcgcaacg
caattaatgtgagttagctcactcattaggcaccccaggctttacacttatgcttccggctcgtatgttgtgtggaattgtga
gcggataacaatttcacacaggaaacagctatgacatgattacgaatttcacaaataaagcattttttttcactgcattcta
gttgtggtttgtccaaactcatcaatgtatcttatcatgtctggatcaactggataactcaagctaaccaaaatcatcccaa
acttcccaccccataccctattaccactgccaattacctgtggtttcatttactctaaacctgtgattcctctgaattattttcatt
ttaaagaaattgtatttgttaaatatgtactacaaacttagtagt

Full sequence of vector pHAGE2-TetOminiCV-BRN22AKlf4-IRES-Sox2E2AZic3-W-loxp (SEQ ID NO:2):

tggaagggctaattcactcccaaagaagacaagatatccttgatctgtggatctaccacacacaaggctacttccctga
ttagcagaactacacaccagggccagggggtcagatatccactgacctttggatggtgctacaagctagtaccagttga
gccagataaggtagaagaggccaataaaggagagaacaccagcttgttacaccctgtgagcctgcatgggatgga
tgacccggagagagaagtgttagagtggaggtttgacagccgcctagcatttcatcacgtggcccgagagctgcatc
cggagtacttcaagaactgctgatatcgagcttgctacaagggactttccgctggggactttccagggaggcgtggcct
gggcgggactggggagtggcgagccctcagatcctgcatataagcagctgctttttgcctgtactgggtctctctggtta
gaccagatctgagcctgggagctctctggctaactagggaacccactgcttaagcctcaataaagcttgccttgagtgc
ttcaagtagtgtgtgcccgtctgttgtgtgactctggtaactagagatccctcagacccttttagtcagtgtggaaaatctct
agcagtggcgcccgaacagggacttgaaagcgaaagggaaaccagaggagctctctcgacgcaggactcggctt
gctgaagcgcgcacggcaagaggcgaggggcggcgactggtgagtacgccaaaaattttgactagcggaggcta
gaaggagagagatgggtgcgagagcgtcagtattaagcggggggagaattagatcgcgatgggaaaaaattcggtt
aaggccagggggaaagaaaaaatataaattaaaacatatagtatgggcaagcagggagctagaacgattcgcag
ttaatcctggcctgttagaaacatcagaaggctgtagacaaatactgggacagctacaaccatcccttcagacaggat
cagaagaacttagatcattatataatacagtagcaaccctctattgtgtgcatcaaaggatagagataaaagacacca
aggaagctttagacaagatagaggaagagcaaaacaaaagtaagaccaccgcacagcaagcggccggccgct
gatcttcagacctggaggaggagatatgagggacaattggagaagtgaattatataaatataaagtagtaaaaattg
aaccattaggagtagcacccaccaaggcaaagagaagagtggtgcagagagaaaaaagagcagtgggaatag
gagctttgttccttgggttcttgggagcagcaggaagcactatgggcgcagcgtcaatgacgctgacggtacaggcca
gacaattattgtctggtatagtgcagcagcagaacaatttgctgagggctattgaggcgcaacagcatctgttgcaactc
acagtctggggcatcaagcagctccaggcaagaatcctggctgtggaaagatacctaaaggatcaacagctcctgg
ggatttggggttgctctggaaaactcatttgcaccactgctgtgccttggaatgctagttggagtaataaatctctggaac
agatttggaatcacacgacctggatggagtgggacagagaaattaacaattacacaagcttaatacactccttaattg
aagaatcgcaaaaccagcaagaaaagaatgaacaagaattattggaattagataaatgggcaagtttgtggaattg
gtttaacataacaaattggctgtggtatataaaattattcataatgatagtaggaggcttggtaggtttaagaatagttttttgc
tgtactttctatagtgaatagagttaggcagggatattcaccattatcgtttcagacccacctcccaaccccgaggggac
ccgacaggcccgaaggaatagaagaagaaggtggagagagagacagagacagatccattcgattagtgaacgg
atctcgacggtatcgccgaattcacaaatggcagtattcatccacaattttaaaagaaaaggggggattggggggtac
agtgcaggggaaagaatagtagacataatagcaacagacatacaaactaaagaattacaaaaacaaattacaaa
aattcaaaattttcgggtttattacagggacagcagagatccagtttggactagtccacaccctaactgacacactcga
gtttaccactccctatcagtgatagagaaaagtgaaagtcgagtttaccactccctatcagtgatagagaaaagtgaa

agtcgagtttaccactccctatcagtgatagagaaaagtgaaagtcgagtttaccactccctatcagtgatagagaaaa
gtgaaagtcgagtttaccactccctatcagtgatagagaaaagtgaaagtcgagtttaccactccctatcagtgataga
gaaaagtgaaagtcgagtttaccactccctatcagtgatagagaaaagtgaaagtcgagctcggtacccgggtcga
ggtaggcgtgtacggtgggaggcctatataagcagagctcgtttagtgaaccgtcagatcgcctggagacgccatcc
acgctgttttgacctccatagaagacaccgggaccgatccagcctgcggccgccATGGCGACCGCAGCG
TCTAACCACTACAGCCTGCTCACCTCCAGCGCCTCCATCGTGCACGCCGAGCC
GCCCGGCGGCATGCAGCAGGGCGCGGGGGGCTACCGCGAAGCGCAGAGCCT
GGTGCAGGGCGACTACGGCGCTCTGCAGAGCAACGGACACCCGCTCAGCCAC
GCTCACCAGTGGATCACCGCGCTGTCCCACGGCGGCGGCGGCGGGGGCGGT
GGCGGCGGCGGGGGGGGCGGGGGCGGCGGCGGGGGCGGCGGCGACGGCT
CCCCGTGGTCCACCAGCCCCCTGGGCCAGCCGGACATCAAGCCCTCGGTGGT
GGTGCAGCAGGGCGGCCGCGGAGACGAGCTGCACGGGCCAGGCGCCCTGCA
GCAGCAGCATCAGCAGCAGCAACAGCAACAGCAGCAGCAACAGCAGCAACAGC
AGCAGCAGCAGCAGCAACAGCGGCCGCCGCATCTGGTGCACCACGCCGCTAA
CCACCACCCGGGACCCGGGGCATGGCGGAGCGCGGCGGCTGCAGCGCACCT
CCCACCCTCCATGGGAGCGTCCAACGGCGGCTTGCTCTACTCGCAGCCCAGCT
TCACGGTGAACGGCATGCTGGGCGCCGGCGGGCAGCCGGCCGGTCTGCACCA
CCACGGCCTGCGGGACGCGCACGACGAGCCACACCATGCCGACCACCACCCG
CACCCGCACTCGCACCCACACCAGCAGCCGCCGCCCCCGCCGCCCCCGCAGG
GTCCGCCTGGCCACCCAGGCGCGCACCACGACCCGCACTCGGACGAGGACAC
GCCGACCTCGGACGACCTGGAGCAGTTCGCCAAGCAGTTCAAGCAGCGGCGG
ATCAAACTGGGATTTACCCAAGCGGACGTGGGGCTGGCTCTGGGCACCCTGTA
TGGCAACGTGTTCTCGCAGACCACCATCTGCAGGTTTGAGGCCCTGCAGCTGA
GCTTCAAGAACATGTGCAAGCTGAAGCCTTTGTTGAACAAGTGGTTGGAGGAGG
CGGACTCGTCCTCGGGCAGCCCCACGAGCATAGACAAGATCGCAGCGCAAGG
GCGCAAGCGGAAAAAGCGGACCTCCATCGAGGTGAGCGTCAAGGGGGCTCTG
GAGAGCCATTTCCTCAAATGCCCCAAGCCCTCGGCCCAGGAGATCACCTCCCT
CGCGGACAGCTTACAGCTGGAGAAGGAGGTGGTGAGAGTTTGGTTTTGTAACA
GGAGACAGAAAGAGAAAAGGATGACCCCTCCCGGAGGGACTCTGCCGGGCGC
CGAGGATGTGTACGGGGGGAGTAGGGACACTCCACCACACCACGGGGTGCAG
ACGCCCGTCCAGgaagtggcgtgaaacagactttgaattttgaccttctcaagttggcgggagacgtggagt
ccaacccagggccccatgGCTGTCAGCGACGCTCTGCTCCgtccttctccacgttcgcgtccggcccg
gcgggaagggagaagacactgcgtccagcaggtgccccgactaaccgttggcgtgaggaactctctcacatgaag
cgacttcccccacttcccggccgcccctacgacctggcggcgacggtggccacagacctggagagtggcggagct
ggtgcagcttcagcagtaacaacccggccctcctagcccggagggagaccgaggagttcaacgacctcctggac
ctagactttatcctttccaactcgctaacccaccaggaatcggtggccgccaccgtgaccacctcggcgtcagcttcatc
ctcgtcttccccagcgagcagcggccctgccagcgcgccctccacctgcagcttcagctatccgatccgggccgggg
gtgacccgggcgtggctgccagcaacacaggtggagggctcctctacagccgagaatctgcgccacctcccacgg
ccccttcaacctggcggacatcaatgacgtgagccctcgggcggcttcgtggctgagctcctgcggccggagttgg
acccagtatacattccgccacagcagcctcagccgccaggtggcgggctgatgggcaagtttgtgctgaaggcgtct
ctgaccacccctggcagcgagtacagcagcccttcggtcatcagtgttagcaaaggaagcccagacggcagccac
cccgtggtagtggcgccctacagcggtggcccgccgcgcatgtgccccaagattaagcaagaggcggtcccgtcct
gcacggtcagccggtccctagaggcccatttgagcgctggaccccagctcagcaacggccaccggcccaacacac
acgacttccccctggggcggcagctccccaccaggactacccctacactgagtcccgaggaactgctgaacagca
gggactgtcaccctggcctgcctcttcccccaggattccatccccatccggggcccaactaccctcctttcctgccagac
cagatgcagtcacaagtcccctctctccattatcaagagctcatgccaccgggttcctgcctgccagaggagcccaag
ccaaagagggggaagaaggtcgtggccccggaaaagaacagccacccacacttgtgactatgcaggctgtggcaa
aacctataccaagagttctcatctcaaggcacacctgcgaactcacacaggcgagaaaccttaccactgtgactggg
acggctgtgggtggaaattcgcccgctccgatgaactgaccaggcactaccgcaaacacacagggcaccggcccctt
tcagtgccagaagtgtgacagggcctttttccaggtcggaccaccttgccttacacatgaagaggcacttttaaagatcc
ctcccccccccctaacgttactggccgaagccgcttggaataaggccggtgtgcgtttgtctatatgttattttccaccatat

tgccgtcttttggcaatgtgagggcccggaaacctggccctgtcttcttgacgagcattcctaggggtctttcccctctcgc
caaaggaatgcaaggtctgttgaatgtcgtgaaggaagcagttcctctggaagcttcttgaagacaaacaacgtctgt
agcgacccttttgcaggcagcggaacccccacctggcgacaggtgcctctgcggccaaaagccacgtgtataagat
acacctgcaaaggcggcacaaccccagtgccacgttgtgagttggatagttgtggaaagagtcaaatggctctcctc
aagcgtattcaacaaggggctgaaggatgcccagaaggtaccccattgtatgggatctgatctggggcctcggtgca
catgctttacatgtgtttagtcgaggttaaaaaaacgtctaggcccccccgaaccacggggacgtggttttcctttgaaaa
acacgatgataatatggccacacatgatgtataacatgatggagacggagctgaagccgccgggcccgcagca
agcttcggggggcggcggcggaggaggcaacgccacggcggcggcgaccggcggcaaccagaagaacagcc
cggaccgcgtcaagaggcccatgaacgccttcatggtatggtcccggggggcagcggcgtaagatggcccaggaga
accccaagatgcacaactcggagatcagcaagcgcctgggcgcggagtggaaactttgtccgagaccgagaagc
ggccgttcatcgacgaggccaagcggctgcgcgctctgcacatgaaggagcacccggattataaataccggccgc
ggcggaaaaccaagacgctcatgaagaaggataagtacacgcttcccggaggcttgctggcccccggcgggaac
agcatggcgagcggggttggggtgggcgccggcctgggtgcgggcgtgaaccagcgcatggacagctacgcgca
catgaacggctggagcaacggcagctacagcatgatgcaggagcagctgggctacccgcagcacccgggcctca
acgctcacggcgcggcacagatgcaaccgatgcaccgctacgacgtcagcgccctgcagtacaactccatgacca
gctcgcagacctacatgaacggctcgcccacctacagcatgtcctactcgcagcagggcacccccggtatggcgct
gggctccatgggctctgtggtcaagtccgaggccagctccagccccccccgtggttacctcttcctcccactccagggcg
ccctgccaggccgggggacctccgggacatgatcagcatgtacctccccggcgccgaggtgccggagcccgctgcg
cccagtagactgcacatggcccagcactaccagagcggcccggtgcccggcacggccattaacggcacactgccc
ctgtcgcacatgggtagtgggcaatgtactaactacgctttgttgaaactcgctggcgatgttgaaagtaaccccggtcc
tatgacgatgctcctggacggaggcccgcagttccctgggttgggagtgggcagcttcggtgctccgcgccaccacg
agatgcccaaccgcgagcctgcaggcatgggattgaatcccttcggggactcaacccacgctgcggccgccgccg
ctgccgccgctgccttcaagctgagcccagccaccgctcacgatctgtcttcgggccagagctcagcgttcacaccgc
agggttcaggttatgccaatgccctgggccaccatcaccaccaccatcaccaccatcacgccagccaggtgcccac
ctacggcggcgctgcctccgccgctttcaactccacgcgcgactttctgttccgtcagcgcgcggttctgggctcagcgag
gcagcctccggggggcgggcagcacgggctttcgctggctcggcgagcagtcttcacgctccagctggtattcctgag
cctcctagctacttgctctttcctgggcttcatgagcagggcgctgggcacccgtcgcccacagggcacgtggacaac
aaccaggtccatctggggctgcgcggggagctatttggccgtgcagacccataccgccccgtggctagcccgcgca
cggacccctacgcggccagtgcgcagttccctaactatagccccatgaacatgaacatgggcgtgaacgtggcggc
ccaccacgggccgggcgccttcttccgttacatgcggcagcccatcaagcaggagctgtcctgtaagtggatcgagg
aggctcagctgagccggcccaagaagagctgcgaccggaccttcagcaccatgcatgagttggttacgcatgttacc
atggagcatgtggggggggcccggagcagaacaaccacgtctgctattgggaggaatgtccccgcgaaggcaagtcc
ttcaaggcgaagtacaaactggtcaaccatatccgagtgcacactggcgagaaacccttcccgtgtcccttcccgggc
tgcgggaagattttgcccgctctgagaacctcaagatccacaagaggacccatacaggtgagaaacctttcaaatgt
gaattcgaaggctgtgacagacggtttgccaacagcagcgaccgcaagaagcacatgcatgtgcacacctcggac
aagcccctatatctgtaaagtgtgcgacaagtcctacacacacccgagctccctgcgcaaacacatgaaggttcatga
atctcaagggtcagattcctcccctgctgccagttcaggctatgaatcttccactccacccgctatagcttctgcaaacag
taaagataccactaaaacccttctgcagttcaaactagcaccagccacaaccctggacttcctcccaattttaacgaa
tggtacgtctgaatcgatagatcctaatcaacctctggattacaaaatttgtgaaagattgactggtattcttaactatgttg
ctccttttacgctatgtggatacgctgctttaatgccttttgtatcatgctattgcttcccgtatggctttcattttctcctccttgtata
aatcctggttgctgtctctttatgaggagttgtggcccgttgtcaggcaacgtggcgtggtgtgcactgtgtttgctgacgca
acccccactggttggggcattgccaccacctgtcagctcctttccgggactttcgctttccccctccctattgccacggcg
gaactcatcgccgcctgccttgcccgctgctggacaggggctcggctgttgggcactgacaattccgtggtgttgtcgg
ggaaatcatcgtcctttccttggctgctcgcctgtgttgccacctggattctgcgcgggacgtccttctgctacgtcccttcg
gccctcaatccagcggaccttccttcccgcggcctgctgccggctctgcggcctcttccgcgtcttcgccttcgccctcag
acgagtcggatctccctttgggccgcctccccgcctggtacctttaagaccaatgacttacaaggcagctgtagatctta
gccactttttaaaagaaaaggggggactggaagggctaattcactcccaacgaagacaagatcacctgcaggaca
ggcgcgccataacttcgtatagcatacattatacgaagttatggtacctgcgcgccctgctttttgcttgtactgggtctctct
ggttagaccagatctgagcctgggagctctctggctaactagggaacccactgcttaagcctcaataaagcttgccttg
agtgcttcaagtagtgtgtgcccgtctgttgtgtgactctggtaactagagatccctcagacccttttagtcagtgtggaaa
atctctagcacccgggcgattaaggaaagggctagatcattcttgaagacgaaagggcctcgtgatacgcctattttat

aggttaatgtcatgataataatggtttcttagacgtcaggtggcacttttcggggaaatgtgcgcggaaccccctatttgttta ttttctaaatacattcaaatatgtatccgctcatgagacaataaccctgataaatgcttcaataatattgaaaaaggaag agtatgagtattcaacatttccgtgtcgcccttattcccttttttgcggcattttgccttcctgttttttgctcacccagaaacgctg gtgaaagtaaaagatgctgaagatcagttgggtgcacgagtgggttacatcgaactggatctcaacagcggtaagat ccttgagagttttcgccccgaagaacgttttccaatgatgagcacttttaaagttctgctatgtggcgcggtattatcccgtg ttgacgccgggcaagagcaactcggtcgccgcatacactattctcagaatgacttggttgagtactcaccagtcacag aaaagcatcttacggatggcatgacagtaagagaattatgcagtgctgccataaccatgagtgataacactgcggcc aacttacttctgacaacgatcggaggaccgaaggagctaaccgcttttttgcacaacatgggggatcatgtaactcgc cttgatcgttgggaaccggagctgaatgaagccataccaaacgacgagcgtgacaccacgatgcctgtagcaatgg caacaacgttgcgcaaactattaactggcgaactacttactctagcttcccggcaacaattaatagactggatggagg cggataaagttgcaggaccacttctgcgctcggcccttccggctggctggtttattgctgataaatctggagccggtgag cgtgggtctcgcggtatcattgcagcactggggccagatggtaagccctcccgtatcgtagttatctacacgacgggga gtcaggcaactatggatgaacgaaatagacagatcgctgagataggtgcctcactgattaagcattggtaactgtcag accaagtttactcatatatactttagattgatttaaaacttcatttttaatttaaaaggatctaggtgaagatcctttttgataatc tcatgaccaaaatcccttaacgtgagttttcgttccactgagcgtcagacccgtagaaaagatcaaaggatcttcttga gatcctttttttctgcgcgtaatctgctgcttgcaaacaaaaaaaccaccgctaccagcggtggtttgtttgccggatcaag agctaccaactctttttccgaaggtaactggcttcagcagagcgcagataccaaatactgttcttctagtgtagccgtagt taggccaccacttcaagaactctgtagcaccgcctacatacctcgctctgctaatcctgttaccagtggctgctgccagt ggcgataagtcgtgtcttaccgggttggactcaagacgatagttaccggataaggcgcagcggtcgggctgaacggg gggttcgtgcacacagcccagcttggagcgaacgacctacaccgaactgagatacctacagcgtgagctatgagaa agcgccacgcttcccgaagggagaaaggcggacaggtatccggtaagcggcagggtcggaacaggagagcgc acgagggagcttccaggggggaaacgcctggtatctttatagtcctgtcgggtttcgccacctctgacttgagcgtcgattt ttgtgatgctcgtcaggggggcggagcctatggaaaaacgccagcaacgcggcctttttacggttcctggccttttgctg gccttttgctcacatgttctttcctgcgttatcccctgattctgtggataaccgtattaccgcctttgagtgagctgataccgct cgccgcagccgaacgaccgagcgcagcgagtcagtgagcgaggaagcggaagagcgcccaatacgcaaacc gcctctccccgcgcgttggccgattcattaatgcagcaagctcatggctgactaatttttttatttatgcagaggccgagg ccgcctcggcctctgagctattccagaagtagtgaggaggctttttttggaggcctaggcttttgcaaaaagctccccgtg gcacgacaggtttcccgactggaaagcgggcagtgagcgcaacgcaattaatgtgagttagctcactcattaggcac cccaggctttacactttatgcttccggctcgtatgttgtgtggaattgtgagcggataacaatttcacacaggaaacagct atgacatgattacgaatttcacaaataaagcatttttttcactgcattctagttgtggtttgtccaaactcatcaatgtatcttat catgtctggatcaactggataactcaagctaaccaaaatcatcccaaacttcccaccccataccctattaccactgcca attacctgtggtttcatttactctaaacctgtgattcctctgaattattttcattttaaagaaattgtatttgttaaatatgtactaca aacttagtagt

**Claims**

1. An *in vitro* method for generating induced neural border stem cells by the direct reprogramming of somatic human cells, comprising the step of culturing said somatic human cells in the presence of a mixture of transcription factors, wherein said mixture comprises the factors BRN2, SOX2, KLF4 and ZIC3, and wherein said culturing is performed in the presence of GSK-3 inhibitor Chir99021; Alk5 inhibitor II; and hedgehog/smoothened agonist Purmorphamine.

2. The *in vitro* method of claim 1, wherein said culturing is performed in the additional presence of monoamine-oxidase inhibitor Tranylcypromine.

3. The *in vitro* method of claim 2, wherein Tranylcypromine is only present during an induction phase, particularly in the first 12 to 21 days of said culturing, particularly in the first 12 to 16 days when said somatic cells are pMSCs, and 17 to 21 days when said somatic cells are PBMCs.

4. The *in vitro* method of any one of claims 1 to 3, further comprising the step of isolating an induced neural border stem cell line, wherein said induced neural border stem cell line is **characterized by** being positive both (i) for early neural markers PAX6, ASCL1, BRN2 and SOX1; and (ii) for stem cell markers NESTIN and SOX2.

5. The *in vitro* method of any one of claims 1 to 4, further comprising the step of expanding said induced neural border stem cells or said isolated induced neural border stem cell line, comprising the step of culturing said induced neural border stem cells or cells from said isolated induced neural border stem cell line, particularly wherein said culturing is performed in the presence of a substance selected from DLL1, DLL3 and DLL4, Jagged-1, and Jagged-2, more particularly from DLL4 and JAGGED-1.

6. The *in vitro* method of claim 5, wherein said culturing is performed on a layer of murine fibroblast cells as supportive feeder cells.

7. An *in vitro* method for generating and differentiating induced neural border stem cells comprising the steps of (i) performing the method of any one of claims 1 to 6, and (ii) culturing said induced neural border stem cells or cells from said isolated induced neural border stem cell line in the presence of differentiation factors.

8. The *in vitro* method of claim 7, further comprising the step of isolating a central nervous system primed neural progenitor cell line of the central nervous system lineage, particularly wherein said cell line is **characterized by** progenitor markers LONRF2, ZNF217, NESTIN, SOX1 and SOX2, particularly LONRF2 and ZNF217, and by being negative for MSX1, PAX3 and TFAP2.

9. An *in vitro* method for generating CNS progenitor cells, comprising the steps of (i) performing the method of any one of claims 1 to 6, and (ii) culturing induced neural border stem cells or cells from said isolated induced neural border stem cell line in a medium comprising GSK-3 inhibitor Chir99021 ; ALK 4,5,7 inhibitor SB431542; hedgehog/smoothened agonist Purmorphamine; bFGF; and LIF.

10. The in vitro method of claim 9, wherein the culture is maintained on a gelatinous protein mixture secreted by Engelbreth-Holm-Swarm mouse sarcoma cells (Matrigel).

11. An *in vitro* method for generating and differentiating central nervous system progenitor cells, comprising the steps of (i) performing the method of claim 9 or 10, and (ii) culturing said central nervous system progenitor cells in the presence of differentiation factors.

12. The *in vitro* method of claim 7, wherein said induced neural border stem cells are differentiated to cells of a neural crest lineage.

13. An *in vitro* method for generating neural crest progenitor cells, comprising the steps of (i) performing the method of any one of claims 1 to 6, and (ii) culturing induced neural border stem cells or cells from said isolated induced neural border stem cell line for three days in the presence of GSK-3 inhibitor Chir99021; Alk5 inhibitor II; and BMP4; followed by culturing in the presence of GSK-3 inhibitor Chir99021, FGF8, IGF1 and DAPT.

14. An *in vitro* method for generating and differentiating a neural crest progenitor cell line,-comprising the steps of (i) performing the method of claim 13, and (ii) culturing said neural crest progenitor cells in the presence of differentiation factors.

**Patentansprüche**

1. In-vitro-Verfahren zur Erzeugung von induzierten Stammzellen der neuralen Grenze durch direkte Umprogrammierung somatischer menschlicher Zellen, umfassend den Schritt der Kultivierung der besagten somatischen menschlichen Zellen in Gegenwart eines Gemisches von Transkriptionsfaktoren, wobei das Gemisch die Faktoren BRN2, S0X2, KLF4 und ZIC3 umfasst, und wobei die Kultivierung in Gegenwart des GSK-3-Inhibitors Chir99021, Alk5-Inhibitors II und des Hedgehog/Smoothened-Agonisten Purmorphamin durchgeführt wird.

2. In-vitro-Verfahren nach Anspruch 1, wobei die Kultivierung in zusätzlicher Anwesenheit des Monoamin-Oxidase-Inhibitors Tranylcypromin durchgeführt wird.

3. In-vitro-Verfahren nach Anspruch 2, wobei Tranylcypromin nur während einer Induktionsphase vorhanden ist, insbesondere in den ersten 12 bis 21 Tagen der Kultivierung, insbesondere in den ersten 12 bis 16 Tagen, wenn es sich bei den somatischen Zellen um pMSCs handelt, und in den ersten 17 bis 21 Tagen, wenn es sich bei den somatischen Zellen um PBMCs handelt.

4. In vitro-Verfahren nach einem der Ansprüche 1 bis 3, das ferner den Schritt der Isolierung einer induzierten Stammzelllinie der neuralen Grenze umfasst, wobei die induzierte neurale Stammzelllinie der neuralen Grenze **dadurch gekennzeichnet ist, dass** sie sowohl (i) für frühe neurale Marker PAX6, ASCL1, BRN2 und SOX1 als auch (ii) für Stammzellmarker NESTIN und SOX2 positiv ist.

5. In-vitro-Verfahren nach einem der Ansprüche 1 bis 4, das ferner den Schritt des Expandierens der induzierten Stammzellen der neuralen Grenze oder der isolierten induzierten Stammzelllinie der neuralen Grenze umfasst, der den Schritt des Kultivierens der induzierten Stammzellen der neuralen Grenze oder der Zellen aus der isolierten induzierten Stammzelllinie der neuralen Grenze umfasst, wobei das Kultivieren insbesondere in Gegenwart einer Substanz durchgeführt wird, die ausgewählt ist aus DLL1, DLL3 und DLL4, Jagged-1 und Jagged-2, insbesondere aus DLL4 und JAGGED-1.

6. In-vitro-Verfahren nach Anspruch 5, wobei die Kultivierung auf einer Schicht aus murinen Fibroblastenzellen als unterstützende Fütterzellen durchgeführt wird.

7. In-vitro-Verfahren zur Erzeugung und Differenzierung von induzierten Stammzellen der neuralen Grenze, umfassend die Schritte: (i) Durchführen des Verfahrens nach einem der Ansprüche 1 bis 6 und (ii) Kultivieren der induzierten Stammzellen der neuralen Grenze oder von Zellen aus der isolierten induzierten Stammzelllinie der neuralen Grenze in Gegenwart von Differenzierungsfaktoren.

8. In vitro-Verfahren nach Anspruch 7, das ferner den Schritt der Isolierung einer für das Zentralnervensystem vorbereiteten neuralen Vorläuferzelllinie der Zentralnervensystemlinie umfasst, wobei besagte Zelllinie insbesondere durch die Vorläufermarker LONRF2, ZNF217, NESTIN, SOX1 und S0X2, insbesondere LONRF2 und ZNF217 gekennzeichnet ist und dadurch, dass sie negativ für MSX1, PAX3 und TFAP2 ist.

9. In-vitro-Verfahren zur Erzeugung von ZNS-Vorläuferzellen, umfassend die Schritte: (i) Durchführen des Verfahrens nach einem der Ansprüche 1 bis 6 und (ii) Kultivieren von induzierten Stammzellen der neuralen Grenze oder Zellen aus der isolierten induzierten Stammzelllinie der neuralen Grenze in einem Medium, das den GSK-3-Inhibitor Chir99021, den ALK 4,5,7-Inhibitor SB431542, den Hedgehog/Smoothened-Agonisten Purmorphamin, bFGF und LIF umfasst.

10. In-vitro-Verfahren nach Anspruch 9, wobei die Kultur auf einem gelatinösen Proteingemisch gehalten wird, das von Engelbreth-Holm-Swarm-Maus-Sarkomzellen (Matrigel) sezerniert wird.

11. In-vitro-Verfahren zur Erzeugung und Differenzierung von Vorläuferzellen des Zentralnervensystems, umfassend die Schritte: (i) Durchführung des Verfahrens nach Anspruch 9 oder 10 und (ii) Kultivierung der Vorläuferzellen des Zentralnervensystems in Gegenwart von Differenzierungsfaktoren.

12. In vitro-Verfahren nach Anspruch 7, wobei die induzierten Stammzellen der neuralen Grenze zu Zellen einer Neuralleistenlinie differenziert werden.

13. In-vitro-Verfahren zur Erzeugung von Vorläuferzellen der Neuralleiste, umfassend die Schritte: (i) Durchführen des Verfahrens nach einem der Ansprüche 1 bis 6 und (ii) Kultivieren von induzierten Stammzellen der neuralen Grenze oder Zellen aus der isolierten induzierten Stammzelllinie der neuralen Grenze für drei Tage in Gegenwart des GSK-3-Inhibitors Chir99021, Alk5-Inhibitors II und von BMP4, gefolgt von Kultivieren in Gegenwart des GSK-3-Inhibitors Chir99021, von FGF8, IGF1 und DAFT.

14. In-vitro-Verfahren zur Erzeugung und Differenzierung einer Vorläuferzelllinie der Neuralleiste, umfassend die Schritte: (i) Durchführung des Verfahrens nach Anspruch 13 und (ii) Kultivierung der Vorläuferzellen der Neuralleiste in Gegenwart von Differenzierungsfaktoren.

**Revendications**

1. Procédé *in vitro* pour générer des cellules souches de la bordure neurale induites par la reprogrammation directe de cellules humaines somatiques, comprenant l'étape de culture desdites cellules humaines somatiques en présence d'un mélange de facteurs de transcription, dans lequel ledit mélange comprend les facteurs BRN2, SOX2, KLF4 et ZIC3, et dans lequel ladite culture est effectuée en présence de l'inhibiteur de GSK-3 Chir99021 ; de l'inhibiteur d'ALK5 II ; et de l'agoniste de la voie de signalisation hedgehog/smoothened purmorphamine.

2. Procédé *in vitro* selon la revendication 1, dans lequel ladite culture est effectuée avec la présence supplémentaire de l'inhibiteur de la monoamine-oxydase tranylcypromine.

3. Procédé *in vitro* selon la revendication 2, dans lequel la tranylcypromine est uniquement présente pendant une phase d'induction, en particulier dans les 12 à 21 premiers jours de ladite culture, en particulier dans les 12 à 16 premiers jours lorsque lesdites cellules somatiques sont des cellules stromales mésenchymateuses d'origine pancréatique CSMp, et dans les 17 à 21 jours lorsque lesdites cellules somatiques sont des cellules mononucléées du sang périphérique CMSP.

4. Procédé *in vitro* selon l'une quelconque des revendications 1 à 3, comprenant en outre l'étape d'isolement d'une lignée de cellules souches de la bordure neurale induites, dans lequel ladite lignée de cellules souches de la bordure neurale induites est **caractérisée par** le fait d'être positive à la fois (i) pour les marqueurs neuraux précoces PAX6, ASCL1, BRN2 et SOX1 ; et (ii) pour les marqueurs de cellules souches NESTIN et SOX2.

5. Procédé *in vitro* selon l'une quelconque des revendications 1 à 4, comprenant en outre l'étape d'expansion desdites cellules souches de la bordure neurale induites ou de ladite lignée isolée de cellules souches de la bordure neurale induites, comprenant l'étape de culture desdites cellules souches de la bordure neurale induites ou des cellules de ladite lignée isolée de cellules souches de la bordure neurale induites, en particulier dans lequel ladite culture est effectuée en présence d'une substance choisie parmi DLL1, DLL3 et DLL4, JAGGED-1, et JAGGED-2, plus particulièrement parmi DLL4 et JAGGED-1.

6. Procédé *in vitro* selon la revendication 5, dans lequel ladite culture est effectuée sur une couche de cellules de fibroblastes murins comme cellules nourricières de soutien.

7. Procédé *in vitro* pour générer et différencier des cellules souches de la bordure neurale induites comprenant les étapes consistant à (i) mettre en oeuvre le procédé selon l'une quelconque des revendications 1 à 6, et (ii) cultiver lesdites cellules souches de la bordure neurale induites ou les cellules de ladite lignée isolée de cellules souches de la bordure neurale induites en présence de facteurs de différenciation.

8. Procédé *in vitro* selon la revendication 7, comprenant en outre l'étape d'isolement d'une lignée de cellules progénitrices neurales du système nerveux central (SNC) à l'état amorcé, en particulier dans lequel ladite lignée cellulaire est **caractérisée par** les marqueurs de progéniteurs LONRF2, ZNF217, NESTIN, SOX1 et SOX2, en particulier LONRF2 et ZNF217, et par le fait qu'elle est négative pour MSX1, PAX3 et TFAP2.

9. Procédé *in vitro* pour générer des cellules progénitrices du SNC, comprenant les étapes consistant à (i) mettre en oeuvre le procédé selon l'une quelconque des revendications 1 à 6, et (ii) cultiver des cellules souches de la bordure neurale induites ou des cellules de ladite lignée isolée de cellules souches de la bordure neurale induites dans un milieu comprenant l'inhibiteur de GSK-3 Chir99021 ; l'inhibiteur d'ALK4, ALK5, ALK7 SB431542 ; l'agoniste de la voie de signalisation hedgehog/smoothened purmorphamine ; bFGF ; et LIF.

10. Procédé *in vitro* selon la revendication 9, dans lequel la culture est maintenue sur un mélange gélatineux de protéines sécrété par des cellules de sarcome murin d'Engelbreth-Holm-Swarm (Matrigel).

11. Procédé *in vitro* pour générer et différencier des cellules progénitrices du système nerveux central, comprenant les étapes consistant à (i) mettre en oeuvre le procédé selon la revendication 9 ou 10, et (ii) cultiver lesdites cellules progénitrices du système nerveux central en présence de facteurs de différenciation.

12. Procédé *in vitro* selon la revendication 7, dans lequel lesdites cellules souches de la bordure neurale induites sont différenciées en cellules d'une lignée de la crête neurale.

13. Procédé *in vitro* pour générer des cellules progénitrices de la crête neurale, comprenant les étapes consistant à (i) mettre en oeuvre le procédé selon l'une quelconque des revendications 1 à 6, et (ii) cultiver des cellules souches de la bordure neurale induites ou des cellules de ladite lignée isolée de cellules souches de la bordure neurale induites pendant trois jours en présence de l'inhibiteur de GSK-3 Chir99021 ; de l'inhibiteur d'ALK5 II ; et de BMP4 ; puis cultiver en présence de l'inhibiteur de GSK-3 Chir99021, de FGF8, d'IGF1 et de DAPT.

**14.** Procédé *in vitro* pour générer et différencier une lignée de cellules progénitrices de la crête neurale, comprenant les étapes consistant à (i) mettre en oeuvre le procédé selon la revendication 13, et (ii) cultiver lesdites cellules progénitrices de la crête neurale en présence de facteurs de différenciation.

# Figure 1:

*f*

*Biological Process (GO)*

| | pathway ID | pathway description | count in gene set | e.g. | false discovery rate |
|---|---|---|---|---|---|
| **Stem Cell Identity** | GO:0010721 | negative regulation of cell development | 14 | LIN28A, SOX11, DLL1 | 3.19E-05 |
| | GO:0097150 | neuronal stem cell population maintenance | 4 | HES5, SOX2, NOTCH1 | 4.05E-03 |
| | GO:0021915 | neural tube development | 7 | SALL4, ZIC2, PAX6 | 3.41E-02 |
| | GO:2000179 | positive regulation of neural precursor cell proliferation | 7 | INSM1, NOTCH1, FZD3 | 6.21E-05 |
| **CNS related pathways** | GO:0007399 | nervous system development | 53 | NES, FGFR3, ZIC3 | 7.04E-11 |
| | GO:0007420 | brain development | 22 | PAX6, ZIC2, FABP7 | 2.47E-05 |
| | GO:0007417 | central nervous system development | 24 | POU3F2, NHLH2, PTPRZ1 | 1.01E-04 |
| **Crest related pathways** | GO:0007423 | sensory organ development | 20 | ASCL1, LEF1, CHD7 | 1.13E-05 |
| | GO:0060322 | head development | 22 | HOXB2, FZD3, SOX3 | 6.25E-05 |
| | GO:0043583 | ear development | 10 | VANGL2, FGFR3, FRZB | 1.78E-03 |
| | *KEGG Pathways* | pathway description | count in gene set | e.g. | false discovery rate |
| **Signaling** | 4110 | Cell cycle | 10 | CDC7, E2F2, MCM2 | 4.37E-05 |
| | 4330 | Notch signaling pathway | 5 | HES5, NOTCH1, DLL3 | 9.27E-03 |
| | 4310 | Wnt signaling pathway | 7 | CCND2, LEF1, FZD9 | 2.14E-02 |

i

NES

## Figure 2:

**n**

**Developmental Period**

O

# Figure 3:

a

h

40 mV

200 ms

i

100 µm

# Figure 4:

a

E8.5 embryo

pNBSCs

EP 3 418 378 B1

75

**Figure 5:**

**b** | **Biological Processes upregulated in iNBSCs and pNBSCs**

*Biological Process (GO)*

| | pathway ID | pathway description | count in gene set | e.g. | false discovery rate |
|---|---|---|---|---|---|
| Stem Cell Identity | GO:0050768 | negative regulation of neurogenesis | 13 | DLL1, SOX2 | 1.03E-04 |
| | GO:0061351 | neural precursor cell proliferation | 10 | EPHB1, FABP7 | 2.10E-06 |
| | GO:0045665 | negative regulation of neuron differentiation | 13 | SOX2, SOX3 | 9.53E-06 |
| | GO:0097150 | neuronal stem cell population maintenance | 5 | NOTCH1, SALL4 | 4.82E-04 |
| Early Neural Development | GO:0021915 | neural tube development | 10 | HES5,GBX2 | 1.91E-03 |
| | GO:0035239 | tube morphogenesis | 14 | CHD7,MYCN | 6.07E-03 |
| | GO:0060562 | epithelial tube morphogenesis | 13 | LAMA1, GBX2 | 6.41E-03 |
| CNS related pathways | GO:0007399 | nervous system development | 59 | HES6, NES | 2.41E-09 |
| | GO:0007420 | brain development | 24 | FABP7, POU3F2 | 9.39E-05 |
| Crest related pathways | GO:0007423 | sensory organ development | 25 | HOXA2, SMOC1 | 4.56E-07 |
| | GO:0048485 | sympathetic nervous system development | 4 | FZD3, INSM1 | 6.41E-03 |
| | **KEGG Pathways** | **pathway description** | **count in gene set** | **e.g.** | **false discovery rate** |
| Signalling | 4110 | cell cycle | 16 | MCM3, E2F5 | 3.39E-10 |

**c** | **Biological Processes downregulated in iNBSCs and pNBSCs**

*Biological Process (GO)*

| | pathway ID | pathway description | count in gene set | e.g. | false discovery rate |
|---|---|---|---|---|---|
| Fibroblast identity | GO:0030198 | extracellular matrix organization | 42 | COL3A1, COL1A1 | 3.77E-30 |
| | GO:0009888 | tissue development | 42 | CEBPB, EGFR | 3.3E-08 |
| | GO:0009611 | response to wounding | 28 | ANXA5, CTGF | 1.08E-07 |

**Figure 6:**

b

iNBSC-derived
sensory neurons

iNBSCs          WT          SCN9A$^{-/-}$

Nav1.7

ACTIN

C

e

f

## Extended Data Figure 1:

a

b

c

OCT4

d

| | Colonies picked | Expandable lines | Characterized lines | Passages |
|---|---|---|---|---|
| FPFs | 22 | 18 | 5 | > 40 |
| ADFs | 35 | 25 | 12 | > 40 |
| PBMCs | 32 | 28 | 14 | > 40 |

e

Conversion efficiency

f

g

**Transgene levels (mRNA)**

h

**Transgene level (gDNA)**

i

## PBMC-derived iNBSC

k

**iPSC-derived NBSCs**

# Extended Data Figure 2:

b

C

SOX10 PAX6

d

| Name | P-value | Adjusted p-value | Z-score | Combined score |
|------|---------|------------------|---------|----------------|
| Neural Crest Differentiation_Homo sapiens_WP2064 | 1,16E-05 | 1,79E-03 | -1,92 | 12,17 |
| PDGF Pathway_Homo sapiens_WP2526 | 9,73E-04 | 1,96E-02 | -2,03 | 7,97 |
| IL-2 Signaling Pathway_Homo sapiens_WP49 | 1,21E-03 | 1,96E-02 | -1,96 | 7,71 |
| IL-5 Signaling Pathway_Homo sapiens_WP127 | 1,05E-03 | 1,96E-02 | -1,94 | 7,63 |

e

iNBSCs:

iNBSC to MSC Differentiation:

g

| | pathway ID | pathway description | count in gene set | e.g. | false discovery rate |
|---|---|---|---|---|---|
| MSC-like cells | GO:0030198 | extracellular matrix organization | 29 | COL12A1, SPARC | 1.25E-19 |
| | GO:0009611 | response to wounding | 34 | COL3A1, COL1A1, MYL9 | 6.36E-16 |
| | GO:0001501 | skeletal system development | 20 | LRRC17, TGBR2, CTGF | 4.9E-09 |
| | GO:0001503 | ossification | 15 | FSTL3, COL5A2, CDH11 | 3.22E-08 |

h

k

m

O

p

**EMX1**

**FOXG1**

**OTX2**

**GBX2**

**IRX3**

r

**Extended Data Figure 3:**

a

d

Single NPCs
embedded in MG

Chir
Alk5-Inh.

bFGF
BMP4

250 µm

e

f

**Extended Data Figure 4:**

a

b

| Number of experiments | Embryonic stage | polyclonal, short term expansion? (<3 passages) | Derivation of stable clones from separate experiments | Number of clones derived |
|---|---|---|---|---|
| 2 | E7.5 | 2/2 | 0/2 | 0 |
| 5 | E8.5 | 5/5 | 4/5 | 19 |
| 3 | E9.5 | 3/3 | 0/3 | 0 |

c

e

f

**Clonogenicity**

h

j

## Biological Processes upregulated in pNBSCs compared to MEFs

| BiologicalProcess (GO) | | | | | |
|---|---|---|---|---|---|
| | pathway ID | pathway description | count in gene set | e.g. | false discovery rate |
| Stem Cell Identity | GO:2000177 | regulation of neural precursor cell proliferation | 7 | CD24a, Bex1, Insm1 | 3.38E-04 |
| | GO:0019827 | stem cell population maintenance | 6 | Hes5, Sox2, Lin28a | 2.96E-02 |
| | GO:0035295 | tube development | 24 | Fgf3, Sox2, Sall1 | 4.17E-08 |
| Nervous system related pathways | GO:0007399 | nervous system development | 66 | Irx3,Zfp423, Nefm | 9.43E-24 |
| | GO:0007420 | brain development | 28 | Fabp7, Zic5, Nefl | 1.28E-10 |
| | GO:0007417 | central nervous system development | 34 | Pou3f1, Sox1 | 1.05E-12 |
| Crest related pathways | GO:0060322 | head development | 28 | Nr2f1, HoxA2, Sox3 | 4.04E-10 |
| | GO:0043583 | ear development | 9 | Fgf3, Sall1, Chd7 | 3.36E-03 |

## Biological Processes upregulated in pNBSC-derived RG-like SC compared to pNBSCs

| BiologicalProcess (GO) | | | | | |
|---|---|---|---|---|---|
| | pathway ID | pathway description | count in gene set | e.g. | false discovery rate |
| RG Stem Cell related pathways | GO:0010001 | glial cell differentiation | 13 | S100b, Kcnj10, Nkx2-2 | 1.71E-07 |
| | GO:0007399 | nervous system development | 41 | Slc1a3, Omg, Tnc | 6.33E-07 |
| | GO:0048709 | oligodendrocyte differentiation | 8 | Olig2,Cnp, Ptprz1 | 1.33E-05 |

## Biological Processes upregulated in pNBSC-derived NC compared to pNBSCs

| BiologicalProcess (GO) | | | | | |
|---|---|---|---|---|---|
| | pathway ID | pathway description | count in gene set | e.g. | false discovery rate |
| Crest related pathways | GO:0048701 | embryonic cranial skeleton morphogenesis | 7 | Mmp14, Pdgfra, Dlx2 | 2.63E-05 |
| | GO:0042692 | muscle cell differentiation | 13 | Pdgfrb, Myh9, Speg | 3.00E-05 |
| | GO:0009611 | response to wounding | 17 | Fn1, Bmp4, Pdgfa | 2.42E-06 |

**Extended Data Figure 5:**

## a

b

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KIM, Y. J. et al.** *Cell Stem Cell*, 2014, vol. 15 (4), 497-506
- **REINHARDT, P. et al.** *PLoS ONE*, 2013, vol. 8 (3), e59252
- **LI, W. et al.** *PNAS*, 2011, vol. 108 (20), 8299-8304
- **KOCH, P. et al.** *PNAS*, 2009, vol. 106 (9), 3225-3230
- **WANG, J. et al.** *Proc Natl Acad Sci USA.*, 2014, vol. 111 (28), E2885-94
- **LU, H. et al.** *Dev Cell*, 2013, vol. 27 (5), 560-73
- **SCOGNAMIGLIO, R et al.** *Cell*, 2016, vol. 164 (4), 668-680
- **IWAFUCHI-DOI, M. et al.** *Development*, 2012, vol. 139 (24), 4675-4675
- **CONTI, L. et al.** Niche-independent symmetrical self-renewal of a mammalian tissue stem cell. *Plos Biol*, 2005, vol. 3, e283
- **ELKABETZ, Y.** ; **STUDER, L.** Human ESC-derived neural rosettes and neural stem cell progression.. *Cold Spring Harb Symp Quant Biol*, 2007, vol. 73, 377-387
- **KOCH, P.** ; **OPITZ, T.** ; **STEINBECK, J. A.** ; **LADEWIG, J.** ; **BRÜSTLE, O.** A rosette-type, self-renewing human ES cell-derived neural stem cell with potential for in vitro instruction and synaptic integration.. *Proc Natl Acad Sci USA*, 2009, vol. 106, 3225-3230
- **LI, W. et al.** Rapid induction and long-term self-renewal of primitive neural precursors from human embryonic stem cells by small molecule inhibitors.. *Proc Natl Acad Sci USA*, 2011, vol. 108, 8299-8304
- **TAILOR, J. et al.** Stem cells expanded from the human embryonic hindbrain stably retain regional specification and high neurogenic potency.. *J. Neurosci.*, 2013, vol. 33, 12407-12422
- **REINHARDT, P. et al.** Derivation and expansion using only small molecules of human neural progenitors for neurodegenerative disease modeling.. *PLoS ONE*, 2013, vol. 8, e59252
- **GORRIS, R. et al.** Pluripotent stem cell-derived radial glia-like cells as stable intermediate for efficient generation of human oligodendrocytes.. *Glia*, 2015, vol. 63, 2152-2167
- **EFE, J. A. et al.** Conversion of mouse fibroblasts into cardiomyocytes using a direct reprogramming strategy.. *Nat Cell Biol*, 2011, vol. 13, 215-222

- **KIM, J. et al.** Direct reprogramming of mouse fibroblasts to neural progenitors.. *Proc Natl Acad Sci USA*, 2011, vol. 108, 7838-7843
- **MARGARITI, A. et al.** Direct reprogramming of fibroblasts into endothelial cells capable of angiogenesis and reendothelialization in tissue-engineered vessels.. *Proc Natl Acad Sci USA*, 2012, vol. 109, 13793-13798
- **THIER, M. et al.** Direct conversion of fibroblasts into stably expandable neural stem cells.. *Cell Stem Cell*, 2012, vol. 10, 473-479
- **BUNG, R. et al.** Partial Dedifferentiation of Murine Radial Glia-Type Neural Stem Cells by Brn2 and c-Myc Yields Early Neuroepithelial Progenitors.. *J. Mol. Biol.*, 2016, vol. 428, 1476-1483
- **ARTINGER, K. B.** ; **FRASER, S.** ; **BRONNER-FRASER, M.** Dorsal and Ventral Cell Types Can Arise from Common Neural Tube Progenitors.. *Developmental Biology*, 1995, vol. 172, 11-11
- **GARNETT, A. T. A.** ; **SQUARE, T. A. T.** ; **MEDEIROS, D. M. D.** BMP, Wnt and FGF signals are integrated through evolutionarily conserved enhancers to achieve robust expression of Pax3 and Zic genes at the zebrafish neural plate border.. *Development*, 2012, vol. 139, 4220-4231
- **LE DRÉAU, G.** ; **MARTÍ, E.** Dorsal-ventral patterning of the neural tube: a tale of three signals.. *Dev Neurobiol*, 2012, vol. 72, 1471-1481
- **EMINLI, S. et al.** Differentiation stage determines potential of hematopoietic cells for reprogramming into induced pluripotent stem cells.. *Nat Genet*, 2009, vol. 41, 968-976
- **BRAMBRINK, T. et al.** Sequential Expression of Pluripotency Markers during Direct Reprogramming of Mouse Somatic Cells.. *Stem Cell*, 2008, vol. 2, 9-9
- **NORI, S. et al.** Long-term safety issues of iPSC-based cell therapy in a spinal cord injury model: oncogenic transformation with epithelial-mesenchymal transition.. *Stem Cell Reports*, 2015, vol. 4, 360-373
- **GALAT, V. et al.** Transgene Reactivation in Induced Pluripotent Stem Cell Derivatives and Reversion to Pluripotency of Induced Pluripotent Stem Cell-Derived Mesenchymal Stem Cells.. *Stem Cells and Development*, 2016, vol. 25, 1060-1072
- **SCOGNAMIGLIO, R. et al.** Myc Depletion Induces a Pluripotent Dormant State Mimicking Diapause.. *Cell*, 2016, vol. 164, 668-680

- **CHEN, E. Y. et al.** Enrichr: interactive and collaborative HTML5 gene list enrichment analysis tool.. *BMC Bioinformatics*, 2013, vol. 14, 128
- **JOHNSON, M. B. et al.** Single-cell analysis reveals transcriptional heterogeneity of neural progenitors in human cortex.. *Nat Neurosci*, 2015, vol. 18, 637-646
- **STEIN, J. L. et al.** A quantitative framework to evaluate modeling of cortical development by neural stem cells.. *Neuron*, 2014, vol. 83, 69-86
- **LU, J. et al.** Generation of serotonin neurons from human pluripotent stem cells.. *Nat Biotechnol*, 2016, vol. 34, 89-94
- **LANCASTER, M. A. et al.** Cerebral organoids model human brain development and microcephaly.. *Nature*, 2013
- **MEINHARDT, A. et al.** 3D reconstitution of the patterned neural tube from embryonic stem cells.. *Stem Cell Reports*, 2014, vol. 3, 987-999
- **QIAN, X. et al.** Brain-Region-Specific Organoids Using Mini-bioreactors for Modeling ZIKV Exposure.. *Cell*, 2016, vol. 165, 1238-1254
- **JO, J. et al.** Midbrain-like Organoids from Human Pluripotent Stem Cells Contain Functional Dopaminergic and Neuromelanin-Producing Neurons.. *Cell Stem Cell*, 2016, vol. 19, 248-257
- **CHAMBERS, S. M. et al.** Combined small-molecule inhibition accelerates developmental timing and converts human pluripotent stem cells into nociceptors.. *Nat Biotechnol*, 2012, vol. 30, 715-720
- **POUNDS, S. et al.** A procedure to statistically evaluate agreement of differential expression for cross-species genomics.. *Bioinformatics*, 2011, vol. 27, 2098-2103
- **SZKLARCZYK, D. et al.** STRING v10: protein-protein interaction networks, integrated over the tree of life.. *Nucleic Acids Research*, 2015, vol. 43, D447-52
- **LUJAN, E.** ; **CHANDA, S.** ; **AHLENIUS, H.** ; **SÜDHOF, T. C.** ; **WERNIG, M.** Direct conversion of mouse fibroblasts to self-renewing, tripotent neural precursor cells.. *Proc Natl Acad Sci USA*, 2012, vol. 109, 2527-2532
- **HAN, D. W. et al.** Direct Reprogramming of Fibroblasts into Neural Stem Cells by Defined Factors.. *Cell Stem Cell*, 2012, vol. 8
- **CAIRNS, D. M. et al.** Expandable and Rapidly Differentiating Human Induced Neural Stem Cell Lines for Multiple Tissue Engineering Applications.. *Stem Cell Reports*, 2016, vol. 0, 557-570
- **HOU, P.-S. et al.** Direct Conversion of Human Fibroblasts into Neural Progenitors Using Transcription Factors Enriched in Human ESC-Derived Neural Progenitors.. *Stem Cell Reports*, 2016, vol. 8, 54-68
- **LEE, J. H. et al.** Single Transcription Factor Conversion of Human Blood Fate to NPCs with CNS and PNS Developmental Capacity.. *Cell Reports*, 2015, vol. 11, 1367-1376
- **ELKABETZ, Y. et al.** Human ES cell-derived neural rosettes reveal a functionally distinct early neural stem cell stage.. *Genes & Development*, 2008, vol. 22, 152-165
- **COX, J. J. et al.** An SCN9A channelopathy causes congenital inability to experience pain.. *Nature*, 2006, vol. 444, 894-898
- **HOCKEMEYER, D. et al.** A Drug-Inducible System for Direct Reprogramming of Human Somatic Cells to Pluripotency.. *Cell Stem Cell*, 2008, vol. 3, 346-353
- **SOMMER, A. G. et al.** Generation of human induced pluripotent stem cells from peripheral blood using the STEMCCA lentiviral vector.. *J Vis Exp*, 2012, e4327-e4327
- **MEYER, S.** ; **WÖRSDÖRFER, P.** ; **GÜNTHER, K.** ; **THIER, M.** ; **EDENHOFER, F.** Derivation of Adult Human Fibroblasts and their Direct Conversion into Expandable Neural Progenitor Cells.. *J Vis Exp*, 2015, e52831-e52831
- **SCHINDELIN, J. et al.** Fiji: an open-source platform for biological-image analysis.. *Nat Meth*, 2012, vol. 9, 676-682
- **RAN, F. A. et al.** Genome engineering using the CRISPR-Cas9 system.. *Nat Protoc*, 2013, vol. 8, 2281-2308
- **DEHAIRS, J.** ; **TALEBI, A.** ; **CHERIFI, Y.** ; **SWINNEN, J. V.** CRISP-ID: decoding CRISPR mediated indels by Sanger sequencing.. *Sci Rep*, 2016, vol. 6, 28973
- **SUBRAMANIAN, A.** ; **KUEHN, H.** ; **GOULD, J.** ; **TAMAYO, P.** ; **MESIROV, J. P.** GSEA-P: a desktop application for Gene Set Enrichment Analysis.. *Bioinformatics*, 2007, vol. 23, 3251-3253
- **POUNDS, S. et al.** A procedure to statistically evaluate agreement of differential expression for cross-species genomics. *Bioinformatics*, 2011, vol. 27, 2098-2103
- **ARYEE, M. J. et al.** Minfi: a flexible and comprehensive Bioconductor package for the analysis of Infinium DNA methylation microarrays.. *Bioinformatics*, 2014, vol. 30, 1363-1369
- **ASSENOV, Y. et al.** Comprehensive analysis of DNA methylation data with RnBeads.. *Nat Meth*, 2014, vol. 11, 1138-1140